# EUROPEAN PATENT APPLICATION

(11) **EP 4 837 922 A1**
(43) Date of publication of application: **11.11.2026**
(21) Application number: 25175564.1
(22) Date of filing: 10.05.2025
(51) Int. Cl.: C07K 16/30

(54) **ANTIBODIES AND RADIOPHARMACEUTICAL COMPLEXES TARGETING CEACAM5**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present disclosure relates to antibodies or antigen-binding fragments thereof having binding affinity for the carcinoembryonic antigen-related cell adhesion molecule 5 (CEACAM5), to conjugates and to tissue-targeting compounds of formula (I) comprising antibodies or antigen-binding fragments thereof having binding affinity for CEACAM5. In particular, the present disclosure relates to tissue-targeting compounds comprising an antibody or an antigen-binding fragment thereof having binding affinity for CEACAM5. Further, the present disclosure relates to combinations, preferably pharmaceutical combinations comprising a tissue-targeting compound of formula (I) and a further pharmaceutical agent.

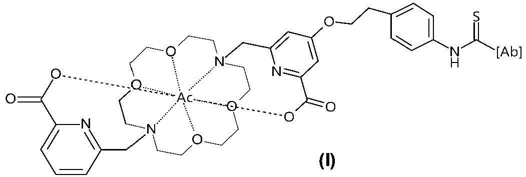

## Description

The present disclosure relates to novel monoclonal antibodies having binding affinity for the carcinoembryonic antigen-related cell adhesion molecule 5 (CEACAM5, CEAM5, CD66e, CEA), i.e., novel monoclonal antibodies capable of binding to the carcinoembryonic antigen-related cell adhesion molecule 5 (CEACAM5, CEAM5, CD66e, CEA) or antigen-binding fragments thereof and conjugates comprising these anti-CEACAM5 antibodies or fragments thereof. The present disclosure further relates to targeted radionuclide conjugates such as targeted actinium-225 (Ac-225) conjugates (TACs). Preferred are targeted actinium conjugates of formula (I) comprising the anti-CEACAM5 antibodies according to the present disclosure or fragments thereof. Said antibodies or antigen binding fragments thereof, and/or said antibody conjugates and/or said targeted actinium conjugates are useful in diagnostics and/or therapy, preferably in therapy, more preferably in treating hyperproliferative diseases such as cancer, Further, the present disclosure relates to combinations, preferably pharmaceutical combinations comprising a tissue-targeting compound of formula (I) and a further pharmaceutical agent. Said tissue-targeting compounds and/or combinations are useful in diagnostics and/or therapy, preferably in therapy, more preferably in treating hyperproliferative diseases such as cancer.

The present disclosure further provides methods and tools to generate the antibodies, functional fragments and conjugates according to the disclosure. For example, provided are polynucleotides encoding the foregoing antibodies or fragments, vectors containing the same, and cells for production. Further, the present disclosure relates to a process for the preparation of said tissue-targeting compounds and to their use in diagnostics and/or in the treatment of disease, preferably in the treatment of disease, particularly against hyperproliferative diseases, in particular against cancer.

### BACKGROUND

Specific cell killing can be essential for the successful treatment of a variety of diseases in mammalian subjects. To achieve this, Typical examples of this are the treatment of malignant diseases such as hyperproliferative diseases, in particular cancer, sarcomas and carcinomas. Further, the selective elimination of certain cell types can also play a key role in the treatment of other diseases, especially hyperplastic and neoplastic diseases. To achieve maximal specific cell killing of cancerous cells, high selectivity towards a specific cancer antigen as well as high affinity to retain binding on the cancer antigen is necessary.

The most common methods of selective treatment are currently surgery and external beam irradiation. Targeted radionuclide therapy is, however, a promising and developing area with the potential to deliver highly cytotoxic radiation specifically to cell types associated with disease. One advantage of targeted radionuclide therapies (TRTs) is their inherent mode of action and in particular its independence of cancer antigen internalization. This is in contrast to antibody drug conjugates (ADCs) which require cell internalization in order to release their toxic payload into the cell cytoplasm to induce cytotoxicity. Compared to antibody drug conjugates, targeted radionuclide therapies may result in higher cancer cell cytotoxicity. The most common forms of radiopharmaceuticals currently authorized for use in humans employ beta-emitting and/or gamma-emitting radionuclides. There has, however, been some interest in the use of alpha-emitting radionuclides in therapy because of their potential for more potent cell killing.

The unique mode-of-action of targeted alpha therapy (TAT) is the induction of difficult-to-repair clustered DNA double strand breaks through specific delivery of alpha-particle emitting radionuclides, like actinium-225, to tumors using tumor antigen targeting moieties such as small molecules, peptides or monoclonal antibodies. The exceptional cytotoxicity of alpha emitting radionuclides demands specific biological targeting antibodies in order to avoid unacceptable side effects. Biological targeting of the isotopes strongly depends on the stability of the chelator, the robustness of the antibody to the chelation process, and the targeting properties of the antibody to its particular target in the specific biological context.

The radiation range of typical alpha emitters in physiological surroundings is generally less than 100 micrometers, the equivalent of only a few cell diameters. This makes these sources well suited for the treatment of tumors, including micro-metastases, because they have the range to reach neighboring cells within a tumor but if they are well targeted then little of the radiated energy will pass beyond the target cells. Thus, not every cell need be targeted but damage to surrounding healthy tissue may be minimized (see Feinendegen et al., Radiat. Res. 148:195-201 (1997)). In contrast, a beta particle has a range of 1 mm or more in water (see Wilbur, Antibody Immunocon. Radiopharm. 4: 85-96 (1991)).

The energy of alpha-particle radiation is high in comparison with that carried by beta particles, gamma rays and X-rays, typically being 5-8 MeV, or 5 to 10 times that of a beta particle and 20 or more times the energy of a gamma ray. Thus, this deposition of a large amount of energy over a very short distance gives alpha-radiation an exceptionally high linear energy transfer (LET), high relative biological efficacy (RBE) and low oxygen enhancement ratio (OER) compared to gamma and beta radiation (see Hall, "Radiobiology for the radiologist", Fifth edition, Lippincott Williams & Wilkins, Philadelphia PA, USA, 2000). This explains the exceptional cytotoxicity of alpha emitting radionuclides and also imposes stringent demands on the biological targeting of such isotopes and upon the level of control and study of alpha emitting radionuclide distribution which is necessary in order to avoid unacceptable side effects.

So far, with regards to the application in radioimmunotherapy the main attention has been focused on 211At, 213Bi and 225Ac and these three nuclides have been explored in clinical immunotherapy trials. However, although targeted radiotherapy has been practiced for some time using macrocyclic complexes of radionuclides, the compounds currently in use, which typically employ the macrocyclic chelator DOTA (2,2',2",2‴-(1,4,7,10-Tetraazacyclododecane-1,4,7,10-tetrayl)tetraacetic acid) are lacking in stability, leading to the dissociation of the radionuclide from the chelating macrocycle, which results in a reduced selectivity and activity to the targeted tissue and an increased toxicity to the non-targeted tissue. Serum stability studies have shown that the [225Ac]-DOTA complex dissociates over time (Ramogida et al., Evaluation of Polydentate Picolinic Acid Chelating Ligands and an α-Melanocyte- Stimulating Hormone Derivative for Targeted Alpha Therapy Using ISOL-Produced 225Ac. EJNMMI Radiopharm. Chem. 2019, 4, 21) leading to deposition of 225Ac in the liver and femur after the administration of DOTA-based conjugates. (Deal et al., Improved in Vivo Stability of Actinium-225 Macrocyclic Complexes. J. Med. Chem. 1999, 42, 2988-2992.).

Further effort was made for the identification and development of suitable chelating agents for the large Ac³⁺ ion (Thiele et al., Actinium-225 for Targeted α Therapy: Coordination Chemistry and Current Chelation Approaches. Cancer Biother. Radiopharm. 2018, 33, 336-348). Acyclic chelators like ethylenediamine tetraacetic acid (H4EDTA) and diethylenetriamine pentaacetic acid (H5DTPA) are suffering of lability of the resulting complexes (Davis et al., Comparison of 225Actinium Chelates: Tissue Distribution and Radiotoxicity. Nucl. Med. Biol. 1999, 26, 581-589). Macrocyclic chelators as tetraazacy-clododecane-1,4,7,10-tetraacetic acid (H4DOTA) have shown to be more inert. However, radiolabeling of H4DOTA with 225Ac either requires high temperatures, which is incompatible with temperature-sensitive macro-molecular biomolecules such as antibodies, or long incubation times, which yields constructs with relatively low specific activities and low purity.

For example, it has been reported that a high chelator-to-antigen (CAR) of 10 has been used in a pre-clinical study using a one-step radiolabeling procedure of a DOTA antibody chelator conjugate (ACC) (William F. Maguire, Michael R. McDevitt, Peter M. Smith-Jones, David A. Scheinberg. "Efficient one-step radiolabeling of monoclonal antibodies to high specific activity with Actinium-225 for alpha-particle radioimmunotherapy of cancer", J. Nucl. Med. 2014; 55(9): 1492-1498). However, after 2h labeling at 37 °C, quantitative labeling was not achieved which subsequentially requires a purification step to remove non-labeled Ac-225. This is in contrast to the tissue-targeting compounds of the present disclosure, in which Ac-225 can be chelated quantitatively at room temperature after one hour leading to complexes with significantly lower CAR ratios (<1).

While the use of larger macrocycles has led to more stable complexation of especially radionuclides of larger size, there is a need for the development of targeting compounds with improved stability, selectivity and efficacy, especially in the field of hyperproliferative diseases such as cancer. Further, there is an increased need for new agents capable of selectively targeting specific cells and cell types in malignant diseases such as cancer which are highly effective and reduce, mitigate and/or avoid the prevalence of resistances. In particular, there is a need to provide new therapeutic agents for the treatment of colorectal cancer (CRC), non-small-cell lung cancer (NSCLC), gastric cancer (GAC), neuroendocrine cancer (NENC) and pancreatic cancer (PAC), as well as breast, liver and thyroid cancer, in which patients may initially respond to therapy but eventually develop resistances (Zhao B, Wang L, Qiu H, Zhang M, Sun L, Peng P, Yu Q, Yuan X. Mechanisms of resistance to anti-EGFR therapy in colorectal cancer. Oncotarget. 2017 Jan 17;8(3):3980-4000. doi: 10.18632/oncotarget.14012. PMID: 28002810; PMCID: PMC5354808, Wu J, Lin Z. Non-Small Cell Lung Cancer Targeted Therapy: Drugs and Mechanisms of Drug Resistance. Int J Mol Sci. 2022 Dec 1;23(23):15056. doi: 10.3390/ijms232315056. PMID: 36499382; PMCID: PMC9738331, Tafe LJ. Molecular mechanisms of therapy resistance in solid tumors: chasing "moving" targets. Virchows Arch. 2017 Aug;471(2):155-164. doi: 10.1007/s00428-017-2101-7. Epub 2017 Mar 10. PMID: 28280929, Blangé D, Stroes CI, Derks S, Bijlsma MF, van Laarhoven HWM. Resistance mechanisms to HER2-targeted therapy in gastroesophageal adenocarcinoma: A systematic review. Cancer Treat Rev. 2022 Jul;108:102418. doi: 10.1016/j.ctrv.2022.102418. Epub 2022 May 30. PMID: 35689885, Massironi S, Albertelli M, Hasballa I, Paravani P, Ferone D, Faggiano A, Danese S. "Cold" Somatostatin Analogs in Neuroendocrine Neoplasms: Decoding Mechanisms, Overcoming Resistance, and Shaping the Future of Therapy. Cells. 2025 Feb 9;14(4):245. doi: 10.3390/cells14040245. PMID: 39996718; PMCID: PMC11854070, Chen Z, Li Y, Tan B, Zhao Q, Fan L, Li F, Zhao X. Progress and current status of molecule-targeted therapy and drug resistance in gastric cancer. Drugs Today (Barc). 2020 Jul;56(7):469-482. doi: 10.1358/dot.2020.56.7.3112071. PMID: 32648857, Pang WM, Lim WM, Ngai ZN, Koh RY. Molecular Mechanisms of Immune Resistance in Pancreatic Cancer: An Update. Curr Pharm Biotechnol. 2025 Apr 15. doi: 10.2174/0113892010365485250330110031. Epub ahead of print. PMID: 40237046).

The carcinoembryonic antigen-related cell adhesion molecule 5 (CEACAM5, CD66e, CEA) encoded by the CEACAM5 (carcinoembryonic antigen-related cell adhesion molecule 5) gene, has been described to be highly expressed in those above-referenced cancer types, for which there is a particular therapeutic need, making it a suitable target for the development of new therapeutic agents.

Antibody drug conjugates (ADC) have received increased attention as therapeutic agents in the treatment of cancer (see, for example: Bujnak AC, Solaru SA, Tewari K.Gynecol Oncol. 2025 Mar 25;195:180-191. doi: 10.1016/j.ygyno.2025.03.013; Jiang K, Wang S.Transl Breast Cancer Res. 2025 Jan 21;6:9. doi: 10.21037/tbcr-24-38) and in particular for CEACAM5 (Hu Y, Du X, Yuan J, Gong X, Zhu Y, Li H, Lin X, Zheng F, Ran Y, Na Z, Hu H. A high-affinity antibody-drug conjugates Actuximab-MMAE for potent and selective targeting of CEACAM5-Positive tumors. Cancer Lett. 2025 Jun 28;620:217685. doi: 10.1016/j.canlet.2025.217685. Epub 2025 Mar 29. PMID: 40158720, Lefebvre AM, Adam J, Nicolazzi C, Larois C, Attenot F, Falda-Buscaiot F, Dib C, Masson N, Ternès N, Bauchet AL, Demers B, Chadjaa M, Sidhu S, Combeau C, Soria JC, Scoazec JY, Naimi S, Angevin E, Chiron M, Henry C. The search for therapeutic targets in lung cancer: Preclinical and human studies of carcinoembryonic antigen-related cell adhesion molecule 5 expression and its associated molecular landscape. Lung Cancer. 2023 Oct;184:107356. doi: 10.1016/j.lungcan.2023.107356. Epub 2023 Aug 26. PMID: 37660479). Two such ADCs are Tusamitamab ravtansine and M9140. While the latter one is evaluated in early clinical trials (Scott Kopetz et al. First-in-human trial of M9140, an anti-CEACAM5 antibody drug conjugate (ADC) with exatecan payload, in patients (pts) with metastatic colorectal cancer (mCRC). JCO 42, 3000-3000(2024). DOI:10.1200/JCO.2024.42.16_suppl.3000.), Tusamitamab ravtansine's clinical development was terminated in December 2023 as it did not meet its primary end point in a phase 3 clincial trial (https://www.sanofi.com/en/media-room/press-releases/2023/2023-12-21-06-30-00-2799759). Another ADC, Labetuzumab govitecan (Dotan E, Cohen SJ, Starodub AN, Lieu CH, Messersmith WA, Simpson PS, Guarino MJ, Marshall JL, Goldberg RM, Hecht JR, Wegener WA, Sharkey RM, Govindan SV, Goldenberg DM, Berlin JD. Phase I/II Trial of Labetuzumab Govitecan (Anti-CEACAM5/SN-38 Antibody-Drug Conjugate) in Patients With Refractory or Relapsing Metastatic Colorectal Cancer. J Clin Oncol. 2017 Oct 10;35(29):3338-3346. doi: 10.1200/JCO.2017.73.9011. Epub 2017 Aug 17. PMID: 28817371; PMCID: PMC8259133.), was also terminated during clinical development. A retrospective subgroup analysis of the phase 3 data suggested a trend of improved relative progression free survival (PFS) and overall survival (OS) for tusamitamab ravtansine versus docetaxel among patients with ≥80% CEACAM5.

Despite the scientific focus on CEACAM5 as a target, there is an unmet medical need for the development of further compounds which are capable of targeting CEACAM5 and provide a therapeutic effect in the treatment of hyperproliferative diseases, such as cancer, related to an overexpression of CEACAM5.

In particular, there is an unmet medical need for the provision of compounds, such as isolated antibodies or antigen-binding fragments thereof, which can efficiently bind to CEACAM5 and which display one or more of the following properties:
(i) enhanced affinity for the target antigen, enabling more precise and effective binding,
(ii) optimized blood clearance profiles, which can improve pharmacokinetics and reduce off-target effects,
(iii) antibody discrimination between cell-bound and soluble CEA,
(iv) optimized selectivity over other CEACAM family members,
(v) superior tumor uptake, facilitating targeted delivery to tumor tissues while minimizing systemic exposure.
(vi) stability enhancements, such as resistance to aggregation or degradation, which may further contribute to improved shelf-life and therapeutic efficacy,
(vii) efficient internalization properties, making them particularly suitable for applications such as antibody-drug conjugates (ADCs),
(viii) reduced immunogenicity, achieved through humanization or sequence optimization, which may lower the risk of adverse immune responses, enhancing safety and tolerability.
(ix) novel binding characteristics or functional properties due to unique sequence variations in the variable or constant regions of, e.g., isolated antibodies or antigen-binding fragments thereof.

These features, individually or in combination, provide a basis for differentiation and innovation, ensuring that new CEACAM5-targeting compounds address unmet needs in the current therapeutic landscape.

It has surprisingly been found that the isolated antibodies or antigen-binding fragments thereof according to the present disclosure have advantageous properties. The isolated antibodies or antigen-binding fragments thereof disclosed herein exhibit one or more distinguishing features that differentiate them from existing antibodies, providing significant therapeutic and diagnostic advantages. For instance, the isolated antibodies or antigen-binding fragments thereof of the present disclosure exhibit a higher affinity towards CEACAM5 than the reported values for CEACAM5-targeting antibodies known in the art (see, for example, EP4079761). Further, the isolated antibodies or antigen-binding fragments thereof of the present disclosure comprise no lysine in their six CDRs. Given the preferred conjugation of the isolated antibodies or antigen-binding fragments thereof of the present disclosure to the chelating moiety via a lysine to form tissue-targeting compounds of formula (I) of the present disclosure, the absence of lysine in their CDRs renders the isolated antibodies or antigen-binding fragments thereof of the present disclosure more suitable for the formation of tissue-targeting compounds of formula (I) and thereby less prone to loss of activity (i.e., binding towards CEACAM5) due to deactivation of their CDRs during conjugation to form tissue-targeting compounds of formula (I).

Further, it has surprisingly been found that the isolated antibodies or antigen-binding fragments thereof according to the present disclosure can be easily conjugated to a chelating moiety capable of binding an alpha-emitting radionuclide to form tissue-targeting compounds, i.e., the tissue-targeting compounds of formula (I) according to the present disclosure. The tissue-targeting compounds of formula (I) and the combinations comprising a tissue-targeting compound of formula (I) and a further pharmaceutical agent of the present disclosure show advantageous properties with regard to their stability, activity, efficacy and selectivity. In particular, the tissue-targeting compounds of formula (I) of the present disclosure show high values for the immunoreactive fraction (IRF) at low chelator-to-antigen (CAR) ratios and high monomeric purity, thus leading to a higher fraction of radionuclide-labelled compound having affinity for CEACAM5. Further, the tissue-targeting compounds of formula (I) of the present disclosure show a favorable biodistribution in vivo and low accumulation in the liver, which is a sign of reduced dissociation of the radionuclide from the chelating moiety and therefore of increased stability. In contrast to previously reported examples in the literature, the tissue-targeting compounds of formula (I) of the present disclosure can be labelled with the radionuclide under very mild conditions and at reduced temperatures (i.e., room temperature), thereby reducing antibody denaturation and the amount of non-CEACAM5 binding fraction in the final product. Thus, the tissue-targeting compounds of formula (I) of the present disclosure show a better tumor to liver ratio and can therefore treat disease more effectively and selectively, reducing damage to non-targeted tissue while maintaining a high potency against cancer cells in the targeted tissue. In combination with one or more further pharmaceutical agents, a greater therapeutic efficacy can be achieved than when either compound is used alone.

In the field of radiopharmaceuticals, there is an increased need for the provision of radionuclide-containing compounds and/or combinations comprising radionuclide-containing compounds which are stable over sufficient periods of time that allow their delivery to the administering physician and the patient without suffering from radiopharmaceutical degradation, e.g., by decomplexation of the radionuclide from the chelator. Increasing the stability of the radiopharmaceutical in radionuclide-containing compounds and/or combinations comprising radionuclide-containing compounds is especially desirable from a logistical perspective, since this would allow for the reliable delivery of the radiopharmaceutical-containing drug product across countries and/or continents. It would be desirable to have radionuclide-containing compounds and/or combinations comprising radionuclide-containing compounds available which are stable for at least 48 hours, preferably for 96 hours and/or which have a monomer content of at least 85% for at least 48 hours, preferably a monomer content of at least 90% for at least 48 hours. The present disclosure solves these issues by providing tissue-targeting compounds of formula (I) and combinations comprising a tissue-targeting compound of formula (I) and a further pharmaceutical agent.

In particular, combinations comprising a tissue-targeting compound of formula (I) and a further pharmaceutical agent of the present disclosure will serve to:
(1) yield better efficacy in reducing the growth of a tumor or even eliminate the tumor as compared to administration of either agent alone,
(2) provide for the administration of lesser amounts of the administered targeted compounds and/or chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time until tumor progression, and/or
(8) yield efficacy and tolerability results at least as good as those of the agents used alone, compared to known instances where other cancer agent combinations produce inhibitory effects.

### SUMMARY

The present disclosure relates to isolated antibodies or antigen- binding fragments thereof capable of binding to CEACAM5. Further, the present disclosure relates to antibody conjugates targeting CEACAM5 comprising the isolated antibodies or antigen binding fragments according to the present disclosure. Further, the present disclosure relates to tissue-targeting compounds of formula (I) 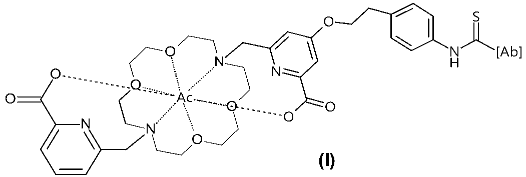 wherein [Ab] is a monoclonal antibody or an antigen-binding fragment thereof having binding affinity for the carcinoembryonic antigen-related cell adhesion molecule 5 (CEACAM5), i.e., capable of binding to CEACAM5 wherein [Ab] is selected from an isolated antibody or antigen-binding fragment as described throughout the present disclosure.

### DEFINITIONS

Unless defined otherwise, all technical and scientific terms used herein have the meaning commonly understood by one of ordinary skill in the art to which this disclosure belongs. The following references, however, can provide one of skill in the art to which this disclosure pertains with a general definition of many of the terms used in this disclosure, and can be referenced and used so long as such definitions are consistent with the meaning commonly understood in the art. Such references include, but are not limited to, Singleton et al., Dictionary of Microbiology and Molecular Biology (2nd ed. 1994); The Cambridge Dictionary of Science and Technology (Walker ed., 1988); Hale & Marham, The Harper Collins Dictionary of Biology (1991); and Lackie et al., The Dictionary of Cell & Molecular Biology (3d ed. 1999); and Cellular and Molecular Immunology, Eds. Abbas, Lichtman and Pober, 2nd Edition, W.B. Saunders Company. Any additional technical resource available to the person of ordinary skill in the art providing definitions of terms used herein having the meaning commonly understood in the art can be consulted. For the purposes of the present disclosure, the following terms are further defined. Additional terms are defined elsewhere in the description. As used herein and in the appended claims, the singular forms "a," and "the" include plural reference unless the context clearly dictates otherwise. Thus, for example, reference to "a gene" is a reference to one or more genes and includes equivalents thereof known to those skilled in the art, and so forth.

The term "comprising" when used in the specification includes "consisting of". In the context of the present disclosure, the term "comprises" or "comprising" means "including, but not limited to". The term is intended to be open-ended, to specify the presence of any stated features, elements, integers, steps or components, but not to preclude the presence or addition of one or more other features, elements, integers, steps, components or groups thereof. The term "comprising" thus includes the more restrictive terms "consisting of" and "essentially consisting of".

In the context of the present disclosure, the term "about" or "approximately" means within 80% to 120%, alternatively within 90% to 110%, including within 95% to 105% of a given value or range.

If within the present text any item is referred to as "as mentioned herein", it means that it may be mentioned anywhere in the present text.

In the context of the present disclosure, "tissue targeting" is used herein to indicate that the substance in question (i.e. a tissue-targeting compound, a tissue-targeting actinium complex and/or a tissue-targeting moiety, particularly when in the form of a tissue-targeting complex as described herein), serves to localize itself (and particularly to localize any conjugated actinium complex) preferentially to at least one tissue site at which its presence (e.g. to deliver a radioactive decay) is desired. Thus, a tissue-targeting compound, complex, group or moiety serves to provide greater localization to at least one desired site in the body of a subject following administration to that subject in comparison with the concentration of an equivalent complex not having the targeting moiety. The targeting moiety in the present case will be preferably selected to bind specifically to cell-surface receptors associated with cancer cells or other receptors associated with the tumor microenvironment.

In the context of the present disclosure, the term "CEACAM5" refers to the carcinoembryonic antigen-related cell adhesion molecule 5 (CEACAM5 gene, CD66e, CEA, CEAM5; gene ID 1048, Uniprot: P06731; https://www.uniprot.org/uniprotkb/P06731/entry), also referred to CEACAM5. The CEACAM5 protein comprises human, cynomolgus and further mammalian and non-mammalian homologues.

Human CEACAM5 (SEQ ID NO: 145):












The terms "anti-CEACAM5 antibody" and "an antibody that binds to CEACAM5" or "an antibody that binds to CEACAM5" or "CEACAM5-binding antibody" or "antibody having affinity for CEACAM5" are used synonymously and/or interchangeably herein and refer to an antibody that is capable of binding CEACAM5, preferably with sufficient affinity such that the antibody is useful as a diagnostic and/or therapeutic agent in targeting CEACAM5. Similar considerations apply to antigen- binding fragments.

A "radionuclide" (also: "radioactive nuclide", "radioisotope" or "radioactive isotope") is an atom that undergoes radioactive decay. Without limitation, for example the radionuclide may be a β-emitting radionuclide (beta emitter), an α-particle-emitting radionuclide (alpha emitter), or an Auger electron emitting radionuclide (Auger electron emitter).

"β-emitting radionuclides" or "beta emitter" are radionuclides which emit beta particles. Examples of beta emitters include without limitation copper-67 (67Cu), strontium-89 (89Sr), yttrium-90 (90Y), rhodium-105 (105Rh), iodine-131 (1311), promethium-149 (149Pm), holmium-166 (166Ho), lutetium-177 (177Lu), rhenium-186 (186Re), rhenium-188 (188Re), gold-198 (198Au) and gold-199 (199Au). Jongho has reviewed various techniques for chelation of various of these radioisotypes (Jeon, Jongho. "Review of therapeutic applications of radiolabeled functional nanomaterials." International journal of molecular sciences 20.9 (2019): 2323.).

"Auger electron emitting radionuclides" or "Auger electron emitter" are radionuclides which emit Auger electrons. Examples of Auger electron emitters include without limitation bromine-77, indium-111, iodine-123, and iodine-125.

"α-emitting radionuclides" or "alpha emitter" are radionuclides which emit alpha particles, i.e. 4He nuclei with a +2 charge. Non limiting examples of alpha emitters include bismuth-213 (213Bi), characterized by a half-life of 45.6 min, actinium-225 (225Ac), characterized by a half-life of 9.9 d, astatine-211 (211At), characterized by a half-life of 7.2 h, radium-223 (223Ra), characterized by a half-life of 11.4 d, radium-224 (224Ra), characterized by a half-life of 3.7 d, and thorium-227 (227Th), characterized by a half-life of 18.7 d.

The tissue-targeting compounds of formula (I) of the present disclosure comprise a chelating moiety comprising a complexed actinium atom (Ac). In the context of the present disclosure, the term "Ac" means an ion of at least one alpha-emitting actinium isotope. Preferably, the alpha-emitting actinium isotope is 225Ac. Preferably, the ion of at the least one alpha-emitting actinium isotope is ²²⁵Ac having a triple positive charge, i.e., ²²⁵Ac³⁺. Thus, in the context of the present disclosure, the term "Ac" preferably, but not exclusively, means ²²⁵Ac³⁺.

Radionuclides can be obtained as known in the art. For example, as described in Poty, Sophie, et al, 211At can be cyclotron-produced by bombarding natural bismuth with a medium energy α -particle beam using the 209Bi(α,2n)211At reaction ("Poty, Sophie, et al. α-Emitters for radiotherapy: from basic radiochemistry to clinical studies-part 1/2." Journal of Nuclear Medicine 59.6/59.7 (2018): 878-884 / 1020-1027). 227Th and 223Ra are both available upon separation from their mutual parent, 227Ac. Clinical production of 223Ra uses 227Ac/227Th-based generators. Parent isotopes are loaded on actinide chromatographic resin and 223Ra chloride solution is obtained after elution with 1M HCl or HNO3, subsequent cation exchange column, evaporation, and dissolution in saline solution.

"Chelation" refers to the formation or presence of two or more separate coordinate bonds between a ligand and a single central metal atom. The ligands which are capable of forming these coordinate bonds are termed "chelators", "chelating agents", or "sequestering agents".

"A chelating moiety" is a chelator which can chelate a given radionuclide or group of radionuclides, such as, without limitation, alpha emitters, beta emitters or Auger electron emitter. Various chelators are known in the art and can be used according to the current disclosure, e.g. as described in Price, Eric W., and Chris Orvig. "Matching chelators to radiometals for radiopharmaceuticals." Chemical Society Reviews 43.1 (2014): 260-290, incorporated herein in its entirety.

In the context of the present disclosure the chelating moiety comprises for example an 18-membered chelating moiety as for example the macrocyclic chelator "macropa" (N,N'-bis[(6-carboxy-2-pyridil)methyl]-4,13-diaza-18-crown-6). The macrocyclic chelator macropa has been described, for example, in Thiele et al., Angew. Chem. Int. Ed. 2017, 56(46), 14712 and references therein. Macropa chelators and derivatives thereof, as for example "Macropa-NCS" (6-[[16-[(6-carboxy-2-pyridyl)methyl]-1,4,10,13-tetraoxa-7,16-diazacyclooctadec-7-yl]methyl]-4-[2-(4-isothiocyanatophenyl)ethoxy]pyridine-2-carboxylic acid), have also been described in WO2018/183906 and WO2020/106886 and references therein.

The chelating moiety for complexation of actinium (or "actinium-complexating chelating moiety") may terminate in an isothiocyanate or carboxylic acid moiety in order to facilitate its coupling with the targeting moiety (e.g an anti-CEACAM5 antibody or an antigen-binding fragment thereof according to the disclosure). When the macropa chelator is used, one of the pyridine rings of the macropa chelator is typically substituted so that it can be coupled to the targeting. This can be a simple functional group (e.g., a carboxylic acid -COOH) or a more complex chemical structure, as long as it is capable of being coupled to a targeting moiety (e.g. an anti- CEACAM5 antibody or an antigen-binding fragment thereof according to the disclosure). For example, the substituent on the pyridine ring of the macropa chelator can terminate in an isothiocyanate or carboxylic acid moiety.

A "targeting moiety" as used herein is any chemical structure binding to a biological target, such as CEACAM5 or a cell expressing CEACAM5. The targeting moiety localizes itself, e.g. as part of a bigger structure at a specific site where the presence is required to exert the intended effect, e.g. delivery of radioactive decay in case of a TAC. Thus, a tissue targeting group or moiety serves to provide greater localization of a molecule or conjugate to at least one desired site in the body of a subject in comparison with the concentration of an equivalent complex not comprising the targeting moiety. The targeting moiety can be for example selected without limitation from the group consisting of nucleotides, DNA and RNA fragments, aptamers, peptides, proteins, antibodies or fragments thereof, nanoparticles, or small molecules, or any combination thereof. According to the current disclosure, the targeting moiety is preferably an antibody or antigen-binding fragment thereof (i.e. the anti-CEACAM5-antibodies or fragments thereof of the present disclosure).

A "targeted actinium conjugate" (also "TAC") is a conjugate comprising a targeting moiety (i.e., the anti-CEACAM5 antibodies or fragments thereof of the present disclosure) and at least one chelator or chelating group for complexation of actinium (e.g. macropa, preferably macropa-NCS), and optionally comprise actinium. A tissue-targeting compound of formula (I) as described throughout the present disclosure is a targeted actinium conjugate and the two terms (i.e., "targeted actinium conjugate, TAC" and "tissue-targeting compound of formula (I)") can be used interchangeably.

In the context of the present disclosure, term "DOTA" refers to 2,2',2",2‴-(1,4,7,10-tetraazacyclododecane-1,4,7,10- tetrayl)tetraaceticacid or 1,4,7,10-tetra-azacycloododecane-N,N',N",N‴-tetraacetic acid and derivatives thereof, which can chelate a radionuclide. DOTA is a chelator arranged for complexation of an α-particle-emitting radionuclide, such as 212Bi, 213Bi, 225Ac, 227Th. Chelators such as DOTA form stable complexes with a chelated As-225, which exists in the 3+ oxidation state. In order to achieve sufficient labeling of DOTA-coupled antibodies, the complexation step should preferably be performed as a two-step process or directly at elevated temperatures.

In the compounds of formula (I) of the present disclosure, the actinium (Ac) atom is shown as being complexed to four oxygen and two nitrogen atoms of the macrocyclic ring as well as to two carboxylate groups. While this is the assumed complexation pattern for the actinium atom, this depiction includes all possible and conceivable cases in which one or more of the bonds is not present, e.g., where the actinium atom is not bound to all heteroatoms of the macrocyclic ring, or to the carboxylate groups, etc.

In the context of the present disclosure, the term IRF refers to the Immunoreactive Fraction i.e., the fraction of the labelled product (i.e., the compounds of formula (I) of the present disclosure) which is capable of binding to the target (i.e., CEACAM5). The Lindmo assay (Lindmo T., et al. (1984) "Determination of the immunoreactive fraction of radiolabeled monoclonal antibodies by linear extrapolation to binding at infinite antigen excess." J. Immunol. Methods. 72, 77-89) is the most commonly used method for assessing the immunoreactive fraction.

In the context of the present disclosure, the term CAR refers to the Chelator-to-Antigen Ratio, which is a measure of the specific activity of the radiolabeled compound (e.g., the compounds of formula (I) of the present disclosure).

The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. Unless otherwise indicated, a particular polypeptide sequence also implicitly encompasses conservatively modified variants thereof.

Amino acids may be referred to herein by their commonly known three letter symbols or by the one-letter symbols recommended by the IUPAC-IUB Biochemical Nomenclature Commission. Nucleotides, likewise, may be referred to by their commonly accepted single-letter codes.

The term "antibody", as used herein, is intended to refer to immunoglobulin molecules including, but not limited to, full-length antibodies and monovalent antibodies. "Full-length antibodies" are preferably comprised of four polypeptide chains, two heavy (H) chains and two light (L) chains which are typically inter-connected by disulfide bonds. Each heavy chain is comprised of a heavy chain variable region (abbreviated herein as VH) and a heavy chain constant region. The heavy chain constant region can comprise e.g., three domains CH1, CH2 and CH3. Each light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs arranged from amino-terminus to carboxy-terminus e.g., in the following order: FR1, CDR1, FR2, CDR2, FR3, CDR3, FR4. "Monovalent antibodies" as used herein are preferably comprised of three polypeptide chains, two heavy (H) chains and one light (L) chain which are typically inter-connected by disulfide bonds. One heavy chain is comprised of a heavy chain variable region (abbreviated herein as VH) and a heavy chain constant region. The heavy chain constant region can comprise e.g., three domains CH1, CH2 and CH3. The other heavy chain is comprised of a heavy chain constant region only. The light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs arranged from amino-terminus to carboxy-terminus e.g., in the following order: FR1, CDR1, FR2, CDR2, FR3, CDR3, FR4.

As used herein, the term "Complementarity Determining Regions" (CDRs; e.g., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (e.g. about residues 24-34 (L1), 50-56 (L2) and 89-97 (L3) in the light chain variable domain and 31-35 (H1), 50-65 (H2) and 95-102 (H3) in the heavy chain variable domain; (Kabat et al., Sequences of Proteins of Immunolological Interest, 5th Ed. Public Health Service, National Institutes of Health, Bethesda, MD. (1991)) and/or those residues from a "hypervariable loop" (e.g. about residues 26-32 (L1), 50-52 (L2) and 91-96 (L3) in the light chain variable domain and 26- 32 (H1), 53-55 (H2) and 96-101 (H3) in the heavy chain variable domain (Chothia and Lesk; J Mol Biol 196: 901-917 (1987)). In some instances, a complementarity determining region can include amino acids from both a CDR region defined according to Kabat and a hypervariable loop.

Depending on the amino acid sequence of the constant domain of their heavy chains, intact antibodies can be assigned to different "classes". There are five major classes of intact antibodies: IgA, IgD, IgE, IgG, and IgM, and several of these maybe further divided into "subclasses" (isotypes), e.g., IgG1, IgG2, IgG3, IgG4, IgA1, and IgA2. A preferred class of immunoglobulins for use in the present disclosure is IgG. In particular embodiments, the antibody according to the present disclosure is an IgG1, an IgG2, an IgG3 or an IgG4 antibody, more particularly an IgG1 antibody. Different isotypes may have different effector functions. Human light chains are classified as kappa (K) and lambda (λ) light chains. Within light and heavy chains, the variable and constant regions are joined by a "J" region of about 12 or more amino acids, with the heavy chain also including a "D" region of about 10 more amino acids. See generally, Fundamental Immunology, Ch. 7 (Paul, W., ed., 2nd ed. Raven Press, N.Y. (1989)). The heavy-chain constant domains that correspond to the different classes of antibodies are called [alpha], [delta], [epsilon], [gamma], and [mu], respectively. The subunit structures and three-dimensional configurations of different classes of immunoglobulins are well known. As used herein antibodies are conventionally known antibodies and functional fragments thereof.

A "functional fragment" or "antigen-binding antibody fragment" of an antibody/immunoglobulin hereby is defined as a fragment of an antibody/immunoglobulin (e.g., a variable region of an IgG) that retains the antigen-binding region. An "antigen-binding region" of an antibody typically is found in one or more hyper variable region(s) of an antibody, e.g., the CDR1, -2, and/or -3 regions; however, the variable "framework" regions can also play an important role in antigen binding, such as by providing a scaffold for the CDRs. Preferably, the "antigen-binding region" comprises at least amino acid residues 4 to 103 of the variable light (VL) chain and 5 to 109 of the variable heavy (VH) chain, more preferably amino acid residues 3 to 107 of VL and 4 to 111 of VH, and particularly preferred are the complete VL and VH chains (amino acid positions 1 to 109 of VL and 1 to 113 of VH; numbering according to WO 97/08320).

Nonlimiting examples of "functional fragments" or "antigen-binding antibody fragments" include Fab, Fab', F(ab')2, Fv fragments, domain antibodies (dAb), complementarity determining region (CDR) fragments, single-chain antibodies (scFv), single chain antibody fragments, diabodies, triabodies, tetrabodies, minibodies, linear antibodies (Zapata et al., Protein Eng.,8 (10): 1057-1062 (1995)); chelating recombinant antibodies, tribodies or bibodies, intrabodies, nanobodies, small modular immunopharmaceuticals (SMIPs), an antigen-binding-domain immunoglobulin fusion protein, a camelized antibody, a VHH containing antibody, or muteins or derivatives thereof, and polypeptides that contain at least a portion of an immunoglobulin that is sufficient to confer specific antigen binding to the polypeptide, such as a CDR sequence, as long as the antibody retains the desired biological activity; and multispecific antibodies such as bi- and tri-specific antibodies formed from antibody fragments (C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). An antibody other than a "bispecific" or "bifunctional" antibody is understood to have each of its binding sites identical. The F(ab')2 or Fab may be engineered to minimize or completely remove the intermolecular disulfide interactions that occur between the CH1 and CL domains. Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two "Fv" fragments. An "Fv" fragment is the minimum antibody fragment that contains a complete antigen recognition and binding site. This region consists of a dimer of one heavy- and one light-chain variable domain in tight, non-covalent association. It is in this configuration that the three CDRs of each variable domain interact to define an antigen binding site on the surface of the VH-VL dimer. Collectively, the six CDRs confer antigen-binding specificity to the antibody. However, even a single variable domain (or half of an Fv comprising only three CDRs specific for an antigen) has the ability to recognize and bind antigen.

"Single-chain Fv" or "sFv" or "scFv" antibody fragments comprise the VH and VL domains of antibody, wherein these domains are present in a single polypeptide chain.

Preferably, the Fv polypeptide further comprises a polypeptide linker between the VH and VL domains that enables the Fv to form the desired structure for antigen binding. For a review of Fvs see Pluckthun in The Pharmacology of Monoclonal Antibodies, vol. 113, Rosenburg and Moore eds., Springer-Verlag, New York, pp. 269-315 (1994).

The Fab fragment also contains the constant domain of the light chain and the first constant domain (CH1) of the heavy chain. Fab fragments differ from Fab' fragments by the addition of a few residues at the carboxyl terminus of the heavy chain CH1 domain including one or more cysteine residues from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteine residues between them.

The term "mutein" or "variant" can be used interchangeably and refers to an antibody or antigen-binding fragment that contains at least one amino acid substitution, deletion, or insertion in the variable region or the portion equivalent to the variable region, provided that the mutein or variant retains the desired binding affinity or biological activity. Variants of the antibodies or antigen-binding antibody fragments contemplated in the disclosure are molecules in which the binding activity of the antibody or antigen-binding antibody fragment is maintained.

A "humanized antibody" is generally defined as one that is (I) derived from a non-human source (e.g., a transgenic mouse which bears a heterologous immune system), which antibody is based on a human germline sequence; or (II) CDR-grafted, wherein the CDRs of the variable region are from a non-human origin, while one or more framework regions and/or part of the CDR sequence of the variable region are of human origin and typically the constant region (if any) is of human origin.

The term "humanized antibody" thus includes antibodies in which the variable region in either the heavy or light chain or both of a human antibody is altered by at least partial replacement of one or more CDRs from a non-human antibody of known specificity and, if necessary, by partial framework region replacement and sequence changing. In other words, an antibody in which one or more "donor" CDRs from a non-human antibody (such as mouse, rat, rabbit or non-human primate antibody) of known specificity is grafted into a human heavy or light chain framework region is referred to herein as a "humanized antibody." It may not be necessary to replace all of the CDRs with the complete CDRs from the donor variable domain to transfer the antigen binding capacity of one variable domain to another. Rather, it may only be necessary to transfer those residues that are necessary to maintain the activity of the target binding site.

The framework regions (FR) within the variable region in a heavy or light chain, or both, of a humanized antibody may comprise solely residues of human origin, in which case these framework regions of the humanized antibody are referred to as "fully human framework regions." A human framework region that comprises a mixture of human and donor framework residues,and is referred to herein as a "partially human framework region." Furthermore, humanized antibodies may comprise residues that are neither found in the recipient antibody nor in the donor antibody. These modifications are made to further refine antibody performance (e.g., to obtain desired affinity).

In general, the humanized antibody will thus comprise at least one, and typically two, variable regions, in which all or part of the CDRs correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details see Jones et al., Nature 331:522-25 (1986); Riechmann et al., Nature 332:323-27 (1988); and Presta, Curr. Opin. Struct. Biol. 2:593-96 (1992), each incorporated herein by reference.

"Human antibodies" or "fully human antibodies" comprise human derived CDRs, i.e. CDRs of human origin. A "human" antibody is hereby defined as one that is not chimeric or "humanized" and not from (either in whole or in part) a non-human species. A human antibody or functional antibody fragment can be derived from a human or can be a synthetic human antibody. A "synthetic human antibody" is defined herein as an antibody having a sequence derived, in whole or in part, in silico from synthetic sequences that are based on the analysis of known human antibody sequences. In silico design of a human antibody sequence or fragment thereof can be achieved, for example, by analyzing a database of human antibody or antibody fragment sequences and devising an amino acid sequence utilizing the data obtained therefrom. Another example of a human antibody or functional antibody fragment is one that is encoded by a nucleic acid isolated from a library of antibody sequences of human origin (i.e., such library being based on antibodies taken from a human natural source).

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the term "monoclonal" indicates the character of the antibody as not being a mixture of discrete antibodies. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins. The term "monoclonal" is not to be construed as to require production of the antibody by any particular method. For example, the monoclonal antibodies to be used may be made by the hybridoma method first described by Kohler et al., Nature, 256: 495 [1975, or may be made by recombinant DNA methods (see, e.g., U.S. Patent No. 4,816,567). The "monoclonal antibodies" may also be recombinant, chimeric, humanized, human, Human Engineered^{™}, or antibody fragments, for example.

An "isolated" antibody is one that has been identified and separated from a component of the cell that expressed it. Contaminant components of the cell are materials that would interfere with diagnostic or therapeutic uses of the antibody, and may include enzymes, hormones, and other proteinaceous or non-proteinaceous solutes.

An "isolated" nucleic acid is one that has been identified and separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

As used herein, an antibody "binds specifically to", is "specific to/for" or "specifically recognizes" an antigen of interest, e.g. CEACAM5, is one that binds the antigen-target with sufficient affinity such that the antibody is useful as a therapeutic agent in targeting a cell or tissue expressing the antigen or one that binds an antigen-binding polypeptide target with sufficient affinity such that the antibody is useful as a reversal agent to neutralize the therapeutic activity of this antigen-binding polypeptide (e.g. an antigen-binding antibody) and does not significantly cross-react with other proteins or does not significantly cross-react with proteins other than orthologs and variants (e.g. mutant forms, splice variants, or proteolytically truncated forms) of the aforementioned target. The term "specifically recognizes" or "binds specifically to" or is "specific to/for" a particular polypeptide or an epitope on a particular polypeptide target as used herein can be exhibited, for example, by an antibody, or antigen-binding fragment thereof, having a monovalent dissociation constant (KD) for the antigen of less than about 10-4 M, alternatively less than about 10-5 M, alternatively less than about 10-6 M, alternatively less than about 10-7 M, alternatively less than about 10-8 M, alternatively less than about 10-9 M, alternatively less than about 10-10 M, alternatively less than about 10-11 M, alternatively less than about 10-12 M, or less. An antibody "binds specifically to," is "specific to/for" or "specifically recognizes" an antigen if such antibody is able to discriminate between such antigen and one or more reference antigen(s). In its most general form, "specific binding", "binds specifically to", is "specific to/for" or "specifically recognizes" is referring to the ability of the antibody to discriminate between the antigen of interest and an unrelated antigen, as determined, for example, in accordance with one of the following methods. Such methods comprise, but are not limited to, surface plasmon resonance (SPR), Western blots, ELISA-, RIA-, ECL-, IRMA- tests and peptide scans. For example, a standard ELISA assay can be carried out. The scoring may be carried out by standard color development (e.g., secondary antibody with horseradish peroxidase and tetramethyl benzidine with hydrogen peroxide). The reaction in certain wells is scored by the optical density, for example, at 450 nm. Typical background (=negative reaction) may be 0.1 OD; typical positive reaction may be 1 OD. This means the difference positive/negative is more than 5-fold, 10-fold, 50-fold, and preferably more than 100-fold. Typically, determination of binding specificity is performed by using not a single reference antigen, but a set of about three to five unrelated antigens, such as milk powder, BSA, transferrin or the like.

"Binding affinity" or "affinity" refers to the strength of the total sum of non-covalent interactions between a single binding site of a molecule and its binding partner. Unless indicated otherwise, as used herein, "binding affinity" refers to intrinsic binding affinity which reflects a 1:1 interaction between members of a binding pair (e.g., an antibody and an antigen). The affinity of an antibody or fragment thereof for a target can be determined using techniques well known in the art, for example by ELISA, isothermal titration calorimetry (ITC), surface plasmon resonance (SPR), flow cytometry or fluorescent polarization assays. The dissociation constant "KD" is commonly used to describe the affinity between a molecule (such as an antibody) and its binding partner (such as an antigen) i.e., how tightly a ligand binds to a particular protein. Ligand-protein affinities are influenced by non-covalent intermolecular interactions between the two molecules. Affinity can be measured by common methods known in the art, including those described herein. Preferably the affinity is provided as dissociation constant KD, in the alternative the affinity is provided as an EC50 value. In one embodiment, the "KD" or "KD value" according to this disclosure is measured by using surface plasmon resonance assays a Biacore T200 instrument (Cytiva). Other suitable devices are Biacore X100, Biacore S200, Biacore 8K, 30 Biacore 8K+, Biacore 4000 SPR (Cytiva), Alto Digital SPR (Nicoya Lifesciences Inc.), or SIERRA SPR-32 PRO (Bruker Corporation).

"Half maximal effective concentration" (EC50) refers to the concentration of a drug, modulator, antibody, fragment, conjugate or molecule which induces a response halfway between the baseline and maximum after a specified incubation time. In the context of affinity, the EC50 thus reflects the concentration of binder (e.g. antibody) that is needed for half-maximal binding. An EC50 can be determined if an inflection point can be determined by mathematical modeling (e.g., non-linear regression) of the dose-response curve describing the relationship between applied drug, antibody, fragment, conjugate or molecule concentration and signal. For example, if the dose-response curve follows a sigmoidal curve, an EC50 can be determined. Where the response is an inhibition, the EC50 is termed "half maximal inhibitory concentration" (IC50).

As used herein, the term "epitope" includes any protein determinant capable of specific binding to an immunoglobulin or T-cell receptor. Epitopic determinants usually consist of chemically active surface groupings of molecules such as amino acids or sugar side chains, or combinations thereof and usually have specific three-dimensional structural characteristics, as well as specific charge characteristics.

The term "maturated antibodies" or "maturated antigen-binding fragments" such as maturated Fab variants or "optimized" variants includes derivatives of an antibody or antibody fragment exhibiting stronger binding - i. e. binding with increased affinity - to a given antigen such as the extracellular domain of a target protein. Maturation is the process of identifying a small number of mutations within the six CDRs of an antibody or antibody fragment leading to this affinity increase. The maturation process is the combination of molecular biology methods for introduction of mutations into the antibody and screening for identifying the improved binders.

The term "antibody conjugate" refers to a conjugate comprising at least one antibody moiety and one or more further molecules or moieties. The one or more further molecules or moieties may be selected without limitation from a drug, a chelator, a radioactive element, a cytotoxic agent, a further antibody or antigen-binding fragment.

The term "antibody drug conjugate" ("ADC") refers to an antibody conjugate comprising at least one drug moiety.

The term "germlining" refers to replacement of residues in the variable domains of an antibody with those present in the pre-mutated germline genes to reduce the potential for immunogenicity.

"Antibody-dependent cell-mediated cytotoxicity" or "ADCC" refers to a form of cytotoxicity in which secreted Ig bound onto Fc gamma receptors (FcyRs) present on certain cytotoxic cells (e.g. NK cells, neutrophils, and macrophages) enable these cytotoxic effector cells to bind specifically to an antigen-bearing target cell and subsequently kill the target cell e.g. with cytotoxins. To assess ADCC activity of an antibody of interest, an in vitro ADCC assay, such as that described in US Patent No. 5,500,362 or 5,821,337 or U.S. Patent No. 6,737,056 (Presta), may be performed. Useful effector cells for such assays include PBMC and NK cells.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, e.g., as described in Gazzano-Santoro et al., J. Immunol. Methods 202: 163 (1996), may be performed. Polypeptide variants with altered Fc region amino acid sequences (polypeptides with a variant Fc region) and increased or decreased C1q binding are described, e.g., in US Patent No. 6,194,551 B1 and WO 1999/51642.

"Percent (%) sequence identity" with respect to a reference polynucleotide or polypeptide sequence, respectively, is defined as the percentage of nucleic acid or amino acid residues, respectively, in a candidate sequence that are identical with the nucleic acid or amino acid residues, respectively, in the reference polynucleotide or polypeptide sequence, respectively, after aligning the sequences and introducing gaps, if necessary, to achieve the maximum percent sequence identity. Conservative substitutions are not considered as part of the sequence identity. Preferred are un-gapped alignments. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full-length of the sequences being compared.

Substantial sequence identity/similarity may be taken as having a sequence similarity/identity of at least 80% to the complete sequences and/or at least 90% to the specific binding regions (e.g., the CDR regions). Preferable sequence similarity or more preferably identity may be at least 92%, 95%, 97%, 98% or 99%. Sequence similarity and/or identity may be determined using the "BestFit" program of the Genetics Computer Group Version 10 software package from the University of Wisconsin. The program uses the local had algorithm of Smith and Waterman with default values: Gap creation penalty=8, Gap extension penalty=2, Average match=2.912, average mismatch 2.003.

The terms "polynucleotide" or "nucleic acid", as used interchangeably herein, refer to chains of nucleotides of any length, and include DNA and RNA. The nucleotides can be deoxyribonucleotides, ribonucleotides, modified nucleotides or bases, and/or their analogs, or any substrate that can be incorporated into a chain by DNA or RNA polymerase. A polynucleotide may comprise modified nucleotides, such as methylated nucleotides and their analogs.

"Sequence homology" indicates the percentage of amino acids that either is identical or that represent conservative amino acid substitutions.

The term "vector", as used herein, refers to a nucleic acid molecule capable of propagating another nucleic acid to which it is linked. The term includes the vector as a self- replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The terms "host cell", "host cell line", and "host cell culture" are used interchangeably and refer to cells into which at least one exogenous nucleic acid has been introduced, including the progeny of such cells. Host cells include "transformants" and "transformed cells", "transfectants" and "transfected cells" and "transduced cells" which include the primary transformed/transfected/transduced cell and progeny derived therefrom without regard to the number of passages. Progeny may not be completely identical in nucleic acid content to a parent cell but may contain mutations. Mutant progeny that have the same function or biological activity as screened or selected for in the originally transformed cell are included herein.

As used herein, the phrase "therapeutically effective amount" is meant to refer to an amount of therapeutic or prophylactic antibody that would be appropriate to elicit the desired therapeutic or prophylactic effect or response, including alleviating some or all of such symptoms of disease or reducing the predisposition to the disease, when administered in accordance with the desired treatment regimen.

The term "pharmaceutical formulation" / "pharmaceutical composition" refers to a preparationwhich is in such form as to permit the biological activity of an active ingredient contained therein to be effective, and which contains no additional components which are unacceptably toxic to a subject to which the formulation would be administered.

"Binding proteins" contemplated in the disclosure are for example antibody mimetics, such as Affibodies, Adnectins, Anticalins, DARPins, Avimers, Nanobodies (reviewed by Gebauer M. et al., Curr. Opinion in Chem. Biol. 2009; 13:245-255; Nuttall S.D. et al., Curr. Opinion in Pharmacology 2008; 8:608-617).

The present disclosure comprises combinations comprising a tissue-targeting compound of formula (I) as described herein and one or more further compounds having biological activity (i.e., a further pharmaceutical agent), preferably against cancer, especially colorectal cancer (CRC), non-small-cell lung cancer (NSCLC), gastric cancer (GAC), neuroendocrine cancer (NENC) and pancreatic cancer (PAC), as well as breast, liver and thyroid cancer. These compounds are defined by their classes, thus allowing a skilled person to identify such compounds belonging to said class.

In the context of the present disclosure, external beam radiotherapy refers to radiation therapy in which an external source of ionizing radiation is pointed at and/or applied to a particular part of the body of a subject.

In the context of the present disclosure, the term "combination" means not only a dosage form which contains all the components (i.e., the tissue-targeting compound of formula (I) and a further pharmaceutical agent, also called fixed-dose combinations), and combination packs containing the components separate from one another, but also components which are administered simultaneously or sequentially, as long as they are employed for the prophylaxis or treatment of the same disease.

The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

### DESCRIPTION

### Antibodies

According to a first aspect, the present disclosure relates to isolated antibodies or antigen-binding fragments thereof capable of binding to CEACAM5, i.e., to isolated antibodies or antigen- binding fragments thereof having binding affinity for CEACAM5. In the context of the present disclosure and particularly when referring to isolated antibodies or antigen- binding fragments thereof, the terms "capable of binding to CEACAM5" and "having binding affinity for CEACAM5" can be used interchangeably.

The antibodies can be, for example, IgG antibodies, such as a human IgG1, IgG2, IgG3, or IgG4, or a mouse IgG1, IgG2a, IgG2b or IgG2c. In some highly preferred embodiments, the isolated antibodies or antigen-binding fragment thereof according to the current aspect are humanized IgG1 antibodies. In some further preferred embodiments, the antibodies or antigen-binding fragments thereof according to the current aspect are a Fab', F(ab')2, Fab, Fv, rIgG, or scFv fragments.

In some preferred embodiments, the antibodies or antigen-binding fragments thereof according to the current aspect is binding to human CEACAM5. In some of these preferred embodiments the antibodies or antigen-binding fragments thereof according to the current aspect cross-react with CEACAM5 from other species e.g. monkey, macaca fascicularis (cynomolgus monkey), macaca mulatta (rhesus macaque), rodent, mouse, rat, horse, bovine, pig, dog, cat and/or camel CEACAM5, preferable with cynomolgus CEACAM5.

According to some preferred embodiments, the antibodies or antigen-binding fragments have a binding affinity KD for human CEACAM5 of SEQ ID 145 which is below 2E-07 M, preferably below 1E-08 M, 1E-09 M, 10E-10 M or 1E-10 M. According to some embodiments, which may be the same or different, the antibodies or antigen-binding fragments have a binding affinity KD to cynomolgus CEACAM5 of SEQ ID 145 which is below 2E-07 M, preferably below 1E-08 M, 1E-09 M, 10E-10 M or 1E-10 M.

Preferably, the antibodies or antigen-binding fragments bind human and cynomolgus CEACAM5 with a KD in the same order of magnitude, e.g. with a KD below 2E-07 M, preferably below 1E- 08 M, 1E-09 M, 10E-10 M or 1E-10 M.

In further preferred embodiments, the antibodies or antigen-binding fragments thereof bind to human CEACAM5 of SEQ ID 145 with an EC50 which is below 1E-07 M preferably below 10 E-08 M, 1E-08 M, 10E-9 M, or 1E-09 M. According to some embodiments, which may be the same or different, the antibodies or antigen-binding fragments have a EC50 to cynomolgus CEACAM5 of SEQ ID 323 which is below 2E-07 M, preferably below 10 E-08 M, 1E-08 M, 10E-9 M, or 1E- 09 M.

Preferably, the antibodies or antigen-binding fragments bind human and cynomolgus CEACAM5 with a EC50 in the same order of magnitude, e.g. with a EC50 below 2E-07 M, preferably below 10 E-08 M, 1E-08 M, 10E-9 M, or 1E-09 M.

In some preferred embodiments the antibodies or antigen-binding fragments thereof according to the current aspect have a low number of germline deviations from the closest germline light chain IGKV1-33*01/IGKJ2*01 and the closest germline heavy chain identified outside IGHV3-7*05/IGHJ5*01 which is below 32 preferably below 30, 25, 20, 19, 18, 17, 16, 15, 14, 13, 12, 11, 10, 5, 4, 3, 2, or 1.

In further preferred embodiments the antibodies or antigen-binding fragments thereof according to the current aspect have a low risk for immunogenicity as reflected by an IPAD score below 1000, preferably below 900, 800, 700, 600, or 550.

In further preferred embodiments the antibodies or antigen-binding fragments thereof according to the current aspect comprise a low number of lysine residues within their CDRs. Preferably the number of lysine residues within the six CDRs of the antibodies or antigen-binding fragments thereof is below 4, more preferably below 3, even more preferably below 2, and most preferably below 1. According to a highly preferred embodiment the antibodies or antigen-binding fragments thereof according to the current aspect comprise no lysine residue within their six CDRs.

The isolated antibodies or antigen-binding fragments according to the present disclosure may exhibit any combination of the above-described characteristics.

In some preferred embodiments, the isolated antibodies or antigen-binding fragments according to the present disclosure comprise:
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291), or
x) a heavy chain antigen-binding region that comprises an H-CDR1 comprising SEQ ID NO: 91, an H-CDR2 comprising SEQ ID NO: 92, and an H-CDR3 comprising SEQ ID NO: 93 and a light chain antigen-binding region that comprises an L-CDR1 comprising SEQ ID NO: 96, an L-CDR2 comprising SEQ ID NO: 97, and an L-CDR3 comprising SEQ ID NO:98 (DPP-64106), or
xi) a heavy chain antigen-binding region that comprises an H-CDR1 comprising SEQ ID NO: 100, an H-CDR2 comprising SEQ ID NO: 101, and an H-CDR3 comprising SEQ ID NO: 102 and a light chain antigen-binding region that comprises an L-CDR1 comprising SEQ ID NO: 105, an L-CDR2 comprising SEQ ID NO: 106, and an L-CDR3 comprising SEQ ID NO:107 (DPP-64107), or
xii) a heavy chain antigen-binding region that comprises an H-CDR1 comprising SEQ ID NO: 109, an H-CDR2 comprising SEQ ID NO: 110 and an H-CDR3 comprising SEQ ID NO: 111 and a light chain antigen-binding region that comprises an L-CDR1 comprising SEQ ID NO: 114, an L-CDR2 comprising SEQ ID NO: 115, and an L-CDR3 comprising SEQ ID NO:116 (DPP-64204), or
xiii) a heavy chain antigen-binding region that comprises an H-CDR1 comprising SEQ ID NO: 118, an H-CDR2 comprising SEQ ID NO: 119, and an H-CDR3 comprising SEQ ID NO: 120 and a light chain antigen-binding region that comprises an L-CDR1 comprising SEQ ID NO: 123, an L-CDR2 comprising SEQ ID NO: 124, and an L-CDR3 comprising SEQ ID NO:125 (DPP-64209), or
xiv) a heavy chain antigen-binding region that comprises an H-CDR1 comprising SEQ ID NO: 127, an H-CDR2 comprising SEQ ID NO: 128, and an H-CDR3 comprising SEQ ID NO: 129 and a light chain antigen-binding region that comprises an L-CDR1 comprising SEQ ID NO: 132, an L-CDR2 comprising SEQ ID NO: 133, and an L-CDR3 comprising SEQ ID NO:134 (DPP-64227), or
xv) a heavy chain antigen-binding region that comprises an H-CDR1 comprising SEQ ID NO: 136, an H-CDR2 comprising SEQ ID NO: 137, and an H-CDR3 comprising SEQ ID NO: 138 and a light chain antigen-binding region that comprises an L-CDR1 comprising SEQ ID NO: 141, an L-CDR2 comprising SEQ ID NO: 142, and an L-CDR3 comprising SEQ ID NO:143 (DPP-64262).

In a preferred embodiment, the isolated antibodies or antigen-binding fragments according to the present disclosure comprise:
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291).

In further preferred embodiments the isolated antibodies or antigen-binding fragments according to the present disclosure comprise:
i) a variable heavy chain domain having at least 90 %, 95 %, 98 %, 99 % or 100 % sequence identity with SEQ ID NO: 4 and/or a variable light chain domain having at least 90 %, 95 %, 98 %, 99 % or 100 % sequence identity with SEQ ID NO: 9; or
ii) a variable heavy chain domain having at least 90 %, 95 %, 98 %, 99 % or 100 % sequence identity with SEQ ID NO: 14 and/or a variable light chain domain having at least 90 %, 95 %, 98 %, 99 % or 100 % sequence identity with SEQ ID NO: 19; or
iii) a variable heavy chain domain having at least 90 %, 95 %, 98 %, 99 % or 100 % sequence identity with SEQ ID NO: 24 and/or a variable light chain domain having at least 90 %, 95 %, 98 %, 99 % or 100 % sequence identity with SEQ ID NO: 29; or
iv) a variable heavy chain domain having at least 90 %, 95 %, 98 %, 99 % or 100 % sequence identity with SEQ ID NO: 34 and/or a variable light chain domain having at least 90 %, 95 %, 98 %, 99 % or 100 % sequence identity with SEQ ID NO: 39; or
v) a variable heavy chain domain having at least 90 %, 95 %, 98 %, 99 % or 100 % sequence identity with SEQ ID NO: 44 and/or a variable light chain domain having at least 90 %, 95 %, 98 %, 99 % or 100 % sequence identity with SEQ ID NO: 49; or
vi) a variable heavy chain domain having at least 90 %, 95 %, 98 %, 99 % or 100 % sequence identity with SEQ ID NO: 54 and/or a variable light chain domain having at least 90 %, 95 %, 98 %, 99 % or 100 % sequence identity with SEQ ID NO: 59; or
vii) a variable heavy chain domain having at least 90 %, 95 %, 98 %, 99 % or 100 % sequence identity with SEQ ID NO: 64 and/or a variable light chain domain having at least 90 %, 95 %, 98 %, 99 % or 100 % sequence identity with SEQ ID NO: 69; or
viii) a variable heavy chain domain having at least 90 %, 95 %, 98 %, 99 % or 100 % sequence identity with SEQ ID NO: 74 and/or a variable light chain domain having at least 90 %, 95 %, 98 %, 99 % or 100 % sequence identity with SEQ ID NO: 79, or
ix) a variable heavy chain domain having at least 90 %, 95 %, 98 %, 99 % or 100 % sequence identity with SEQ ID NO: 84 and/or a variable light chain domain having at least 90 %, 95 %, 98 %, 99 % or 100 % sequence identity with SEQ ID NO: 89; or
x) a variable heavy chain domain having at least 90 %, 95 %, 98 %, 99 % or 100 % sequence identity with SEQ ID NO: 94 and/or a variable light chain domain having at least 90 %, 95 %, 98 %, 99 % or 100 % sequence identity with SEQ ID NO: 99; or
xi) a variable heavy chain domain having at least 90 %, 95 %, 98 %, 99 % or 100 % sequence identity with SEQ ID NO: 103 and/or a variable light chain domain having at least 90 %, 95 %, 98 %, 99 % or 100 % sequence identity with SEQ ID NO: 108; or
xii) a variable heavy chain domain having at least 90 %, 95 %, 98 %, 99 % or 100 % sequence identity with SEQ ID NO: 112 and/or a variable light chain domain having at least 90 %, 95 %, 98 %, 99 % or 100 % sequence identity with SEQ ID NO: 117; or
xiii) a variable heavy chain domain having at least 90 %, 95 %, 98 %, 99 % or 100 % sequence identity with SEQ ID NO: 121 and/or a variable light chain domain having at least 90 %, 95 %, 98 %, 99 % or 100 % sequence identity with SEQ ID NO: 126; or
xiv) a variable heavy chain domain having at least 90 %, 95 %, 98 %, 99 % or 100 % sequence identity with SEQ ID NO: 130 and/or a variable light chain domain having at least 90 %, 95 %, 98 %, 99 % or 100 % sequence identity with SEQ ID NO: 135; or
xv) a variable heavy chain domain having at least 90 %, 95 %, 98 %, 99 % or 100 % sequence identity with SEQ ID NO: 139 and/or a variable light chain domain having at least 90 %, 95 %, 98 %, 99 % or 100 % sequence identity with SEQ ID NO: 144.

In a preferred embodiment the isolated antibodies or antigen-binding fragments according to the present disclosure comprise:
i) a variable heavy chain domain having at least 90 %, 95 %, 98 %, 99 % or 100 % sequence identity with SEQ ID NO: 4 and/or a variable light chain domain having at least 90 %, 95 %, 98 %, 99 % or 100 % sequence identity with SEQ ID NO: 9; or
ii) a variable heavy chain domain having at least 90 %, 95 %, 98 %, 99 % or 100 % sequence identity with SEQ ID NO: 14 and/or a variable light chain domain having at least 90 %, 95 %, 98 %, 99 % or 100 % sequence identity with SEQ ID NO: 19; or
iii) a variable heavy chain domain having at least 90 %, 95 %, 98 %, 99 % or 100 % sequence identity with SEQ ID NO: 24 and/or a variable light chain domain having at least 90 %, 95 %, 98 %, 99 % or 100 % sequence identity with SEQ ID NO: 29; or
iv) a variable heavy chain domain having at least 90 %, 95 %, 98 %, 99 % or 100 % sequence identity with SEQ ID NO: 34 and/or a variable light chain domain having at least 90 %, 95 %, 98 %, 99 % or 100 % sequence identity with SEQ ID NO: 39; or
v) a variable heavy chain domain having at least 90 %, 95 %, 98 %, 99 % or 100 % sequence identity with SEQ ID NO: 44 and/or a variable light chain domain having at least 90 %, 95 %, 98 %, 99 % or 100 % sequence identity with SEQ ID NO: 49; or
vi) a variable heavy chain domain having at least 90 %, 95 %, 98 %, 99 % or 100 % sequence identity with SEQ ID NO: 54 and/or a variable light chain domain having at least 90 %, 95 %, 98 %, 99 % or 100 % sequence identity with SEQ ID NO: 59; or
vii) a variable heavy chain domain having at least 90 %, 95 %, 98 %, 99 % or 100 % sequence identity with SEQ ID NO: 64 and/or a variable light chain domain having at least 90 %, 95 %, 98 %, 99 % or 100 % sequence identity with SEQ ID NO: 69; or
viii) a variable heavy chain domain having at least 90 %, 95 %, 98 %, 99 % or 100 % sequence identity with SEQ ID NO: 74 and/or a variable light chain domain having at least 90 %, 95 %, 98 %, 99 % or 100 % sequence identity with SEQ ID NO: 79, or
ix) a variable heavy chain domain having at least 90 %, 95 %, 98 %, 99 % or 100 % sequence identity with SEQ ID NO: 84 and/or a variable light chain domain having at least 90 %, 95 %, 98 %, 99 % or 100 % sequence identity with SEQ ID NO: 89.

In further preferred embodiments, the isolated antibodies or antigen-binding fragments according to the present disclosure comprise:
i) a variable heavy chain comprising SEQ ID NO: 4 and/or a variable light chain domain comprising SEQ ID NO: 9; or
ii) a variable heavy chain domain comprising SEQ ID NO: 14 and/or a variable light chain domain comprising SEQ ID NO: 19; or
iii) a variable heavy chain domain comprising SEQ ID NO: 24 and/or a variable light chain domain comprising SEQ ID NO: 29; or
iv) a variable heavy chain domain comprising SEQ ID NO: 34 and/or a variable light chain domain comprising SEQ ID NO: 39; or
v) a variable heavy chain domain comprising SEQ ID NO: 44 and/or a variable light chain domain comprising SEQ ID NO: 49; or
vi) a variable heavy chain domain comprising SEQ ID NO: 54 and/or a variable light chain domain comprising SEQ ID NO: 59; or
vii) a variable heavy chain domain comprising SEQ ID NO: 64 and/or a variable light chain domain comprising SEQ ID NO: 69; or
viii) a variable heavy chain domain comprising SEQ ID NO: 74 and/or a variable light chain domain comprising SEQ ID NO: 79, or
ix) a variable heavy chain domain comprising SEQ ID NO: 84 and/or a variable light chain domain comprising SEQ ID NO: 89; or
x) a variable heavy chain domain comprising SEQ ID NO: 94 and/or a variable light chain domain comprising SEQ ID NO: 99; or
xi) a variable heavy chain domain comprising SEQ ID NO: 103 and/or a variable light chain domain comprising SEQ ID NO: 108; or
xii) a variable heavy chain domain comprising SEQ ID NO: 112 and/or a variable light chain domain comprising SEQ ID NO: 117; or
xiii) a variable heavy chain domain comprising SEQ ID NO: 121 and/or a variable light chain domain comprising SEQ ID NO: 126; or
xiv) a variable heavy chain domain comprising SEQ ID NO: 130 and/or a variable light chain domain comprising SEQ ID NO: 135; or
xv) a variable heavy chain domain comprising SEQ ID NO: 139 and/or a variable light chain domain comprising SEQ ID NO: 144.

In a preferred embodiment, the isolated antibodies or antigen-binding fragments according to the present disclosure comprise:
i) a variable heavy chain comprising SEQ ID NO: 4 and/or a variable light chain domain comprising SEQ ID NO: 9; or
ii) a variable heavy chain domain comprising SEQ ID NO: 14 and/or a variable light chain domain comprising SEQ ID NO: 19; or
iii) a variable heavy chain domain comprising SEQ ID NO: 24 and/or a variable light chain domain comprising SEQ ID NO: 29; or
iv) a variable heavy chain domain comprising SEQ ID NO: 34 and/or a variable light chain domain comprising SEQ ID NO: 39; or
v) a variable heavy chain domain comprising SEQ ID NO: 44 and/or a variable light chain domain comprising SEQ ID NO: 49; or
vi) a variable heavy chain domain comprising SEQ ID NO: 54 and/or a variable light chain domain comprising SEQ ID NO: 59; or
vii) a variable heavy chain domain comprising SEQ ID NO: 64 and/or a variable light chain domain comprising SEQ ID NO: 69; or
viii) a variable heavy chain domain comprising SEQ ID NO: 74 and/or a variable light chain domain comprising SEQ ID NO: 79, or
ix) a variable heavy chain domain comprising SEQ ID NO: 84 and/or a variable light chain domain comprising SEQ ID NO: 89.

In further preferred embodiments the isolated antibodies or antigen-binding fragments according to the present disclosure comprise:
i) a heavy chain having at least 90 %, 95 %, 98 %, 99 % or 100 % sequence identity with SEQ ID NO: 5 and/o a light chain having at least 90 %, 95 %, 98 %, 99 % or 100 % sequence identity with SEQ ID NO: 10; or
ii) a heavy chain having at least 90 %, 95 %, 98 %, 99 % or 100 % sequence identity with SEQ ID NO: 15 and/or a light chain having at least 90 %, 95 %, 98 %, 99 % or 100 % sequence identity with SEQ ID NO: 20; or
iii) a heavy chain having at least 90 %, 95 %, 98 %, 99 % or 100 % sequence identity with SEQ ID NO: 25 and/or a light chain having at least 90 %, 95 %, 98 %, 99 % or 100 % sequence identity with SEQ ID NO: 30; or
iv) a heavy chain having at least 90 %, 95 %, 98 %, 99 % or 100 % sequence identity with SEQ ID NO: 35 and/or a light chain having at least 90 %, 95 %, 98 %, 99 % or 100 % sequence identity with SEQ ID NO: 40; or
v) a heavy chain having at least 90 %, 95 %, 98 %, 99 % or 100 % sequence identity with SEQ ID NO: 45 and/or a light chain having at least 90 %, 95 %, 98 %, 99 % or 100 % sequence identity with SEQ ID NO: 50 or
vi) a heavy chain having at least 90 %, 95 %, 98 %, 99 % or 100 % sequence identity with SEQ ID NO: 55 and/or a light chain having at least 90 %, 95 %, 98 %, 99 % or 100 % sequence identity with SEQ ID NO: 60, or
vii) a heavy chain having at least 90 %, 95 %, 98 %, 99 % or 100 % sequence identity with SEQ ID NO: 65 and/or a light chain having at least 90 %, 95 %, 98 %, 99 % or 100 % sequence identity with SEQ ID NO: 70; or
viii) a heavy chain having at least 90 %, 95 %, 98 %, 99 % or 100 % sequence identity with SEQ ID NO: 75 and/or a light chain having at least 90 %, 95 %, 98 %, 99 % or 100 % sequence identity with SEQ ID NO: 80; or
ix) a heavy chain having at least 90 %, 95 %, 98 %, 99 % or 100 % sequence identity with SEQ ID NO: 85 and/or a light chain having at least 90 %, 95 %, 98 %, 99 % or 100 % sequence identity with SEQ ID NO: 90 or
x) a heavy chain having at least 90 %, 95 %, 98 %, 99 % or 100 % sequence identity with SEQ ID NO: 95, or
xi) a heavy chain having at least 90 %, 95 %, 98 %, 99 % or 100 % sequence identity with SEQ ID NO: 104, or
xii) a heavy chain having at least 90 %, 95 %, 98 %, 99 % or 100 % sequence identity with SEQ ID NO: 113; or
xiii) a heavy chain having at least 90 %, 95 %, 98 %, 99 % or 100 % sequence identity with SEQ ID NO: 122; or
xiv) a heavy chain having at least 90 %, 95 %, 98 %, 99 % or 100 % sequence identity with SEQ ID NO: 131; or
xv) a heavy chain having at least 90 %, 95 %, 98 %, 99 % or 100 % sequence identity with SEQ ID NO: 140.

In a preferred embodiment the isolated antibodies or antigen-binding fragments according to the present disclosure comprise:
i) a heavy chain having at least 90 %, 95 %, 98 %, 99 % or 100 % sequence identity with SEQ ID NO: 5 and/o a light chain having at least 90 %, 95 %, 98 %, 99 % or 100 % sequence identity with SEQ ID NO: 10; or
ii) a heavy chain having at least 90 %, 95 %, 98 %, 99 % or 100 % sequence identity with SEQ ID NO: 15 and/or a light chain having at least 90 %, 95 %, 98 %, 99 % or 100 % sequence identity with SEQ ID NO: 20; or
iii) a heavy chain having at least 90 %, 95 %, 98 %, 99 % or 100 % sequence identity with SEQ ID NO: 25 and/or a light chain having at least 90 %, 95 %, 98 %, 99 % or 100 % sequence identity with SEQ ID NO: 30; or
iv) a heavy chain having at least 90 %, 95 %, 98 %, 99 % or 100 % sequence identity with SEQ ID NO: 35 and/or a light chain having at least 90 %, 95 %, 98 %, 99 % or 100 % sequence identity with SEQ ID NO: 40; or
v) a heavy chain having at least 90 %, 95 %, 98 %, 99 % or 100 % sequence identity with SEQ ID NO: 45 and/or a light chain having at least 90 %, 95 %, 98 %, 99 % or 100 % sequence identity with SEQ ID NO: 50 or
vi) a heavy chain having at least 90 %, 95 %, 98 %, 99 % or 100 % sequence identity with SEQ ID NO: 55 and/or a light chain having at least 90 %, 95 %, 98 %, 99 % or 100 % sequence identity with SEQ ID NO: 60, or
vii) a heavy chain having at least 90 %, 95 %, 98 %, 99 % or 100 % sequence identity with SEQ ID NO: 65 and/or a light chain having at least 90 %, 95 %, 98 %, 99 % or 100 % sequence identity with SEQ ID NO: 70; or
viii) a heavy chain having at least 90 %, 95 %, 98 %, 99 % or 100 % sequence identity with SEQ ID NO: 75 and/or a light chain having at least 90 %, 95 %, 98 %, 99 % or 100 % sequence identity with SEQ ID NO: 80; or
ix) a heavy chain having at least 90 %, 95 %, 98 %, 99 % or 100 % sequence identity with SEQ ID NO: 85 and/or a light chain having at least 90 %, 95 %, 98 %, 99 % or 100 % sequence identity with SEQ ID NO: 90.

In further preferred embodiments the isolated antibodies or antigen-binding fragments according to the present disclosure comprise:
i) a heavy chain comprising SEQ ID NO: 5 and/or a light chain comprising SEQ ID NO: 10; or
ii) a heavy chain comprising SEQ ID NO: 15 and/or a light chain comprising SEQ ID NO: 20; or
iii) a heavy chain comprising SEQ ID NO: 25 and/or a light chain comprising SEQ ID NO: 30; or
iv) a heavy chain comprising SEQ ID NO: 35 and/or a light chain comprising SEQ ID NO: 40; or
v) a heavy chain comprising SEQ ID NO: 45 and/or a light chain comprising SEQ ID NO: 50 or
vi) a heavy chain comprising SEQ ID NO: 55 and/or a light chain comprising SEQ ID NO: 60, or
vii) a heavy chain comprising SEQ ID NO: 65 and/or a light chain comprising SEQ ID NO: 70; or
viii) a heavy chain comprising SEQ ID NO: 75 and/or a light chain comprising SEQ ID NO: 80; or
ix) a heavy chain comprising SEQ ID NO: 85 and/or a light chain comprising SEQ ID NO: 90 or
x) a heavy chain comprising SEQ ID NO: 95; or
xi) a heavy chain comprising SEQ ID NO: 104; or
xii) a heavy chain comprising SEQ ID NO: 113; or
xiii) a heavy chain comprising SEQ ID NO: 122; or
xiv) a heavy chain comprising SEQ ID NO: 131; or
xv) a heavy chain comprising SEQ ID NO: 140.

In a preferred embodiment the isolated antibodies or antigen-binding fragments according to the present disclosure comprise:
i) a heavy chain comprising SEQ ID NO: 5 and/or a light chain comprising SEQ ID NO: 10; or
ii) a heavy chain comprising SEQ ID NO: 15 and/or a light chain comprising SEQ ID NO: 20; or
iii) a heavy chain comprising SEQ ID NO: 25 and/or a light chain comprising SEQ ID NO: 30; or
iv) a heavy chain comprising SEQ ID NO: 35 and/or a light chain comprising SEQ ID NO: 40; or
v) a heavy chain comprising SEQ ID NO: 45 and/or a light chain comprising SEQ ID NO: 50 or
vi) a heavy chain comprising SEQ ID NO: 55 and/or a light chain comprising SEQ ID NO: 60, or
vii) a heavy chain comprising SEQ ID NO: 65 and/or a light chain comprising SEQ ID NO: 70; or
viii) a heavy chain comprising SEQ ID NO: 75 and/or a light chain comprising SEQ **ID** NO: 80; or
ix) a heavy chain comprising SEQ ID NO: 85 and/or a light chain comprising SEQ ID NO: 90.

In particular embodiments, the isolated antibodies according to the present disclosure are IgG1, IgG2, IgG3 or an IgG4 antibodies. Most particularly, the isolated antibodies according to the present disclosure are IgG1 antibodies.

In particular embodiments, the antigen-binding fragments according to the present disclosure are scFv, Fab, Fab' fragment or a F(ab')2 fragments.

In particularly preferred embodiments, the antigen-binding fragments according to the present disclosure are Fab', F(ab')2, Fab, Fv, rIgG, VHH (sdAb), VHH-Fc or scFv fragments.

In particular embodiments, the isolated antibodies or antigen-binding fragments according to the present disclosure are monoclonal antibodies or antigen-binding fragments.

In particular embodiments, the isolated antibodies or antigen-binding fragments according to the present disclosure are human, humanized or chimeric antibodies or antigen-binding fragments, more particularly humanized antibodies or antigen-binding fragments.

In particular embodiments, the isolated antibodies or antigen-binding fragments according to the present disclosure are monospecific antibodies. In particular other embodiments, the isolated antibodies or antigen-binding fragments according to the present disclosure are multispecific antibodies that bind to CEACAM5 and at least one further antigen, such bispecific, trispecific or tetraspecific antibodies.

Amino acid sequences of preferred antibodies according to the present disclosure are listed in Table 1.

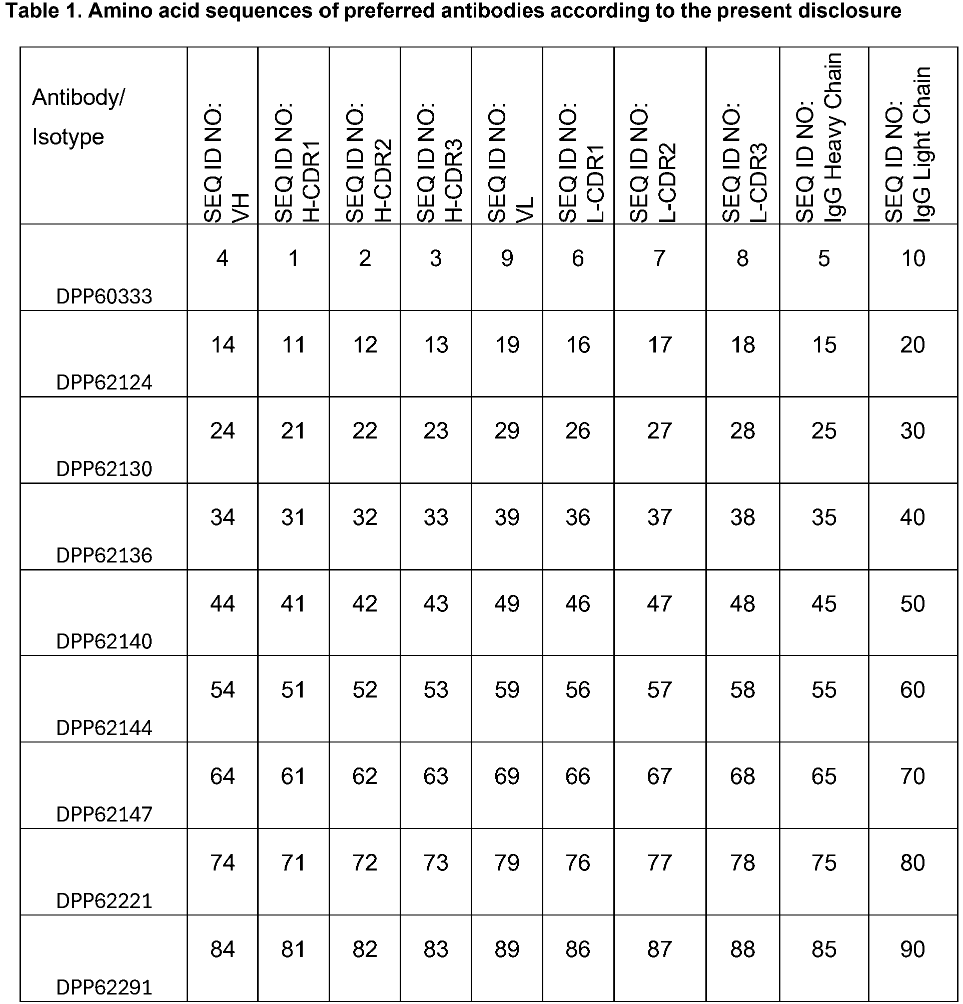

### Peptide variants

Antibodies or antigen-binding fragments of the disclosure are not limited to the specific peptide sequences provided herein. Rather, the disclosure also embodies variants of these polypeptides. With reference to the instant disclosure and conventionally available technologies and references, the skilled worker will be able to prepare, test and utilize functional variants of the antibodies disclosed herein, while appreciating these variants having the ability to bind to CEACAM5 fall within the scope of the present disclosure.

A variant can include, for example, an antibody that has at least one altered complementary determining region (CDR) (hyper-variable) and/or framework (FR) (variable) domain/position, vis-à-vis a peptide sequence disclosed herein.

By altering one or more amino acid residues in a CDR or FR region, the skilled worker routinely can generate mutated or diversified antibody sequences, which can be screened against the antigen, for new or improved properties, for example.

A further preferred embodiment of the disclosure is an antibody or antigen-binding fragment in which the VH and VL sequences are selected as shown in Table 1. The skilled worker can use the data in Table 1 to design peptide variants that are within the scope of the present disclosure. It is preferred that variants are constructed by changing amino acids within one or more CDR regions; a variant might also have one or more altered framework regions. Alterations also may be made in the framework regions. For example, a peptide FR domain might be altered where there is a deviation in a residue compared to a germline sequence.

Alternatively, the skilled worker could make the same analysis by comparing the amino acid sequences disclosed herein to known sequences of the same class of such antibodies, using, for example, the procedure described by Knappik A., et al., JMB 2000, 296:57-86.

Furthermore, variants may be obtained by using one antibody as starting point for further optimization by diversifying one or more amino acid residues in the antibody, preferably amino acid residues in one or more CDRs, and by screening the resulting collection of antibody variants for variants with improved properties. Particularly preferred is diversification of one or more amino acid residues in CDR3 of VL and/or VH. Diversification can be done e.g. by synthesizing a collection of DNA molecules using trinucleotide mutagenesis (TRIM) technology (Virnekäs B. et al., Nucl. Acids Res. 1994, 22: 5600.). Antibodies or antigen-binding fragments thereof include molecules with modifications/variations including but not limited to e.g. modifications leading to altered half-life (e.g. modification of the Fc part or attachment of further molecules such as PEG), altered binding affinity or altered ADCC or CDC activity.

### Conservative amino acid variants

Polypeptide variants may be made that conserve the overall molecular structure of an antibody peptide sequence described herein. Given the properties of the individual amino acids, some rational substitutions will be recognized by the skilled worker. Amino acid substitutions, i.e., "conservative substitutions," may be made, for instance, on the basis of similarity in polarity, charge, solubility, hydrophobicity, hydrophilicity, and/or the amphipathic nature of the residues involved.

For example, (a) nonpolar (hydrophobic) amino acids include alanine, leucine, isoleucine, valine, proline, phenylalanine, tryptophane, and methionine; (b) polar neutral amino acids include glycine, serine, threonine, cysteine, tyrosine, asparagine, and glutamine; (c) positively charged (basic) amino acids include arginine, lysine, and histidine; and (d) negatively charged (acidic) amino acids include aspartic acid and glutamic acid. Substitutions typically may be made within groups (a)-(d). In addition, glycine and proline may be substituted for one another based on their ability to disrupt α-helices. Similarly, certain amino acids, such as alanine, cysteine, leucine, methionine, glutamic acid, glutamine, histidine and lysine are more commonly found in α-helices, while valine, isoleucine, phenylalanine, tyrosine, tryptophan and threonine are more commonly found in β-pleated sheets. Glycine, serine, aspartic acid, asparagine, and proline are commonly found in turns. Some preferred substitutions may be made among the following groups: (i) S and T; (ii) P and G; and (iii) A, V, L and I. Given the known genetic code, and recombinant and synthetic DNA techniques, the skilled scientist readily can construct DNAs encoding the conservative amino acid variants.

### Glycosylation variants

Where the antibody comprises an Fc region, the carbohydrate attached thereto may be altered. Native antibodies produced by mammalian cells typically comprise a branched, biantennary oligosaccharide that is generally attached by an N-linkage to Asn297 using Kabat EU numbering of the CH2 domain of the Fc region; see, e.g., Wright et al. Trends Biotechnol. 15: 26-32 (1997).

In certain embodiments, an antibody provided herein is altered to increase or decrease the extent to which the antibody is glycosylated. Addition or deletion of glycosylation sites to an antibody may be conveniently accomplished by altering the expression system (e.g. host cell) and / or by altering the amino acid sequence such that one or more glycosylation sites is created or removed.

In one embodiment of this disclosure, aglycosyl antibodies having decreased effector function or antibody derivatives are prepared by expression in a prokaryotic host. Suitable prokaryotic hosts for include but are not limited to E. coli, Bacillus subtilis, Salmonella typhimurium and various species within the genera Pseudomonas, Streptomyces, and Staphylococcus.

In one embodiment, antibody variants are provided having decreased effector function, which are characterized by a modification at the conserved N-linked site in the CH2 domains of the Fc portion of said antibody. In one embodiment of present disclosure, the modification comprises a mutation at the heavy chain glycosylation site to prevent glycosylation at the site. Thus, in one preferred embodiment of this disclosure, the aglycosyl antibodies or antibody derivatives are prepared by mutation of the heavy chain glycosylation site, - i.e., mutation of N297 using Kabat EU numbering and expressed in an appropriate host cell.

In another embodiment of the present disclosure, aglycosyl antibodies or antibody derivatives have decreased effector function, wherein the modification at the conserved N-linked site in the CH2 domains of the Fc portion of said antibody or antibody derivative comprises the removal of the CH2 domain glycans, - i.e., deglycosylation. These aglycosyl antibodies may be generated by conventional methods and then deglycosylated enzymatically. Methods for enzymatic deglycosylation of antibodies are well known in the art (e.g. Winkelhake & Nicolson (1976), J Biol Chem. 251(4):1074-80).

In another embodiment of this disclosure, deglycosylation may be achieved using the glycosylation inhibitor tunicamycin (Nose & Wigzell (1983), Proc Natl Acad Sci USA, 80(21):6632-6). That is, the modification is the prevention of glycosylation at the conserved N-linked site in the CH2 domains of the Fc portion of said antibody.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%.

The amount of fucose is determined by calculating the average amount of fucose within the sugar chain at Asn297, relative to the sum of all glycostructures attached to Asn 297 (e.g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function.

Examples of publications related to "defucosylated" or "fucose-deficient" antibody variants include Okazaki et al. J Mol. Biol. 336: 1239-1249 (2004); Yamane-Ohnuki et al. Biotech. Bioeng. 87: 614 (2004).

Examples of cell lines capable of producing defucosylated antibodies include Lec13 CHO cells deficient in protein fucosylation (Ripka et al. Arch. Biochem. Biophys. 249:533-545 (1986); and WO 2004/056312), and knockout cell lines, such as alpha-1,6-fucosyltransferase gene, FUT8, knockout CHO cells (see, e.g., Yamane-Ohnuki et al. Biotech. Bioeng. 87: 614 (2004); Kanda, Y. et al., Biotechnol. Bioeng., 94(4):680-688 (2006)).

Antibody variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878; US Patent No. 6,602,684; and US 2005/0123546.

Antibody variants with at least one galactose residue in the oligosaccharide attached to the Fc region are also provided. Such antibody variants may have improved CDC function. Such antibody variants are described, e.g., in WO1997/30087; WO1998/58964; and WO1999/22764.

### Fc region variants

In certain embodiments, one or more amino acid modifications (e.g. a substitution) may be introduced into the Fc region of an antibody (e.g., a human IgG1, IgG2, IgG3 or IgG4 Fc region) provided herein, thereby generating an Fc region variant.

In certain embodiments, the disclosure contemplates an antibody variant that possesses some but not all effector functions, which make it a desirable candidate for applications in which the half-life of the antibody in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity) but retains FcRn binding ability. In some embodiments, alterations are made in the Fc region that result in altered (i.e., either improved or diminished) C1q binding and/or Complement Dependent Cytotoxicity (CDC).

In certain embodiments, the disclosure contemplates an antibody variant that possesses an increased or decreased half-live. Antibodies with increased half-lives and improved binding to the neonatal Fc receptor (FcRn), which is responsible for the transfer of maternal IgGs to the fetus (Guyer et al., J Immunol. 117:587 (1976) and Kim et al., J Immunol. 24:249 (1994)), are described in US2005/0014934 (Hinton et al.). Those antibodies comprise an Fc region with one or more substitutions therein which improve binding of the Fc region to FcRn.

### DNA molecules according to the present disclosure

The present disclosure also relates to an isolated nucleic acid sequence that encodes the antibody or antigen-binding fragment according to the present disclosure. The isolated nucleic acid sequence encoding the antibody or antigen-binding fragment according to the present disclosure can for instance be produced by techniques described in Sambrook et al., 1989, and Ausubel et al., 1989, or alternatively, by chemically synthesis. (e.g. techniques described in Oligonucleotide Synthesis (1984, Gait, ed., IRL Press, Oxford)). DNA molecules of the disclosure are not limited to the sequences disclosed herein, but also include variants thereof. DNA variants within the disclosure may be described by reference to their physical properties in hybridization. The skilled worker will recognize that DNA can be used to identify its complement and, since DNA is double stranded, its equivalent or homolog, using nucleic acid hybridization techniques. It also will be recognized that hybridization can occur with less than 100% complementarity. However, given appropriate choice of conditions, hybridization techniques can be used to differentiate among DNA sequences based on their structural relatedness to a particular probe. For guidance regarding such conditions see, Sambrook et al., 1989 supra and Ausubel et al., 1995 (Ausubel, F. M., Brent, R., Kingston, R. E., Moore, D. D., Sedman, J. G., Smith, J. A., & Struhl, K. eds. (1995). Current Protocols in Molecular Biology. New York: John Wiley and Sons).

Structural similarity between two polynucleotide sequences can be expressed as a function of "stringency" of the conditions under which the two sequences will hybridize with one another. As used herein, the term "stringency" refers to the extent that the conditions disfavor hybridization. Stringent conditions strongly disfavor hybridization, and only the most structurally related molecules will hybridize to one another under such conditions. Conversely, non-stringent conditions favor hybridization of molecules displaying a lesser degree of structural relatedness. Hybridization stringency, therefore, directly correlates with the structural relationships of two nucleic acid sequences.

Hybridization stringency is a function of many factors, including overall DNA concentration, ionic strength, temperature, probe size and the presence of agents which disrupt hydrogen bonding. Factors promoting hybridization include high DNA concentrations, high ionic strengths, low temperatures, longer probe size and the absence of agents that disrupt hydrogen bonding. Hybridization typically is performed in two phases: the "binding" phase and the "washing" phase.

### Functionally equivalent DNA variants

Yet another class of DNA variants within the scope of the disclosure may be described with reference to the product they encode. These functionally equivalent polynucleotides are characterized by the fact that they encode the same peptide sequences due to the degeneracy of the genetic code.

It is recognized that variants of DNA molecules provided herein can be constructed in several different ways. For example, they may be constructed as completely synthetic DNAs. Methods of efficiently synthesizing oligonucleotides are widely available. See Ausubel et al., section 2.11, Supplement 21 (1993). Overlapping oligonucleotides may be synthesized and assembled in a fashion first reported by Khorana et al., J. Mol. Biol. 72:209-217 (1971); see also Ausubel et al., supra, Section 8.2. Synthetic DNAs preferably are designed with convenient restriction sites engineered at the 5' and 3' ends of the gene to facilitate cloning into an appropriate vector.

As indicated, a method of generating variants is to start with one of the DNAs disclosed herein and then to conduct site-directed mutagenesis. See Ausubel et al., supra, chapter 8, Supplement 37 (1997). In a typical method, a target DNA is cloned into a single-stranded DNA bacteriophage vehicle. Single-stranded DNA is isolated and hybridized with an oligonucleotide containing the desired nucleotide alteration(s). The complementary strand is synthesized and the double stranded phage is introduced into a host. Some of the resulting progeny will contain the desired mutant, which can be confirmed using DNA sequencing. In addition, various methods are available that increase the probability that the progeny phage will be the desired mutant. These methods are well known to those in the field and kits are commercially available for generating such mutants.

### Recombinant DNA constructs and expression

The present disclosure further provides recombinant DNA constructs comprising one or more of the nucleotide sequences according to the present disclosure. The recombinant constructs of the present disclosure can be used in connection with a vector, such as a plasmid, phagemid, phage or viral vector, into which a DNA molecule encoding an antibody of the disclosure or antigen-binding fragment thereof or variant thereof is inserted.

Thus, in one aspect, the present disclosure relates to a vector comprising a nucleic acid sequence according to the present disclosure.

An antibody, antigen binding portion, or variant thereof provided herein can be prepared by recombinant expression of nucleic acid sequences encoding light and heavy chains or portions thereof in a host cell. To express an antibody, antigen binding portion, or variant thereof recombinantly a host cell can be transfected with one or more recombinant expression vectors carrying DNA fragments encoding the light and/or heavy chains or portions thereof such that the light and heavy chains are expressed in the host cell. Standard recombinant DNA methodologies are used to prepare and/or obtain nucleic acids encoding the heavy and light chains, incorporate these nucleic acids into recombinant expression vectors and introduce the vectors into host cells, such as those described in Sambrook, Fritsch and Maniatis (eds.), Molecular Cloning; A Laboratory Manual, Second Edition, Cold Spring Harbor, N.Y., (1989), Ausubel, F. M. et al. (eds.) Current Protocols in Molecular Biology, Greene Publishing Associates, (1989) and in U.S. Pat. No. 4,816,397 by Boss et al..

In addition, the nucleic acid sequences encoding variable regions of the heavy and/or light chains can be converted, for example, to nucleic acid sequences encoding full-length antibody chains, Fab fragments, or to scFv. The VL- or VH-encoding DNA fragment can be operatively linked, (such that the amino acid sequences encoded by the two DNA fragments are in-frame) to another DNA fragment encoding, for example, an antibody constant region or a flexible linker. The sequences of human heavy chain and light chain constant regions are known in the art (see e.g., Kabat, E. A., el al. (1991) Sequences of Proteins of Immunological Interest, Fifth Edition, U.S. Department of Health and Human Services, NIH Publication No. 91-3242) and DNA fragments encompassing these regions can be obtained by standard PCR amplification.

To create a polynucleotide sequence that encodes a scFv, the VH- and VL-encoding nucleic acids can be operatively linked to another fragment encoding a flexible linker such that the VH and VL sequences can be expressed as a contiguous single-chain protein, with the VL and VH regions joined by the flexible linker (see e.g., Bird et al. (1988) Science 242:423-426; Huston et al. (1988) Proc. Natl. Acad. Sci. USA 85:5879-5883; McCafferty et al., Nature (1990) 348:552-554).

To express the antibodies, antigen binding fragments thereof or variants thereof standard recombinant DNA expression methods can be used (see, for example, Goeddel; Gene Expression Technology. Methods in Enzymology 185, Academic Press, San Diego, Calif. (1990)). For example, DNA encoding the desired polypeptide can be inserted into an expression vector which is then transfected into a suitable host cell. Suitable host cells are prokaryotic and eukaryotic cells. Examples for prokaryotic host cells are e.g. bacteria, examples for eukaryotic hosts cells are yeasts, insects and insect cells, plants and plant cells, transgenic animals, or mammalian cells. Introduction of the recombinant construct into the host cell can be carried out using standard techniques such as calcium phosphate transfection, DEAE dextran mediated transfection, electroporation, transduction or phage infection.

In some embodiments, the DNAs encoding the heavy and light chains are inserted into separate vectors. In other embodiments, the DNA encoding the heavy and light chains is inserted into the same vector. It is understood that the design of the expression vector, including the selection of regulatory sequences is affected by factors such as the choice of the host cell, the level of expression of protein desired and whether expression is constitutive or inducible.

Thus, in a further aspect, the present disclosure relates to an isolated cell expressing the antibody or antigen-binding fragment according to the present disclosure and/or comprising the nucleic acid according to the present disclosure or the vector according to the present disclosure.

The isolated cell can be virtually any cell for which expression vectors are available. The isolated cell can for example a higher eukaryotic host cell, such as a mammalian cell, a lower eukaryotic host cell, such as a yeast cell, and may be a prokaryotic cell, such as a bacterial cell.

In a further aspect, the present disclosure relates to a method of producing the isolated antibody or antigen-binding fragment according to the present disclosure comprising culturing of the cell according to the present disclosure. In particular embodiments, the cell according to the present disclosure is cultivated under suitable conditions for antibody expression and the antibody or antigen-binding fragment is recovered. In particular embodiments, the antibody or antigen-binding fragment is purified, particularly to at least 95% homogeneity by weight.

### Bacterial Expression

Useful expression vectors for bacterial use are constructed by inserting a DNA sequence encoding a desired protein together with suitable translation initiation and termination signals in operable reading phase with a functional promoter. The vector will comprise one or more phenotypic selectable markers and an origin of replication to ensure maintenance of the vector and, if desirable, to provide amplification within the host. Suitable prokaryotic hosts for transformation include but are not limited to E. coli, Bacillus subtilis, Salmonella typhimurium and various species within the genera Pseudomonas, Streptomyces, and Staphylococcus.

Bacterial vectors may be, for example, bacteriophage-, plasmid- or phagemid-based. These vectors can contain a selectable marker and a bacterial origin of replication derived from commercially available plasmids typically containing elements of the well-known cloning vector pBR322 (ATCC 37017). Following transformation of a suitable host strain and growth of the host strain to an appropriate cell density, the selected promoter is de-repressed/induced by appropriate means (e.g., temperature shift or chemical induction) and cells are cultured for an additional period. Cells are typically harvested by centrifugation, disrupted by physical or chemical means, and the resulting crude extract retained for further purification.

In bacterial systems, a number of expression vectors may be advantageously selected depending upon the use intended for the protein being expressed. For example, when a large quantity of such a protein is to be produced, for the generation of antibodies or to screen peptide libraries, for example, vectors which direct the expression of high levels of fusion protein products that are readily purified may be desirable.

Therefore, an embodiment of the present disclosure is an expression vector comprising a nucleic acid sequence encoding for the novel antibodies of the present disclosure.

Antibodies of the present disclosure or antigen-binding fragments thereof or variants thereof include naturally purified products, products of chemical synthetic procedures, and products produced by recombinant techniques from a prokaryotic host, including, for example, E. coli, Bacillus subtilis, Salmonella typhimurium and various species within the genera Pseudomonas, Streptomyces, and Staphylococcus, preferably, from E. coli cells.

### Mammalian Expression

Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. Expression of the antibodies may be constitutive or regulated (e.g. inducible by addition or removal of small molecule inductors such as Tetracyclin in conjunction with Tet system). For further description of viral regulatory elements, and sequences thereof, see e.g., U.S. 5,168,062 by Stinski, U.S. 4,510,245 by Bell et al. and U.S. 4,968,615 by Schaffner et al.. The recombinant expression vectors can also include origins of replication and selectable markers (see e.g., U.S. 4,399,216, 4,634,665 and U.S. 5,179,017). Suitable selectable markers include genes that confer resistance to drugs such as G418, puromycin, hygromycin, blasticidin, zeocin/bleomycin or methotrexate or selectable marker that exploit auxotrophies such as Glutamine Synthetase (Bebbington et al., Biotechnology (N Y). 1992 Feb;10(2):169-75), on a host cell into which the vector has been introduced. For example, the dihydrofolate reductase (DHFR) gene confers resistance to methotrexate, neo gene confers resistance to G418, the bsd gene from Aspergillus terreus confers resistance to blasticidin, puromycin N-acetyl-transferase confers resistance to puromycin, the Sh ble gene product confers resitance to zeocin, and resistance to hygromycin is conferred by the E. coli hygromycin resistance gene (hyg or hph). Selectable markers like DHFR or Glutamine Synthetase are also useful for amplification techniques in conjunction with MTX and MSX.

Transfection of the expression vector into a host cell can be carried out using standard techniques such as electroporation, nucleofection, calcium-phosphate precipitation, lipofection, polycation-based transfection such as polyethlylenimine (PEI)-based transfection and DEAE-dextran transfection.

Suitable mammalian host cells for expressing the antibodies, antigen binding fragments thereof or variants thereof provided herein include Chinese Hamster Ovary (CHO cells) such as CHO-K1, CHO-S, CHO-K1SV [including dhfr- CHO cells, described in Urlaub and Chasin, (1980) Proc. Natl. Acad. Sci. USA 77:4216-4220 and Urlaub et al., Cell. 1983 Jun;33(2):405-12, used with a DHFR selectable marker, e.g., as described in R. J. Kaufman and P. A. Sharp (1982) Mol. Biol. 159:601-621; and other knockout cells exemplified in Fan et al., Biotechnol Bioeng. 2012 Apr;109(4):1007-15], NSO myeloma cells, COS cells, HEK293 cells, HKB11 cells, BHK21 cells, CAP cells, EB66 cells, and SP2 cells.

Expression might also be transient or semi-stable in expression systems such as HEK293, HEK293T, HEK293-EBNA, HEK293E, HEK293-6E, HEK293-Freestyle, HKB11, Expi293F, 293EBNALT75, CHO Freestyle, CHO-S, CHO-K1, CHO-K1SV, CHOEBNALT85, CHOS-XE, CHO-3E7 or CAP-T cells (for instance Durocher et al., Nucleic Acids Res. 2002 Jan 15;30(2): E9).

In some embodiments, the expression vector is designed such that the expressed protein is secreted into the culture medium in which the host cells are grown. The antibodies, antigen binding fragments thereof or variants thereof can be recovered from the culture medium using standard protein purification methods.

### Purification

Antibodies of the disclosure or antigen-binding fragments thereof or variants thereof can be recovered and purified from recombinant cell cultures by well-known methods including, but not limited to ammonium sulfate or ethanol precipitation, acid extraction, Protein A chromatography, Protein G chromatography, anion or cation exchange chromatography, phospho-cellulose chromatography, hydrophobic interaction chromatography, affinity chromatography, hydroxylapatite chromatography and lectin chromatography. High performance liquid chromatography ("HPLC") can also be employed for purification. See, e.g., Colligan, Current Protocols in Immunology, or Current Protocols in Protein Science, John Wiley & Sons, NY, N.Y., (1997-2001), e.g., Chapters 1, 4, 6, 8, 9, 10, each entirely incorporated herein by reference.

Antibodies of the present disclosure or antigen-binding fragments thereof or variants thereof include naturally purified products, products of chemical synthetic procedures, and products produced by recombinant techniques from a eukaryotic host, including, for example, yeast, higher plant, insect and mammalian cells. Depending upon the host employed in a recombinant production procedure, the antibody of the present disclosure can be glycosylated or can be non- glycosylated. Such methods are described in many standard laboratory manuals, such as Sambrook, supra, Sections 17.37-17.42; Ausubel, supra, Chapters 10, 12, 13, 16, 18 and 20.

In preferred embodiments, the antibody is purified (1) to greater than 95% by weight of antibody as determined e.g. by the Lowry method, UV-Vis spectroscopy or by by SDS-Capillary Gel electrophoresis (for example on a Caliper LabChip GXII, GX 90 or Biorad Bioanalyzer device), and in further preferred embodiments more than 99% by weight, (2) to a degree sufficient to obtain at least 15 residues of N-terminal or internal amino acid sequence, or (3) to homogeneity by SDS-PAGE under reducing or non-reducing conditions using Coomassie blue or, preferably, silver stain. Isolated naturally occurring antibody includes the antibody in situ within recombinant cells since at least one component of the antibody's natural environment will not be present. Ordinarily, however, isolated antibody will be prepared by at least one purification step.

### Antibody conjugates

In addition to naked antibodies or antigen-binding fragment thereof, various antibody- or antibody fragment-based conjugates can be designed using the antibodies or antigen binding fragments disclosed herein.

In a second aspect, the present disclosure relates to antibody conjugates targeting CEACAM5, comprising the isolated antibodies or antigen binding fragments according to the present disclosure. These conjugates may be conjugates for diagnosis, therapy, research applications, and various other purposes. The antibodies or antigen binding fragments thereof may for example be conjugated to radionuclides, cytotoxic agents, organic compounds, protein toxins, immunomodulators such as cytokines, fluorescent moieties, cells, further antibodies or antigen binding fragments thereof.

In some other embodiments, the conjugate comprises a cytotoxic agent, e.g. to form an antibody drug conjugate (ADC). For example, the cytotoxic agent may be selected from an auristatin, a maytansinoid, a kinesin-spindle protein (KSP) inhibitor, a nicotinamide phosphoribosyltransferase (NAMPT) inhibitor or a pyrrolobenzodiazepine derivative.

The cytotoxic and/or cytostatic agent of the ADC may be any agent known to inhibit the growth and/or replication of, and/or kill cells. Numerous agents having cytotoxic and/or cytostatic properties are known in the literature. Non-limiting examples of classes of cytotoxic and/or cytostatic agents include, by way of example and not limitation, cell cycle modulators, apoptosis regulators, kinase inhibitors, protein synthesis inhibitors, alkylating agents, DNA cross-linking agents, intercalating agents, mitochondria inhibitors, nuclear export inhibitors, topoisomerase I inhibitors, topoisomerase II inhibitors, RNA/DNA antimetabolites and antimitotic agents.

Linkers linking the cytotoxic and/or cytostatic agent(s) to the targeting moiety of an ADC may be long, short, flexible, rigid, hydrophilic or hydrophobic in nature, or may comprise segments that have different characteristics, such as segments of flexibility, segments of rigidity, etc. The linker may be chemically stable to extracellular environments, for example, chemically stable in the blood stream, or may include linkages that are not stable and release the cytotoxic and/or cytostatic agents in the extracellular milieu. A wide variety of linkers useful for linking drugs to antigen binding moieties such as antibodies in the context of ADCs are known in the art.

The number of cytotoxic and/or cytostatic agents linked to the targeting moiety of an anti-CEACAM5 ADC (drug-to-antibody ratio: DAR) can vary and will be limited only by the number of available attachments sites on the targeting moiety and the number of agents linked to a single linker. Typically, a linker will link a single cytotoxic and/or cytostatic agent to the targeting moiety of the ADC. In embodiments of ADCs which include more than a single cytotoxic and/or cytostatic agent, each agent may be the same or different.

In some other embodiments, the conjugate is a chimeric antigen receptor conjugate engineered for CEACAM5 targeting. Recently, CAR T cells have gained attention from their clinical successes and expedited FDA approvals, cf. WO2020/102240, incorporated herein in its entirety. In the CAR T cell approach, T cells are engineered to express CARs that are specific for an antigen present on tumor cells. These engineered T cells are then re-administered to the same patient. Upon injection, CAR T cells recognize the targeted antigen on target cells to induce target cell death. T cells expressing chimeric antigen receptors (CAR T cells) thus constitute a novel modality for medical uses such as tumor treatment. The chimeric antigen receptor (CAR) is a genetically engineered receptor that is designed to target a specific antigen, for example, a tumor antigen. This targeting can result in cytotoxicity against the tumor, for example, such that CAR T cells expressing CARs can target and kill tumors via the specific tumor antigens.

According to the present disclosure, the antibodies or antigen binding fragments provided for CEACAM5 can be used to engineer CAR T cells for specific recognition of CEACAM5 expressing cells.

Preferably, the CARs according to the current disclosure comprise a co-stimulation domain (e.g., CD137, CD28 or CD134) to achieve prolonged activation of T cells in vivo. Addition of a co-stimulation domain enhances the in vivo proliferation and survival of T cells containing CARs, and initial clinical data have shown that such constructs are promising therapeutic agents in the treatment of diseases, such as cancer. According to the current disclosure, the CAR T cells can be used to treat any disease with local or systemic aberrant presence of cells expressing CEACAM5.

In some other embodiments, the conjugate is or comprises a bispecific antibody or a multispecific antibody. In some preferred embodiments the bispecific antibody comprises at least one Fc domain.

In some preferred embodiments, the first binding moiety of the bispecific antibody is an antibody or antigen binding fragment according to the present disclosure and the second binding moiety of the bispecific antibody is the same or a different antibody or antigen binding fragment according to the present disclosure.

In some further embodiments, the first binding moiety of the bispecific antibody is an antibody or antigen binding fragment according to the present disclosure and the second binding moiety of the bispecific antibody is an antibody or antigen binding fragment binding to a cell-surface protein such as a cell type-specific antigen. In some of these embodiments, the second binding moiety of the bispecific antibody is an antibody or antigen binding fragment targeting a checkpoint protein, such as an anti PD1 antibody, or an anti PD-L1 antibody. Suitable checkpoint protein targeting antibodies include Nivolumab, Pembrolizumab, Atezolizumab, Avelumab, Durvalumab, Cemiplimab, Dostarlimab, or Ipilimumab. In some other of these embodiments, the second binding moiety of the bispecific antibody is HER2 targeting antibody, such as Trastuzumab, Pertuzumab and/or Margetuximab.

Techniques for making bi- or multispecific antibodies include, but are not limited to, recombinant co-expression of two immunoglobulin heavy chain-light chain pairs having different specificities (see Milstein and Cuello, Nature 305: 537 (1983), WO 93/08829, and Traunecker et al., EMBO J. 10: 3655 (1991)), and chemical conjugation of two different monoclonal antibodies (see Staerz et al. (1985) Nature 314(6012): 628-31). Multispecific antibodies may also be made by cross-linking two or more antibodies or fragments (see, e.g., US Patent No. 4,676,980, and Brennan et al., Science, 229: 81 (1985)), using leucine zippers to produce bi-specific antibodies (see, e.g., Kostelny et al., J. Immunol, 148(5): 1547- 1553 (1992)), using diabody technology for making bispecific antibody fragments (see, e.g., Hollinger et al., Proc. Natl. Acad. Sci. USA, 90:6444-6448 (1993)), using single- chain Fv (sFv) dimers (see,e.g. Gruber et al., J. Immunol, 152:5368 (1994)), by preparing trispecific antibodies as described, e.g., in Tutt et al. J. Immunol. 147: 60 (1991) and by controlled Fab arm exchange (cFAE) according to Labrijn AF et al. Proc Natl Acad Sci USA 2013; 110:5145-50.

In some other embodiments, the conjugate comprises a detectable moiety. Examples of detectable moieties include various enzymes, prosthetic groups, fluorescent materials, luminescent materials, bioluminescent materials, radioactive materials, positron emitting metals, nonradioactive paramagnetic metal ions and reactive moieties.

The detectable substance can be coupled or conjugated either directly to the antibody or fragment thereof or indirectly, e.g. through a linker known in the art or another moiety, using techniques known in the art. Examples of enzymatic labels include luciferases (e.g., firefly luciferase and bacterial luciferase; US4,737,456), luciferin, 2,3-dihydrophthalazinediones, malate dehydrogenase, urease, peroxidase such as horseradish peroxidase (HRPO), alkaline phosphatase, β-galactosidase, acetylcholinesterase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like.

Examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include 125I, 131I, 111In or 99mTc.

In some embodiments the conjugate may comprise a radionuclide, such as a beta particle, an alpha particle, a gamma particle or an Auger electron emitter. For example, the radionuclide may be selected from the group consisting of 43Sc, 44Sc, 47Sc, 89Zr, 90Y, 111In, 149Tb,152Tb, 155Tb, 161Tb, 166Ho, 177Lu, 186Re, 188Re, 212Bi, 213Bi, 225Ac, 227Th, and 232Th. Suitable beta emitters according to the current disclosure are for example 67Cu, 89Sr, 89Zr, 90Y, 105Rh, 1311, 149Pm, 166Ho, 177Lu, 186Re, 188Re, 198Au. In some preferred embodiments of the current disclosure, the radionuclide is 89Zr. Suitable Auger electron emitting radionuclides are for example 67Ga, 71Ge, 77Br, 99mTc, 103Pd, 111In, 123I, 125I, 140Nd, 178Ta, 193Pt, 195mPt, 197Hg. Suitable alpha emitters, according to the current disclosure are for example 211At, 212Pb, 213Bi, 223Ra, 224Ra, 225Ac, or 227Th.

In some preferred embodiments, the radionuclide is an α-particle-emitting radionuclide such as 225Ac. In some particularly preferred embodiments, the radionuclide is 225Ac.

In some preferred embodiments, the conjugate furthermore comprises a chelator for immobilization of the radionuclide, e.g. as described elsewhere herein.

### Targeted radionuclide conjugates

According to a third aspect, the present disclosure relates to targeted radionuclide conjugates comprising the anti-CEACAM5 antibodies or antigen-binding fragments thereof according to the present disclosure and a chelating moiety for chelating a radionuclide.

In some embodiments of this aspect of the present disclosure the chelating moiety is capable of complexing an alpha-emitting radionuclide, a beta-emitting radionuclide, a gamma-emitting radionuclide or an Auger electron emitter.

In some preferred embodiments of this aspect of the present disclosure the chelating moiety is capable of complexing an alpha-emitting radionuclide. I some embodiments the alpha-emitting radionuclide may be for example, but is not limited to, actinium (225Ac3+), radium (233Ra2+), bismuth (213Bi3+, 212Bi3+, or 211Bi3+), lead (212Pb2+ or 212Pb4+), thorium (227Th4+ or 226Th4+), dysprosium (152Dy3+), or astatine (211At+ or 217At+), In some highly preferred embodiments of present aspect of the disclosure the alpha-emitting radionuclide complexed to a chelating moiety is actinium (225Ac), preferably 225Ac3+.

In some preferred embodiments of this aspect of the present disclosure the chelating moiety capable of complexing actinium comprises an 18-membered chelating moiety. In some further preferred embodiments of the present disclosure, the chelating moiety comprises the macrocyclic macropa chelator N,N'-bis[(6-carboxy-2-pyridil)methyl]-4,13-diaza-18-crown-6 . In some highly preferred embodiments of the present disclosure, the chelating moiety comprises the chelator Macropa-NCS (6-[[16-[(6-carboxy-2-pyridyl)methyl]-1,4,10,13-tetraoxa-7,16-diazacyclooctadec-7-yl]methyl]-4-[2-(4-isothiocyanatophenyl)ethoxy]pyridine-2-carboxylic acid) (in the Examples abbreviated as "Macropa") as for example described in WO2020/106886.

In some preferred embodiments of the present disclosure the chelating moiety capable of complexing actinium terminates in an isothiocyanate or carboxylic acid moiety in order to facilitate its coupling with an antibody or an antigen-binding fragment thereof according to the present disclosure. When the macropa chelator is used, one of the pyridine rings of the macropa chelator is typically substituted so that it can be coupled to the anti-CEACAM5 antibody or an antigen-binding fragment thereof according to the disclosure. This can be a simple functional group (e.g., a carboxylic acid -COOH) or a more complex chemical structure, as long as it is capable of being coupled to an anti-CEACAM5 antibody or an antigen-binding fragment thereof.

In particular embodiments of the present disclosure, the substituent on the pyridine ring of the macropa chelator preferably terminates in an isothiocyanate or carboxylic acid moiety.

In one embodiment, the present disclosure relates to a tissue-targeting compound of formula (I) 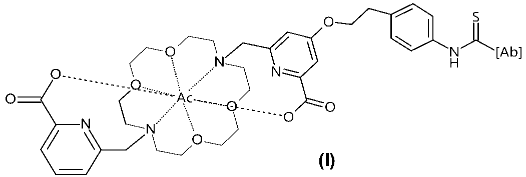 wherein [Ab] is a monoclonal antibody or an antigen-binding fragment thereof capable of binding to CEACAM5 selected from an isolated antibody or antigen-binding fragment according to the first aspect of the present disclosure.

In a further embodiment, the present disclosure relates to a tissue-targeting compound of formula (I) 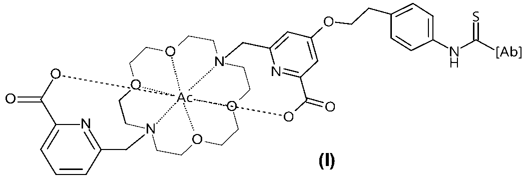 wherein [Ab] is a monoclonal antibody or an antigen-binding fragment thereof capable of binding to CEACAM5 selected from an isolated antibody or antigen-binding fragment according to the first aspect of the present disclosure, wherein [Ab] is bound to the to the rest of the molecule via a terminal amino group of a lysine residue of the monoclonal antibody or antigen-binding fragment thereof.

In a further embodiment, the present disclosure relates to a tissue-targeting compound of formula (I) 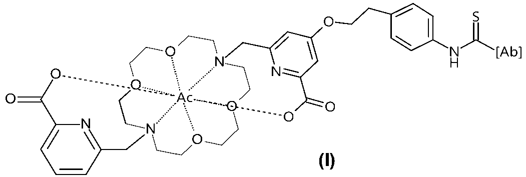 wherein [Ab] is a monoclonal antibody or an antigen-binding fragment thereof capable of binding to CEACAM5, comprising:
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291), or
x) a heavy chain antigen-binding region that comprises an H-CDR1 comprising SEQ ID NO: 91, an H-CDR2 comprising SEQ ID NO: 92, and an H-CDR3 comprising SEQ ID NO: 93 and a light chain antigen-binding region that comprises an L-CDR1 comprising SEQ ID NO: 96, an L-CDR2 comprising SEQ ID NO: 97, and an L-CDR3 comprising SEQ ID NO:98 (DPP-64106), or
xi) a heavy chain antigen-binding region that comprises an H-CDR1 comprising SEQ ID NO: 100, an H-CDR2 comprising SEQ ID NO: 101, and an H-CDR3 comprising SEQ ID NO: 102 and a light chain antigen-binding region that comprises an L-CDR1 comprising SEQ ID NO: 105, an L-CDR2 comprising SEQ ID NO: 106, and an L-CDR3 comprising SEQ ID NO:107 (DPP-64107), or
xii) a heavy chain antigen-binding region that comprises an H-CDR1 comprising SEQ ID NO: 109, an H-CDR2 comprising SEQ ID NO: 110 and an H-CDR3 comprising SEQ ID NO: 111 and a light chain antigen-binding region that comprises an L-CDR1 comprising SEQ ID NO: 114, an L-CDR2 comprising SEQ ID NO: 115, and an L-CDR3 comprising SEQ ID NO:116 (DPP-64204), or
xiii) a heavy chain antigen-binding region that comprises an H-CDR1 comprising SEQ ID NO: 118, an H-CDR2 comprising SEQ ID NO: 119, and an H-CDR3 comprising SEQ ID NO: 120 and a light chain antigen-binding region that comprises an L-CDR1 comprising SEQ ID NO: 123, an L-CDR2 comprising SEQ ID NO: 124, and an L-CDR3 comprising SEQ ID NO:125 (DPP-64209), or
xiv) a heavy chain antigen-binding region that comprises an H-CDR1 comprising SEQ ID NO: 127, an H-CDR2 comprising SEQ ID NO: 128, and an H-CDR3 comprising SEQ ID NO: 129 and a light chain antigen-binding region that comprises an L-CDR1 comprising SEQ ID NO: 132, an L-CDR2 comprising SEQ ID NO: 133, and an L-CDR3 comprising SEQ ID NO:134 (DPP-64227), or
xv) a heavy chain antigen-binding region that comprises an H-CDR1 comprising SEQ ID NO: 136, an H-CDR2 comprising SEQ ID NO: 137, and an H-CDR3 comprising SEQ ID NO: 138 and a light chain antigen-binding region that comprises an L-CDR1 comprising SEQ ID NO: 141, an L-CDR2 comprising SEQ ID NO: 142, and an L-CDR3 comprising SEQ ID NO:143 (DPP-64262).

In a further embodiment, the present disclosure relates to a tissue-targeting compound of formula (I) 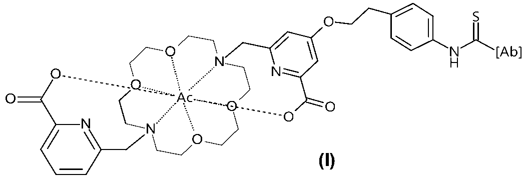 wherein [Ab] is a monoclonal antibody or an antigen-binding fragment thereof capable of binding to CEACAM5, comprising:
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291), or
x) a heavy chain antigen-binding region that comprises an H-CDR1 comprising SEQ ID NO: 91, an H-CDR2 comprising SEQ ID NO: 92, and an H-CDR3 comprising SEQ ID NO: 93 and a light chain antigen-binding region that comprises an L-CDR1 comprising SEQ ID NO: 96, an L-CDR2 comprising SEQ ID NO: 97, and an L-CDR3 comprising SEQ ID NO:98 (DPP-64106), or
xi) a heavy chain antigen-binding region that comprises an H-CDR1 comprising SEQ ID NO: 100, an H-CDR2 comprising SEQ ID NO: 101, and an H-CDR3 comprising SEQ ID NO: 102 and a light chain antigen-binding region that comprises an L-CDR1 comprising SEQ ID NO: 105, an L-CDR2 comprising SEQ ID NO: 106, and an L-CDR3 comprising SEQ ID NO:107 (DPP-64107), or
xii) a heavy chain antigen-binding region that comprises an H-CDR1 comprising SEQ ID NO: 109, an H-CDR2 comprising SEQ ID NO: 110 and an H-CDR3 comprising SEQ ID NO: 111 and a light chain antigen-binding region that comprises an L-CDR1 comprising SEQ ID NO: 114, an L-CDR2 comprising SEQ ID NO: 115, and an L-CDR3 comprising SEQ ID NO:116 (DPP-64204), or
xiii) a heavy chain antigen-binding region that comprises an H-CDR1 comprising SEQ ID NO: 118, an H-CDR2 comprising SEQ ID NO: 119, and an H-CDR3 comprising SEQ ID NO: 120 and a light chain antigen-binding region that comprises an L-CDR1 comprising SEQ ID NO: 123, an L-CDR2 comprising SEQ ID NO: 124, and an L-CDR3 comprising SEQ ID NO:125 (DPP-64209), or
xiv) a heavy chain antigen-binding region that comprises an H-CDR1 comprising SEQ ID NO: 127, an H-CDR2 comprising SEQ ID NO: 128, and an H-CDR3 comprising SEQ ID NO: 129 and a light chain antigen-binding region that comprises an L-CDR1 comprising SEQ ID NO: 132, an L-CDR2 comprising SEQ ID NO: 133, and an L-CDR3 comprising SEQ ID NO:134 (DPP-64227), or
xv) a heavy chain antigen-binding region that comprises an H-CDR1 comprising SEQ ID NO: 136, an H-CDR2 comprising SEQ ID NO: 137, and an H-CDR3 comprising SEQ ID NO: 138 and a light chain antigen-binding region that comprises an L-CDR1 comprising SEQ ID NO: 141, an L-CDR2 comprising SEQ ID NO: 142, and an L-CDR3 comprising SEQ ID NO:143 (DPP-64262),
wherein [Ab] is bound to the to the rest of the molecule via a terminal amino group of a lysine residue of the monoclonal antibody or antigen-binding fragment thereof.

In a further embodiment, the present disclosure relates to a tissue-targeting compound of formula (I) 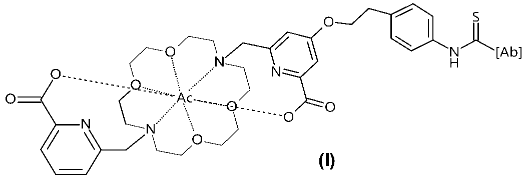 wherein [Ab] is a monoclonal antibody or an antigen-binding fragment thereof capable of binding to CEACAM5, comprising:
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291).

In a further embodiment, the present disclosure relates to a tissue-targeting compound of formula (I) 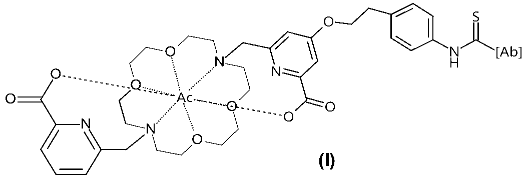 wherein [Ab] is a monoclonal antibody or an antigen-binding fragment thereof capable of binding to CEACAM5, comprising:
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291)
wherein [Ab] is bound to the to the rest of the molecule via a terminal amino group of a lysine residue of the monoclonal antibody or antigen-binding fragment thereof.

In a further embodiment, the present disclosure relates to a tissue-targeting compound of formula (I) 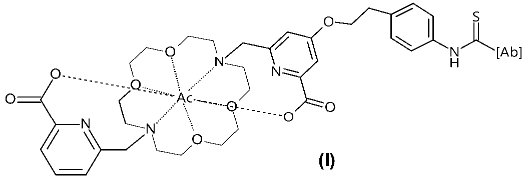 wherein [Ab] is a monoclonal antibody or an antigen-binding fragment thereof capable of binding to CEACAM5 comprising
i) a variable heavy chain comprising SEQ ID NO: 4 and/or a variable light chain domain comprising SEQ ID NO: 9; or
ii) a variable heavy chain domain comprising SEQ ID NO: 14 and/or a variable light chain domain comprising SEQ ID NO: 19; or
iii) a variable heavy chain domain comprising SEQ ID NO: 24 and/or a variable light chain domain comprising SEQ ID NO: 29; or
iv) a variable heavy chain domain comprising SEQ ID NO: 34 and/or a variable light chain domain comprising SEQ ID NO: 39; or
v) a variable heavy chain domain comprising SEQ ID NO: 44 and/or a variable light chain domain comprising SEQ ID NO: 49; or
vi) a variable heavy chain domain comprising SEQ ID NO: 54 and/or a variable light chain domain comprising SEQ ID NO: 59; or
vii) a variable heavy chain domain comprising SEQ ID NO: 64 and/or a variable light chain domain comprising SEQ ID NO: 69; or
viii) a variable heavy chain domain comprising SEQ ID NO: 74 and/or a variable light chain domain comprising SEQ ID NO: 79, or
ix) a variable heavy chain domain comprising SEQ ID NO: 84 and/or a variable light chain domain comprising SEQ ID NO: 89; or
x) a variable heavy chain domain comprising SEQ ID NO: 94 and/or a variable light chain domain comprising SEQ ID NO: 99; or
xi) a variable heavy chain domain comprising SEQ ID NO: 103 and/or a variable light chain domain comprising SEQ ID NO: 108; or
xii) a variable heavy chain domain comprising SEQ ID NO: 112 and/or a variable light chain domain comprising SEQ ID NO: 117; or
xiii) a variable heavy chain domain comprising SEQ ID NO: 121 and/or a variable light chain domain comprising SEQ ID NO: 126; or
xiv) a variable heavy chain domain comprising SEQ ID NO: 130 and/or a variable light chain domain comprising SEQ ID NO: 135; or
xv) a variable heavy chain domain comprising SEQ ID NO: 139 and/or a variable light chain domain comprising SEQ ID NO: 144.

In a further embodiment, the present disclosure relates to a tissue-targeting compound of formula (I) 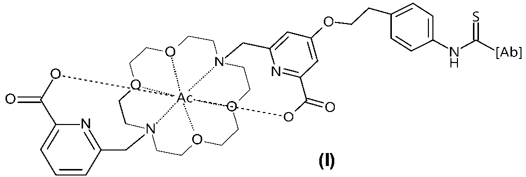 wherein [Ab] is a monoclonal antibody or an antigen-binding fragment thereof capable of binding to CEACAM5 comprising
i) a variable heavy chain comprising SEQ ID NO: 4 and/or a variable light chain domain comprising SEQ ID NO: 9; or
ii) a variable heavy chain domain comprising SEQ ID NO: 14 and/or a variable light chain domain comprising SEQ ID NO: 19; or
iii) a variable heavy chain domain comprising SEQ ID NO: 24 and/or a variable light chain domain comprising SEQ ID NO: 29; or
iv) a variable heavy chain domain comprising SEQ ID NO: 34 and/or a variable light chain domain comprising SEQ ID NO: 39; or
v) a variable heavy chain domain comprising SEQ ID NO: 44 and/or a variable light chain domain comprising SEQ ID NO: 49; or
vi) a variable heavy chain domain comprising SEQ ID NO: 54 and/or a variable light chain domain comprising SEQ ID NO: 59; or
vii) a variable heavy chain domain comprising SEQ ID NO: 64 and/or a variable light chain domain comprising SEQ ID NO: 69; or
viii) a variable heavy chain domain comprising SEQ ID NO: 74 and/or a variable light chain domain comprising SEQ ID NO: 79, or
ix) a variable heavy chain domain comprising SEQ ID NO: 84 and/or a variable light chain domain comprising SEQ ID NO: 89.

In a further embodiment, the present disclosure relates to a tissue-targeting compound of formula (I) 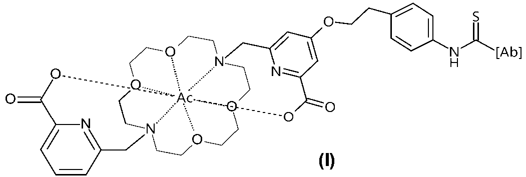 wherein [Ab] is a monoclonal antibody or an antigen-binding fragment thereof capable of binding to CEACAM5 comprising
i) a variable heavy chain comprising SEQ ID NO: 4 and/or a variable light chain domain comprising SEQ ID NO: 9; or
ii) a variable heavy chain domain comprising SEQ ID NO: 14 and/or a variable light chain domain comprising SEQ ID NO: 19; or
iii) a variable heavy chain domain comprising SEQ ID NO: 24 and/or a variable light chain domain comprising SEQ ID NO: 29; or
iv) a variable heavy chain domain comprising SEQ ID NO: 34 and/or a variable light chain domain comprising SEQ ID NO: 39; or
v) a variable heavy chain domain comprising SEQ ID NO: 44 and/or a variable light chain domain comprising SEQ ID NO: 49; or
vi) a variable heavy chain domain comprising SEQ ID NO: 54 and/or a variable light chain domain comprising SEQ ID NO: 59; or
vii) a variable heavy chain domain comprising SEQ ID NO: 64 and/or a variable light chain domain comprising SEQ ID NO: 69; or
viii) a variable heavy chain domain comprising SEQ ID NO: 74 and/or a variable light chain domain comprising SEQ ID NO: 79, or
ix) a variable heavy chain domain comprising SEQ ID NO: 84 and/or a variable light chain domain comprising SEQ ID NO: 89,
wherein [Ab] is bound to the to the rest of the molecule via a terminal amino group of a lysine residue of the monoclonal antibody or antigen-binding fragment thereof.

In a further embodiment, the present disclosure relates to a tissue-targeting compound of formula (I) 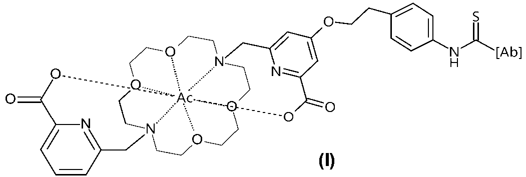 wherein [Ab] is a monoclonal antibody or an antigen-binding fragment thereof capable of binding to CEACAM5 comprising
i) a heavy chain comprising SEQ ID NO: 5 and/or a light chain comprising SEQ ID NO: 10; or
ii) a heavy chain comprising SEQ ID NO: 15 and/or a light chain comprising SEQ ID NO: 20; or
iii) a heavy chain comprising SEQ ID NO: 25 and/or a light chain comprising SEQ ID NO: 30; or
iv) a heavy chain comprising SEQ ID NO: 35 and/or a light chain comprising SEQ ID NO: 40; or
v) a heavy chain comprising SEQ ID NO: 45 and/or a light chain comprising SEQ ID NO: 50 or
vi) a heavy chain comprising SEQ ID NO: 55 and/or a light chain comprising SEQ ID NO: 60, or
vii) a heavy chain comprising SEQ ID NO: 65 and/or a light chain comprising SEQ ID NO: 70; or
viii) a heavy chain comprising SEQ ID NO: 75 and/or a light chain comprising SEQ ID NO: 80; or
ix) a heavy chain comprising SEQ ID NO: 85 and/or a light chain comprising SEQ ID NO: 90 or
x) a heavy chain comprising SEQ ID NO: 95; or
xi) a heavy chain comprising SEQ ID NO: 104; or
xii) a heavy chain comprising SEQ ID NO: 113; or
xiii) a heavy chain comprising SEQ ID NO: 122; or
xiv) a heavy chain comprising SEQ ID NO: 131; or
xv) a heavy chain comprising SEQ ID NO: 140.

In a further embodiment, the present disclosure relates to a tissue-targeting compound of formula (I) 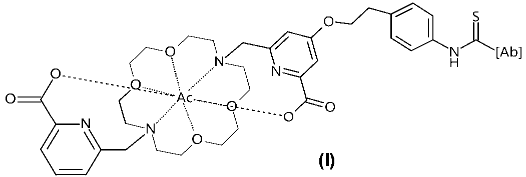 wherein [Ab] is a monoclonal antibody or an antigen-binding fragment thereof capable of binding to CEACAM5 comprising
i) a heavy chain comprising SEQ ID NO: 5 and/or a light chain comprising SEQ ID NO: 10; or
ii) a heavy chain comprising SEQ ID NO: 15 and/or a light chain comprising SEQ ID NO: 20; or
iii) a heavy chain comprising SEQ ID NO: 25 and/or a light chain comprising SEQ ID NO: 30; or
iv) a heavy chain comprising SEQ ID NO: 35 and/or a light chain comprising SEQ ID NO: 40; or
v) a heavy chain comprising SEQ ID NO: 45 and/or a light chain comprising SEQ ID NO: 50 or
vi) a heavy chain comprising SEQ ID NO: 55 and/or a light chain comprising SEQ ID NO: 60, or
vii) a heavy chain comprising SEQ ID NO: 65 and/or a light chain comprising SEQ ID NO: 70; or
viii) a heavy chain comprising SEQ ID NO: 75 and/or a light chain comprising SEQ ID NO: 80; or
ix) a heavy chain comprising SEQ ID NO: 85 and/or a light chain comprising SEQ ID NO: 90.

In a further embodiment, the present disclosure relates to a tissue-targeting compound of formula (I) 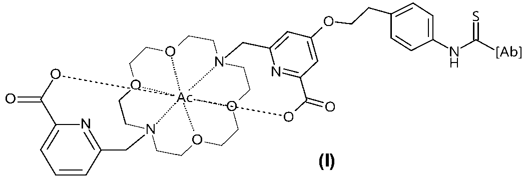 wherein [Ab] is a monoclonal antibody or an antigen-binding fragment thereof capable of binding to CEACAM5 comprising
i) a heavy chain comprising SEQ ID NO: 5 and/or a light chain comprising SEQ ID NO: 10; or
ii) a heavy chain comprising SEQ ID NO: 15 and/or a light chain comprising SEQ ID NO: 20; or
iii) a heavy chain comprising SEQ ID NO: 25 and/or a light chain comprising SEQ ID NO: 30; or
iv) a heavy chain comprising SEQ ID NO: 35 and/or a light chain comprising SEQ ID NO: 40; or
v) a heavy chain comprising SEQ ID NO: 45 and/or a light chain comprising SEQ ID NO: 50 or
vi) a heavy chain comprising SEQ ID NO: 55 and/or a light chain comprising SEQ ID NO: 60, or
vii) a heavy chain comprising SEQ ID NO: 65 and/or a light chain comprising SEQ ID NO: 70; or
viii) a heavy chain comprising SEQ ID NO: 75 and/or a light chain comprising SEQ ID NO: 80; or
ix) a heavy chain comprising SEQ ID NO: 85 and/or a light chain comprising SEQ ID NO: 90
wherein [Ab] is bound to the to the rest of the molecule via a terminal amino group of a lysine residue of the monoclonal antibody or antigen-binding fragment thereof.

In particular embodiments, the isolated monoclonal antibodies comprised in the targeted actinium conjugates according to the present disclosure are IgG1, IgG2, IgG3 or IgG4 antibodies. Most particularly, the isolated monoclonal antibodies according to the present disclosure are IgG1 antibodies.

Thus, in particularly preferred embodiments, the present disclosure comprises tissue-targeting compounds of formula (I) 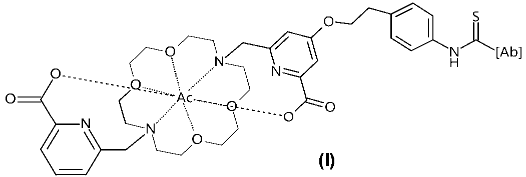 wherein [Ab] is a monoclonal antibody or an antigen-binding fragment thereof capable of binding to CEACAM5 as described supra, wherein [Ab] is an IgG1, IgG2, IgG3 or IgG4 monoclonal antibody, preferably an IgG1 monoclonal antibody.

The tissue-targeting compounds of formula (I) of the present disclosure comprise a chelating moiety capable of binding actinium. Preferably, the chelating moiety comprises an 18-membered chelating moiety. Preferably, the chelating moiety comprises the macrocyclic chelator N,N'-bis[(6-carboxy-2-pyridil)methyl]-4,13-diaza-18-crown-6 (macropa).

The macrocyclic chelator macropa has been described, for example, in Thiele et al., Angew. Chem. Int. Ed. 2017, 56(46), 14712 and references therein. Macropa chelators and derivatives thereof have also been described in WO2018/183906 and WO2020/106886 and references therein.

The actinium-complexating chelating moiety preferably terminates in an isothiocyanate or carboxylic acid moiety in order to facilitate its coupling with an anti-CEACAM5 antibody or an antigen-binding fragment thereof. When the macropa chelator is used, one of the pyridine rings of the macropa chelator is typically substituted so that it can be coupled to the anti-CEACAM5 antibody or an antigen-binding fragment thereof. This can be a simple functional group (e.g., a carboxylic acid -COOH) or a more complex chemical structure, as long as it is capable of being coupled to an anti-CEACAM5 antibody or an antigen-binding fragment thereof. In particular, the substituent on the pyridine ring of the macropa chelator preferably terminates in an isothiocyanate or carboxylic acid moiety.

The anti-CEACAM5 antibody or an antigen-binding fragment thereof is preferably coupled to the rest of the molecule forming the tissue-targeting compounds of the present disclosure through a Lysine residue of the antibody. Thus, the actinium-complexating chelating moiety and the anti-CEACAM5 antibody or the antigen-binding fragment thereof can be coupled via an amide or thiourea moiety. When using the preferred macrocyclic chelator macropa, a terminal isothiocyanate or carboxylic acid moiety is coupled with a terminal amino group of a Lysine residue in the anti-CEACAM5 antibody or an antigen-binding fragment thereof, leading to the formation of a thiourea or an amide group, respectively. Thus, the preferred actinium-complexating chelating moiety comprising a macropa chelator is attached to an anti-CEACAM5 antibody or an antigen-binding fragment thereof via a thiourea or an amide bond derived from a terminal amino group of a Lysine residue of said anti-CEACAM5 antibody or an antigen-binding fragment thereof.

### Combinations

In a further aspect, the present disclosure relates to combinations, preferably pharmaceutical combinations comprising a tissue-targeting compound of formula (I) as described above and a further pharmaceutical agent. Said combinations are useful in therapy, preferably in treating hyperproliferative diseases such as cancer.

Combinations comprising effective amounts of a tissue-targeting compound of formula (I) as described above and a further pharmaceutical agent achieve a greater therapeutic efficacy than when either compound is used alone.

The relative ratios of each compound in the combination can also be selected based on their respective mechanisms of action and the disease biology. The relative ratios of each compound can vary widely.

The release of one or more agents of the combination can also be controlled, where appropriate, to provide the desired therapeutic activity when in a single dosage form, combination pack, kit or when in separate independent dosage forms.

Generally, the combinations of the present disclosure will serve to:
(1) yield better efficacy in reducing the growth of a tumor or even eliminate the tumor as compared to administration of either agent alone,
(2) provide for the administration of lesser amounts of the administered targeted compounds and/or chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time until tumor progression, and/or
(8) yield efficacy and tolerability results at least as good as those of the agents used alone, compared to known instances where other cancer agent combinations produce inhibitory effects.

The tissue-targeting compound of formula (I) is a compound of the present disclosure as described herein.

The further pharmaceutical agent may be selected from an immune checkpoint inhibitor in order to potentiate the immune system's ability to recognize and destroy cancer cells. The further pharmaceutical agent may be a targeted small molecule inhibitor that disrupts specific signaling pathways critical for tumor growth and survival. Depending on the indication and use of the tissue-targeting compound of formula (I), combinations with established chemotherapeutic agents such as angiogenic drugs or drugs targeting DNA damage repair mechanisms, (e.g., PARP inhibitors) is also possible.

Thus, in one embodiment, the present disclosure relates to a combination comprising
(i) a tissue-targeting compound of formula (I) 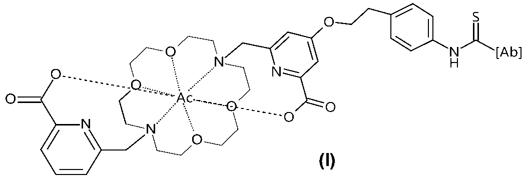 wherein [Ab] is a monoclonal antibody or an antigen-binding fragment thereof having binding affinity for CEACAM5 according to the first aspect of the present disclosure, and
(ii) a further pharmaceutical agent.

In a further embodiment, the present disclosure relates to a combination comprising
(i) a tissue-targeting compound of formula (I) 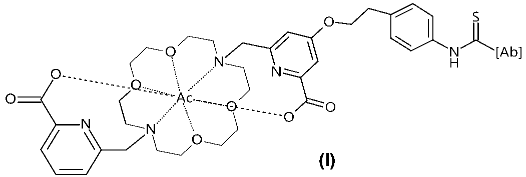 wherein [Ab] is a monoclonal antibody or an antigen-binding fragment thereof having binding affinity for CEACAM5 comprising
   i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
   ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
   iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
   iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
   v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
   vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
   vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
   viii)a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
   ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291), or
   x) a heavy chain antigen-binding region that comprises an H-CDR1 comprising SEQ ID NO: 91, an H-CDR2 comprising SEQ ID NO: 92, and an H-CDR3 comprising SEQ ID NO: 93 and a light chain antigen-binding region that comprises an L-CDR1 comprising SEQ ID NO: 96, an L-CDR2 comprising SEQ ID NO: 97, and an L-CDR3 comprising SEQ ID NO:98 (DPP-64106), or
   xi) a heavy chain antigen-binding region that comprises an H-CDR1 comprising SEQ ID NO: 100, an H-CDR2 comprising SEQ ID NO: 101, and an H-CDR3 comprising SEQ ID NO: 102 and a light chain antigen-binding region that comprises an L-CDR1 comprising SEQ ID NO: 105, an L-CDR2 comprising SEQ ID NO: 106, and an L-CDR3 comprising SEQ ID NO:107 (DPP-64107), or
   xii) a heavy chain antigen-binding region that comprises an H-CDR1 comprising SEQ ID NO: 109, an H-CDR2 comprising SEQ ID NO: 110 and an H-CDR3 comprising SEQ ID NO: 111 and a light chain antigen-binding region that comprises an L-CDR1 comprising SEQ ID NO: 114, an L-CDR2 comprising SEQ ID NO: 115, and an L-CDR3 comprising SEQ ID NO:116 (DPP-64204), or
   xiii)a heavy chain antigen-binding region that comprises an H-CDR1 comprising SEQ ID NO: 118, an H-CDR2 comprising SEQ ID NO: 119, and an H-CDR3 comprising SEQ ID NO: 120 and a light chain antigen-binding region that comprises an L-CDR1 comprising SEQ ID NO: 123, an L-CDR2 comprising SEQ ID NO: 124, and an L-CDR3 comprising SEQ ID NO:125 (DPP-64209), or
   xiv) a heavy chain antigen-binding region that comprises an H-CDR1 comprising SEQ ID NO: 127, an H-CDR2 comprising SEQ ID NO: 128, and an H-CDR3 comprising SEQ ID NO: 129 and a light chain antigen-binding region that comprises an L-CDR1 comprising SEQ ID NO: 132, an L-CDR2 comprising SEQ ID NO: 133, and an L-CDR3 comprising SEQ ID NO:134 (DPP-64227), or
   xv) a heavy chain antigen-binding region that comprises an H-CDR1 comprising SEQ ID NO: 136, an H-CDR2 comprising SEQ ID NO: 137, and an H-CDR3 comprising SEQ ID NO: 138 and a light chain antigen-binding region that comprises an L-CDR1 comprising SEQ ID NO: 141, an L-CDR2 comprising SEQ ID NO: 142, and an L-CDR3 comprising SEQ ID NO:143 (DPP-64262),
      preferably wherein [Ab] is bound to the to the rest of the molecule via a terminal amino group of a lysine residue of the monoclonal antibody or antigen-binding fragment thereof,
   and
(ii) a further pharmaceutical agent.

In a further embodiment, the present disclosure relates to a combination comprising
(i) a tissue-targeting compound of formula (I) 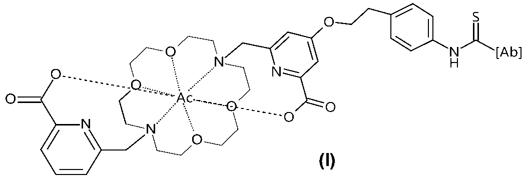 wherein [Ab] is a monoclonal antibody or an antigen-binding fragment thereof having binding affinity for CEACAM5 comprising
   i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
   ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
   iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
   iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
   v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
   vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
   vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
   viii)a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
   ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291),
   preferably wherein [Ab] is bound to the to the rest of the molecule via a terminal amino group of a lysine residue of the monoclonal antibody or antigen-binding fragment thereof. and
(ii) a further pharmaceutical agent.

### Synthesis of targeted radionuclide conjugates

In a further aspect, the present disclosure relates to a process for the preparation of a tissue-targeting compound of formula (I) 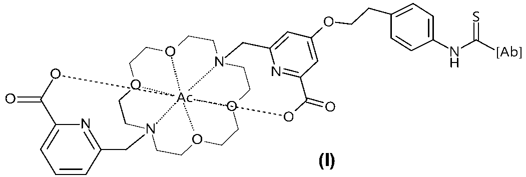 wherein [Ab] is a monoclonal antibody or an antigen-binding fragment thereof having binding affinity for CEACAM5 selected from an isolated antibody or antigen-binding fragment according to the first aspect of the present disclosure,
said method comprising
(i) coupling a chelating moiety of formula (II) 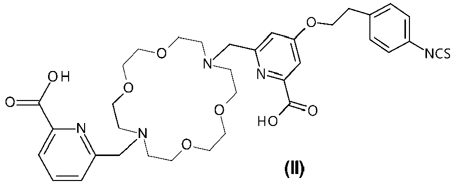 with a monoclonal antibody or an antigen-binding fragment thereof having binding affinity for CEACAM5 selected from an isolated antibody or antigen-binding fragment according to the first aspect of the present disclosure, and
(ii) contacting the resulting product with an aqueous solution comprising an ion of at least one alpha-emitting actinium isotope.

In a further embodiment, the present disclosure relates to a process for the preparation of a tissue-targeting compound of formula (I) 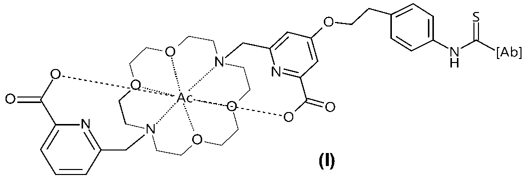 wherein [Ab] is a monoclonal antibody or an antigen-binding fragment thereof having binding affinity for CEACAM5 selected from an isolated antibody or antigen-binding fragment according to the first aspect of the present disclosure,
said method comprising
(i) coupling a chelating moiety of formula (II) 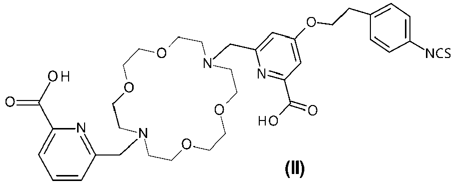 with a monoclonal antibody or an antigen-binding fragment thereof having binding affinity for CEACAM5 selected from an isolated antibody or antigen-binding fragment according to the first aspect of the present disclosure via a Lysine residue of the monoclonal antibody or antigen-binding fragment thereof, and
(ii) contacting the resulting product with an aqueous solution comprising an ion of at least one alpha-emitting actinium isotope.

In a further embodiment, the present disclosure relates to a process for the preparation of a tissue-targeting compound of formula (I) 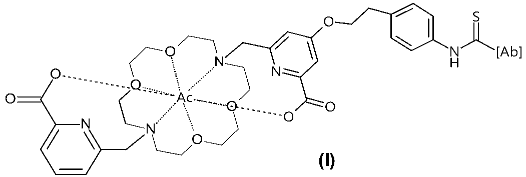 wherein [Ab] is a monoclonal antibody or an antigen-binding fragment thereof having binding affinity for CEACAM5 comprising
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291), or
x) a heavy chain antigen-binding region that comprises an H-CDR1 comprising SEQ ID NO: 91, an H-CDR2 comprising SEQ ID NO: 92, and an H-CDR3 comprising SEQ ID NO: 93 and a light chain antigen-binding region that comprises an L-CDR1 comprising SEQ ID NO: 96, an L-CDR2 comprising SEQ ID NO: 97, and an L-CDR3 comprising SEQ ID NO:98 (DPP-64106), or
xi) a heavy chain antigen-binding region that comprises an H-CDR1 comprising SEQ ID NO: 100, an H-CDR2 comprising SEQ ID NO: 101, and an H-CDR3 comprising SEQ ID NO: 102 and a light chain antigen-binding region that comprises an L-CDR1 comprising SEQ ID NO: 105, an L-CDR2 comprising SEQ ID NO: 106, and an L-CDR3 comprising SEQ ID NO:107 (DPP-64107), or
xii) a heavy chain antigen-binding region that comprises an H-CDR1 comprising SEQ ID NO: 109, an H-CDR2 comprising SEQ ID NO: 110 and an H-CDR3 comprising SEQ ID NO: 111 and a light chain antigen-binding region that comprises an L-CDR1 comprising SEQ ID NO: 114, an L-CDR2 comprising SEQ ID NO: 115, and an L-CDR3 comprising SEQ ID NO:116 (DPP-64204), or
xiii) a heavy chain antigen-binding region that comprises an H-CDR1 comprising SEQ ID NO: 118, an H-CDR2 comprising SEQ ID NO: 119, and an H-CDR3 comprising SEQ ID NO: 120 and a light chain antigen-binding region that comprises an L-CDR1 comprising SEQ ID NO: 123, an L-CDR2 comprising SEQ ID NO: 124, and an L-CDR3 comprising SEQ ID NO:125 (DPP-64209), or
xiv) a heavy chain antigen-binding region that comprises an H-CDR1 comprising SEQ ID NO: 127, an H-CDR2 comprising SEQ ID NO: 128, and an H-CDR3 comprising SEQ ID NO: 129 and a light chain antigen-binding region that comprises an L-CDR1 comprising SEQ ID NO: 132, an L-CDR2 comprising SEQ ID NO: 133, and an L-CDR3 comprising SEQ ID NO:134 (DPP-64227), or
xv) a heavy chain antigen-binding region that comprises an H-CDR1 comprising SEQ ID NO: 136, an H-CDR2 comprising SEQ ID NO: 137, and an H-CDR3 comprising SEQ ID NO: 138 and a light chain antigen-binding region that comprises an L-CDR1 comprising SEQ ID NO: 141, an L-CDR2 comprising SEQ ID NO: 142, and an L-CDR3 comprising SEQ ID NO:143 (DPP-64262),
said method comprising
(i) coupling a chelating moiety of formula (II) 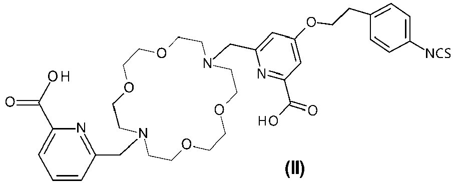 with a monoclonal antibody or an antigen-binding fragment thereof having binding affinity for CEACAM5 comprising
   i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
   ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
   iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
   iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
   v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
   vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
   vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
   viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
   ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291), or
   x) a heavy chain antigen-binding region that comprises an H-CDR1 comprising SEQ ID NO: 91, an H-CDR2 comprising SEQ ID NO: 92, and an H-CDR3 comprising SEQ ID NO: 93 and a light chain antigen-binding region that comprises an L-CDR1 comprising SEQ ID NO: 96, an L-CDR2 comprising SEQ ID NO: 97, and an L-CDR3 comprising SEQ ID NO:98 (DPP-64106), or
   xi) a heavy chain antigen-binding region that comprises an H-CDR1 comprising SEQ ID NO: 100, an H-CDR2 comprising SEQ ID NO: 101, and an H-CDR3 comprising SEQ ID NO: 102 and a light chain antigen-binding region that comprises an L-CDR1 comprising SEQ ID NO: 105, an L-CDR2 comprising SEQ ID NO: 106, and an L-CDR3 comprising SEQ ID NO:107 (DPP-64107), or
   xii) a heavy chain antigen-binding region that comprises an H-CDR1 comprising SEQ ID NO: 109, an H-CDR2 comprising SEQ ID NO: 110 and an H-CDR3 comprising SEQ ID NO: 111 and a light chain antigen-binding region that comprises an L-CDR1 comprising SEQ ID NO: 114, an L-CDR2 comprising SEQ ID NO: 115, and an L-CDR3 comprising SEQ ID NO:116 (DPP-64204), or
   xiii) a heavy chain antigen-binding region that comprises an H-CDR1 comprising SEQ ID NO: 118, an H-CDR2 comprising SEQ ID NO: 119, and an H-CDR3 comprising SEQ ID NO: 120 and a light chain antigen-binding region that comprises an L-CDR1 comprising SEQ ID NO: 123, an L-CDR2 comprising SEQ ID NO: 124, and an L-CDR3 comprising SEQ ID NO:125 (DPP-64209), or
   xiv) a heavy chain antigen-binding region that comprises an H-CDR1 comprising SEQ ID NO: 127, an H-CDR2 comprising SEQ ID NO: 128, and an H-CDR3 comprising SEQ ID NO: 129 and a light chain antigen-binding region that comprises an L-CDR1 comprising SEQ ID NO: 132, an L-CDR2 comprising SEQ ID NO: 133, and an L-CDR3 comprising SEQ ID NO:134 (DPP-64227), or
   xv) a heavy chain antigen-binding region that comprises an H-CDR1 comprising SEQ ID NO: 136, an H-CDR2 comprising SEQ ID NO: 137, and an H-CDR3 comprising SEQ ID NO: 138 and a light chain antigen-binding region that comprises an L-CDR1 comprising SEQ ID NO: 141, an L-CDR2 comprising SEQ ID NO: 142, and an L-CDR3 comprising SEQ ID NO:143 (DPP-64262), via a Lysine residue of the monoclonal antibody or antigen-binding fragment thereof, and
(ii) contacting the resulting product with an aqueous solution comprising an ion of at least one alpha-emitting actinium isotope.

In a further embodiment, the present disclosure relates to a process for the preparation of a tissue-targeting compound of formula (I) 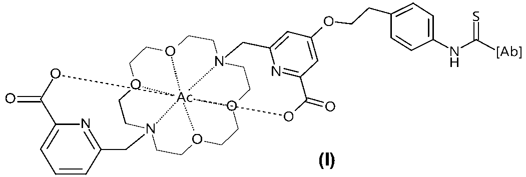 wherein [Ab] is a monoclonal antibody or an antigen-binding fragment thereof having binding affinity for CEACAM5 comprising,
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291),
said method comprising
(i) coupling a chelating moiety of formula (II) 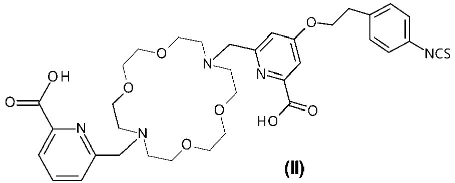 with a monoclonal antibody or an antigen-binding fragment thereof having binding affinity for CEACAM5 comprising
   i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
   ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
   iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
   iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
   v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
   vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
   vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
   viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
   ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291),
   via a Lysine residue of the monoclonal antibody or antigen-binding fragment thereof, and
(ii) contacting the resulting product with an aqueous solution comprising an ion of at least one alpha-emitting actinium isotope.

In step (i), a chelating moiety of formula (II) 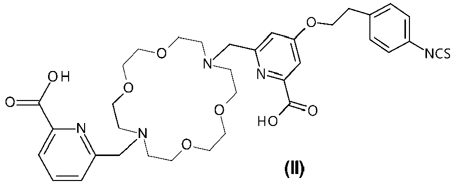 is coupled with a monoclonal antibody or an antigen-binding fragment thereof having binding affinity for CEACAM5. Preferably, the monoclonal antibody or an antigen-binding fragment thereof having binding affinity for CEACAM5 is selected from an isolated antibody or antigen-binding fragment according to the first aspect of the present disclosure.

More preferably, the monoclonal antibody or an antigen-binding fragment thereof having binding affinity for CEACAM5 comprises
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291), or
x) a heavy chain antigen-binding region that comprises an H-CDR1 comprising SEQ ID NO: 91, an H-CDR2 comprising SEQ ID NO: 92, and an H-CDR3 comprising SEQ ID NO: 93 and a light chain antigen-binding region that comprises an L-CDR1 comprising SEQ ID NO: 96, an L-CDR2 comprising SEQ ID NO: 97, and an L-CDR3 comprising SEQ ID NO:98 (DPP-64106), or
xi) a heavy chain antigen-binding region that comprises an H-CDR1 comprising SEQ ID NO: 100, an H-CDR2 comprising SEQ ID NO: 101, and an H-CDR3 comprising SEQ ID NO: 102 and a light chain antigen-binding region that comprises an L-CDR1 comprising SEQ ID NO: 105, an L-CDR2 comprising SEQ ID NO: 106, and an L-CDR3 comprising SEQ ID NO:107 (DPP-64107), or
xii) a heavy chain antigen-binding region that comprises an H-CDR1 comprising SEQ ID NO: 109, an H-CDR2 comprising SEQ ID NO: 110 and an H-CDR3 comprising SEQ ID NO: 111 and a light chain antigen-binding region that comprises an L-CDR1 comprising SEQ ID NO: 114, an L-CDR2 comprising SEQ ID NO: 115, and an L-CDR3 comprising SEQ ID NO:116 (DPP-64204), or
xiii) a heavy chain antigen-binding region that comprises an H-CDR1 comprising SEQ ID NO: 118, an H-CDR2 comprising SEQ ID NO: 119, and an H-CDR3 comprising SEQ ID NO: 120 and a light chain antigen-binding region that comprises an L-CDR1 comprising SEQ ID NO: 123, an L-CDR2 comprising SEQ ID NO: 124, and an L-CDR3 comprising SEQ ID NO:125 (DPP-64209), or
xiv) a heavy chain antigen-binding region that comprises an H-CDR1 comprising SEQ ID NO: 127, an H-CDR2 comprising SEQ ID NO: 128, and an H-CDR3 comprising SEQ ID NO: 129 and a light chain antigen-binding region that comprises an L-CDR1 comprising SEQ ID NO: 132, an L-CDR2 comprising SEQ ID NO: 133, and an L-CDR3 comprising SEQ ID NO:134 (DPP-64227), or
xv) a heavy chain antigen-binding region that comprises an H-CDR1 comprising SEQ ID NO: 136, an H-CDR2 comprising SEQ ID NO: 137, and an H-CDR3 comprising SEQ ID NO: 138 and a light chain antigen-binding region that comprises an L-CDR1 comprising SEQ ID NO: 141, an L-CDR2 comprising SEQ ID NO: 142, and an L-CDR3 comprising SEQ ID NO:143 (DPP-64262).

More preferably, the monoclonal antibody or an antigen-binding fragment thereof having binding affinity for CEACAM5 comprises
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291).

Preferably, the chelating moiety of formula (II) is coupled to the anti-CEACAM5 antibody or antigen-binding fragment thereof selected from an isolated antibody or antigen-binding fragment according to the first aspect of the present disclosure via a Lysine residue of the monoclonal antibody, thus forming a thiourea moiety that links the chelating moiety with the anti-CEACAM5 antibody or antigen-binding fragment thereof.

Preferably, the coupling in step (i) is carried out under basic pH, i.e., at a pH higher than 7. More preferably, the coupling in step (i) is carried out at a pH of from 8 to 10, most preferably at a pH of 9.

After step (i), a tissue-targeting chelator of formula (III) is generated. Thus, in further embodiments, the present disclosure relates to a tissue-targeting chelator of formula (III) 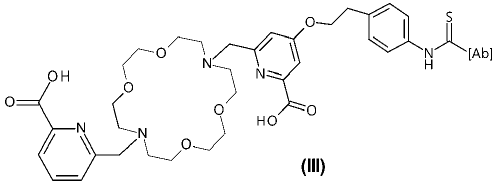 wherein [Ab] is a monoclonal antibody or an antigen-binding fragment thereof selected from an isolated antibody or antigen-binding fragment according to the first aspect of the present disclosure having binding affinity for CEACAM5, preferably wherein [Ab] is bound to the to the rest of the molecule via a terminal amino group of a lysine residue of the monoclonal antibody or antigen-binding fragment thereof.

In a preferred embodiment, the monoclonal antibody or an antigen-binding fragment thereof having binding affinity for CEACAM5 is selected from an isolated antibody or antigen-binding fragment according to the first aspect of the present disclosure, preferably wherein [Ab] is bound to the to the rest of the molecule via a terminal amino group of a lysine residue of the monoclonal antibody or antigen-binding fragment thereof.

In a preferred embodiment, the monoclonal antibody or an antigen-binding fragment thereof having binding affinity for CEACAM5 in the tissue-targeting chelator of formula (III) comprises
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291), or
x) a heavy chain antigen-binding region that comprises an H-CDR1 comprising SEQ ID NO: 91, an H-CDR2 comprising SEQ ID NO: 92, and an H-CDR3 comprising SEQ ID NO: 93 and a light chain antigen-binding region that comprises an L-CDR1 comprising SEQ ID NO: 96, an L-CDR2 comprising SEQ ID NO: 97, and an L-CDR3 comprising SEQ ID NO:98 (DPP-64106), or
xi) a heavy chain antigen-binding region that comprises an H-CDR1 comprising SEQ ID NO: 100, an H-CDR2 comprising SEQ ID NO: 101, and an H-CDR3 comprising SEQ ID NO: 102 and a light chain antigen-binding region that comprises an L-CDR1 comprising SEQ ID NO: 105, an L-CDR2 comprising SEQ ID NO: 106, and an L-CDR3 comprising SEQ ID NO:107 (DPP-64107), or
xii) a heavy chain antigen-binding region that comprises an H-CDR1 comprising SEQ ID NO: 109, an H-CDR2 comprising SEQ ID NO: 110 and an H-CDR3 comprising SEQ ID NO: 111 and a light chain antigen-binding region that comprises an L-CDR1 comprising SEQ ID NO: 114, an L-CDR2 comprising SEQ ID NO: 115, and an L-CDR3 comprising SEQ ID NO:116 (DPP-64204), or
xiii) a heavy chain antigen-binding region that comprises an H-CDR1 comprising SEQ ID NO: 118, an H-CDR2 comprising SEQ ID NO: 119, and an H-CDR3 comprising SEQ ID NO: 120 and a light chain antigen-binding region that comprises an L-CDR1 comprising SEQ ID NO: 123, an L-CDR2 comprising SEQ ID NO: 124, and an L-CDR3 comprising SEQ ID NO:125 (DPP-64209), or
xiv) a heavy chain antigen-binding region that comprises an H-CDR1 comprising SEQ ID NO: 127, an H-CDR2 comprising SEQ ID NO: 128, and an H-CDR3 comprising SEQ ID NO: 129 and a light chain antigen-binding region that comprises an L-CDR1 comprising SEQ ID NO: 132, an L-CDR2 comprising SEQ ID NO: 133, and an L-CDR3 comprising SEQ ID NO:134 (DPP-64227), or
xv) a heavy chain antigen-binding region that comprises an H-CDR1 comprising SEQ ID NO: 136, an H-CDR2 comprising SEQ ID NO: 137, and an H-CDR3 comprising SEQ ID NO: 138 and a light chain antigen-binding region that comprises an L-CDR1 comprising SEQ ID NO: 141, an L-CDR2 comprising SEQ ID NO: 142, and an L-CDR3 comprising SEQ ID NO:143 (DPP-64262),
preferably wherein [Ab] is bound to the to the rest of the molecule via a terminal amino group of a lysine residue of the monoclonal antibody or antigen-binding fragment thereof.

In a preferred embodiment, the monoclonal antibody or an antigen-binding fragment thereof having binding affinity for CEACAM5 in the tissue-targeting chelator of formula (III) comprises
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291)
preferably wherein [Ab] is bound to the to the rest of the molecule via a terminal amino group of a lysine residue of the monoclonal antibody or antigen-binding fragment thereof.

In step (ii), the product resulting from the coupling in step (i) is contacted with an aqueous solution comprising an ion of at least one alpha-emitting actinium isotope. Preferably, the alpha-emitting actinium isotope is 225Ac. Preferably, the ion of at least one alpha-emitting actinium isotope is ²²⁵Ac having a triple positive charge, i.e., ²²⁵Ac³⁺. The present disclosure allows for the execution of step (ii) at room temperature, i.e., at a temperature that is lower than the one required for the preparation of the corresponding DOTA conjugates, which require higher temperatures (60°C).

### Therapeutic Methods

Therapeutic methods involve administering to a subject in need of treatment a therapeutically effective amount of an antibody or an antigen-binding fragment thereof selected from an isolated antibody or antigen-binding fragment according to the first aspect of the present disclosure or a variant thereof and/or a targeted actinium conjugate (TAC) and/or a tissue-targeting compound of formula (I) as described by the present disclosure.

### Uses

In a further aspect, the isolated antibodies and/or the tissue-targeting compounds of formula (I) of the present disclosure and/or the combinations comprising tissue-targeting compounds of formula (I) and a further pharmaceutical agent of the present disclosure may be used for the treatment of disease in a human or in a non-human animal subject. In particular, the isolated antibodies and/or the tissue-targeting compounds of formula (I) of the present disclosure and/or the combinations comprising tissue-targeting compounds of formula (I) and a further pharmaceutical agent of the present disclosure may be used for the treatment of hyperplastic and/or neoplastic diseases, such as cancer. There are a number of targets which are known to be associated with hyperplastic and neoplastic disease. These include certain receptors, cell surface proteins, transmembrane proteins and proteins/peptides found in the extracellular matrix in the vicinity of diseased cells. Examples of cell-surface receptors and antigens which may be associated with neoplastic disease include but are not limited to CD22, CD33, FGFR2 (CD332), CEACAM5, HER2, CEACAM5 and Mesothelin. In one preferred embodiment, the isolated antibodies and/or the tissue-targeting compounds of formula (I) of the present disclosure and/or the combinations comprising tissue-targeting compounds of formula (I) of the present disclosure comprise a tissue-targeting moiety comprising a monoclonal antibody having specificity and/or binding affinity for CEACAM5. This may be reflected, for example by having 50 or more times greater binding affinity for cells expressing CEACAM5 than for non-CEACAM5 expressing cells (preferably at least 100 time greater, more preferably at least 300 times greater). It is believed that CEACAM5 is expressed and/or over-expressed in cells having certain disease states (as indicated herein) and thus the CEACAM5 specific binder may serve to target the complex to such disease-affected cells. Similarly, a tissue targeting moiety may bind to cell-surface markers (e.g. CEACAM5 receptors) present on cells in the vicinity of disease affected cells. CEACAM5 cell-surface markers may be more heavily expressed on diseased cell surfaces than on healthy cell surfaces or more heavily expressed on cell surfaces during periods of growth or replication than during dormant phases. In one embodiment, a CEACAM5-specific tissue-targeting binder may be used in combination with another binder for a disease-specific cell-surface marker, thus giving a dual- binding complex. Tissue-targeting binders for CEACAM5 will typically be peptides or proteins, as discussed herein.

The various aspects of the disclosure as described herein relate to treatment of disease, particularly for the selective targeting of diseased tissue, as well as relating to complexes, conjugates, medicaments, formulation, kits etc. useful in such methods. In all aspects, the diseased tissue may reside at a single site in the body (for example in the case of a localized solid tumor) or may reside at a plurality of sites (for example where several joints are affected in arthritis or in the case of a distributed or metastasized cancerous disease).

The diseased tissue to be targeted may be at a soft tissue site, at a calcified tissue site or a plurality of sites which may all be in soft tissue, all in calcified tissue or may include at least one soft tissue site and/or at least one calcified tissue site. In one embodiment, at least one soft tissue site is targeted. The sites of targeting and the sites of origin of the disease may be the same, but alternatively may be different (such as where metastatic sites are specifically targeted). Where more than one site is involved, this may include the site of origin or may be a plurality of secondary sites.

The term "soft tissue" is used herein to indicate tissues which do not have a "hard" mineralized matrix. In particular, soft tissues as used herein may be any tissues that are not skeletal tissues. Correspondingly, "soft tissue disease" as used herein indicates a disease occurring in a "soft tissue" as used herein. The disclosure is particularly suitable for the treatment of cancers and "soft tissue disease" thus encompasses carcinomas, sarcomas, myelomas, leukemias, lymphomas and mixed type cancers occurring in any "soft" (i.e. non-mineralized) tissue, as well as other non-cancerous diseases of such tissue. Cancerous "soft tissue disease" includes solid tumors occurring in soft tissues as well as metastatic and micro-metastatic tumors. Indeed, the soft tissue disease may comprise a primary solid tumor of soft tissue and at least one metastatic tumor of soft tissue in the same patient. Alternatively, the "soft tissue disease" may consist of only a primary tumor or only metastases with the primary tumor being a skeletal disease.

The isolated antibodies and/or the tissue-targeting compounds of formula (I) of the present disclosure and/or the combinations comprising tissue-targeting compounds of formula (I) and a further pharmaceutical agent of the present disclosure preferably comprise a tissue-targeting moiety comprising a monoclonal antibody having binding affinity for the carcinoembryonic antigen-related cell adhesion molecule 5 (CEACAM5). CEACAM5 expression in tumor cells has been observed in different types of diseases, in particular hyperproliferative diseases such as cancer. Examples of diseases and/or neoplasms suitable for treatment using the isolated antibodies and/or the tissue-targeting compounds of formula (I) of the present disclosure and/or the combinations comprising tissue-targeting compounds of formula (I) of the present disclosure include colorectal cancer (CRC), non-small-cell lung cancer (NSCLC), gastric cancer (GAC), neuroendocrine cancer (NENC) and pancreatic cancer (PAC), as well as breast, liver and thyroid cancer.

In some embodiments, the present disclosure includes a method of using the isolated antibodies and/or the tissue-targeting compounds of formula (I) of the present disclosure and/or a combination comprising a tissue-targeting compound of formula (I) and a further pharmaceutical agent of the present disclosure for the treatment of diseases, more preferably hyperproliferative diseases, more particularly wherein the hyperproliferative disease is cancer, and yet even more particularly wherein the cancer disease is colorectal cancer (CRC), non-small-cell lung cancer (NSCLC), gastric cancer (GAC), neuroendocrine cancer (NENC) and pancreatic cancer (PAC), as well as breast, liver and thyroid cancer. In some embodiments, the present disclosure includes a method of using the isolated antibodies and/or the tissue-targeting compounds of formula (I) of the present disclosure and/or a combination comprising a tissue-targeting compound of formula (I) and a further pharmaceutical agent of the present disclosure for the treatment of diseases, preferably hyperproliferative diseases, more preferably cancer, characterized by an overexpression of CEACAM5 in the tumor cells.

In some embodiments, the present disclosure includes a method of treating a hyperproliferative disease, more particularly cancer, more particularly colorectal cancer (CRC), non-small-cell lung cancer (NSCLC), gastric cancer (GAC), neuroendocrine cancer (NENC) and pancreatic cancer (PAC), as well as breast, liver and thyroid cancer, said method comprising administering an effective amount of at least one isolated antibody and/or one tissue-targeting compound of formula (I) of the present disclosure and/or a combination comprising a tissue-targeting compound of formula (I) and a further pharmaceutical agent of the present disclosure to a subject in need thereof. In some embodiments, the present disclosure includes a method of treating a hyperproliferative disease characterized by an overexpression of CEACAM5 in the tumor cells, more particularly wherein the hyperproliferative disease is cancer, more particularly colorectal cancer (CRC), non-small-cell lung cancer (NSCLC), gastric cancer (GAC), neuroendocrine cancer (NENC) and pancreatic cancer (PAC), as well as breast, liver and thyroid cancer, said method comprising administering an effective amount of at least one isolated antibody and/or one tissue-targeting compound of formula (I) of the present disclosure and/or a combination comprising a tissue-targeting compound of formula (I) and a further pharmaceutical agent of the present disclosure to a subject in need thereof.

In some embodiments, the present disclosure provides an isolated antibody and/or a tissue-targeting compound of formula (I) of the present disclosure as described *supra,* and/or a combination comprising a tissue-targeting compound of formula (I) and a further pharmaceutical agent of the present disclosure as described *supra,* for use in the treatment and/or prophylaxis of diseases, in particular hyperproliferative disorders, more particularly cancer, even more particularly wherein the cancer disease is colorectal cancer (CRC), non-small-cell lung cancer (NSCLC), gastric cancer (GAC), neuroendocrine cancer (NENC) and pancreatic cancer (PAC), as well as breast, liver and thyroid cancer. In some embodiments, the present disclosure includes an isolated antibody and/or a tissue-targeting compound of formula (I) of the present disclosure as described *supra,* and/or a combination comprising a tissue-targeting compound of formula (I) and a further pharmaceutical agent of the present disclosure as described *supra,* for use in the treatment and/or prophylaxis of hyperproliferative diseases characterized by an overexpression of CEACAM5 in the tumor cells.

In some embodiments, the present disclosure includes an isolated antibody and/or a tissue-targeting compound of formula (I) of the present disclosure and/or a combination comprising a tissue-targeting compound of formula (I) and a further pharmaceutical agent of the present disclosure for use in a method of inhibiting proliferation of a cell and/or the induction of apoptosis in a cell, comprising contacting the cell with isolated an antibody and/or a tissue-targeting compound of formula (I) of the present disclosure and/or with a combination comprising a tissue-targeting compound of formula (I) and a further pharmaceutical agent. In some embodiments, the present disclosure includes an isolated antibody and/or a tissue-targeting compound of formula (I) of the present disclosure and/or a combination comprising a tissue-targeting compound of formula (I) and a further pharmaceutical agent of the present disclosure for use in a method of inhibiting proliferation of tumor cells and/or the induction of apoptosis in tumor cells, comprising contacting the tumor cell with an isolated antibody and/or a tissue-targeting compound of formula (I) of the present disclosure and/or with a combination comprising a tissue-targeting compound of formula (I) and a further pharmaceutical agent, wherein CEACAM5 is overexpressed in the tumor cells.

In some embodiments, the present disclosure includes an isolated antibody and/or a tissue-targeting compound of formula (I) of the present disclosure and/or a combination comprising a tissue-targeting compound of formula (I) and a further pharmaceutical agent for use in a method of treating a hyperproliferative disease, more particularly wherein the hyperproliferative disease is cancer, and yet even more particularly wherein the cancer disease is colorectal cancer (CRC), non-small-cell lung cancer (NSCLC), gastric cancer (GAC), neuroendocrine cancer (NENC) and pancreatic cancer (PAC), as well as breast, liver and thyroid cancer. In some embodiments, the present disclosure includes an isolated antibody and/or a tissue-targeting compound of formula (I) of the present disclosure and/or a combination comprising a tissue-targeting compound of formula (I) and a further pharmaceutical agent for use in a method of treating a hyperproliferative disease characterized by an overexpression of CEACAM5 in the tumor cells.

In some embodiments, the present disclosure includes the use of an isolated antibody and/or a tissue-targeting compound of formula (I) of the present disclosure and/or a combination comprising a tissue-targeting compound of formula (I) and a further pharmaceutical agent for the manufacture of a medicament for the treatment and/or prophylaxis of a hyperproliferative disease. In some embodiments, the present disclosure includes the use of an isolated antibody and/or a tissue-targeting compound of formula (I) of the present disclosure and/or a combination comprising a tissue-targeting compound of formula (I) and a further pharmaceutical agent for the manufacture of a medicament for the treatment and/or prophylaxis of a hyperproliferative disease characterized by an overexpression of CEACAM5 in the tumor cells.

In some embodiments, the present disclosure includes the use of an isolated antibody and/or a tissue-targeting compound of formula (I) of the present disclosure and/or a combination comprising a tissue-targeting compound of formula (I) and a further pharmaceutical agent for the manufacture of a medicament for the treatment of a hyperproliferative disease, particularly cancer and more particularly colorectal cancer (CRC), non-small-cell lung cancer (NSCLC), gastric cancer (GAC), neuroendocrine cancer (NENC) and pancreatic cancer (PAC), as well as breast, liver and thyroid cancer.

### Administration

In the combinations comprising a tissue-targeting compound of formula (I) and a further pharmaceutical agent, the tissue-targeting compound of formula (I) and the further pharmaceutical agent may be administered sequentially in either order, or simultaneously.

The isolated antibodies and/or tissue-targeting compounds of formula (I) and/or the combinations of the present disclosure comprising a tissue-targeting compound of formula (I) and a further pharmaceutical agent can be administered in any form by any effective route, including, e.g., oral, parenteral, enteral, intravenous, intraperitoneal, topical, transdermal (e.g., using any standard patch), ophthalmic, nasally, local, non-oral, such as aerosol, inhalation, subcutaneous, intramuscular, buccal, sublingual, rectal, vaginal, intra-arterial, and intrathecal, etc. They can be administered alone, or in combination with any ingredient(s), active or inactive. Preferably, the isolated antibodies and/or tissue-targeting compounds of formula (I) and/or the combinations of the present disclosure are administered intravenously.

The isolated antibodies and/or the tissue-targeting compounds of formula (I) of the present disclosure and/or the combinations of the present disclosure comprising a tissue-targeting compound of formula (I) and a further pharmaceutical agent can be converted in a known manner into the usual pharmaceutical formulations, which may be liquid or solid formulations e.g., without limitation normal and enteric coated tablets, capsules, pills, powders, granules, elixirs, tinctures, solutions, suspensions, syrups, solid and liquid aerosols and emulsions.

The relative ratios of each compound in the combinations of the present disclosure comprising a tissue-targeting compound of formula (I) and a further pharmaceutical agent can also be selected based on their respective mechanisms of action and the disease biology. The relative ratios of each compound can vary widely.

The release of one or more agents of the combinations of the present disclosure comprising a tissue-targeting compound of formula (I) and a further pharmaceutical agent can also be controlled, where appropriate, to provide the desired therapeutic activity when in a single dosage form, combination pack, kit or when in separate independent dosage forms.

The present disclosure includes pharmaceutical compositions which are comprised of a pharmaceutically acceptable carrier and a pharmaceutically effective amount of the isolated antibodies and/or the tissue-targeting compounds of formula (I) of the present disclosure and/or the combinations of the present disclosure i.e., an isolated antibody and/or one tissue-targeting compound of formula (I) of the present disclosure as described above and a further pharmaceutical agent. A pharmaceutically acceptable carrier is any carrier which is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is that amount which produces a result or exerts an influence on the particular condition being treated.

For oral administration, the isolated antibodies and/or the tissue-targeting compounds of formula (I) of the present disclosure and/or the combinations of the present disclosure comprising a tissue-targeting compound of formula (I) and a further pharmaceutical agent can be formulated into solid or liquid preparations such as solid dispersion, capsules, pills, tablets, troches, lozenges, melts, powders, solutions, suspensions, or emulsions, and may be prepared according to methods known to the art for the manufacture of pharmaceutical compositions. The solid unit dosage forms can be a capsule which can be of the ordinary hard- or soft-shelled gelatin type containing, for example, surfactants, lubricants, and inert fillers such as lactose, sucrose, calcium phosphate, and corn starch.

If administered in the form of a tablet, t the isolated antibodies and/or the tissue-targeting compounds of formula (I) of the present disclosure and/or the combinations of the present disclosure comprising a tissue-targeting compound of formula (I) and a further pharmaceutical agent may be tableted with conventional tablet bases such as lactose, sucrose and cornstarch in combination with binders such as acacia, corn starch or gelatin, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, coloring agents, and flavoring agents such as peppermint, oil of wintergreen, or cherry flavoring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance, tablets, pills or capsules may be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavoring and coloring agents described above, may also be present.

The isolated antibodies and/or the tissue-targeting compounds of formula (I) of the present disclosure and/or the combinations of the present disclosure comprising a tissue-targeting compound of formula (I) and a further pharmaceutical agent may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soybean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more coloring agents; one or more flavoring agents; and one or more sweetening agents such as sucrose or saccharin.

Syrups and elixirs may be formulated with sweetening agents such as, for example, glycerol, propylene glycol, sorbitol or sucrose. Such formulations may also contain a demulcent, and preservative, such as methyl and propyl parabens and flavoring and coloring agents.

The isolated antibodies and/or the tissue-targeting compounds of formula (I) of the present disclosure and/or the combinations of the present disclosure comprising a tissue-targeting compound of formula (I) and a further pharmaceutical agent may also be administered parenterally, that is, subcutaneously, intravenously, intraocularly, intrasynovially, intramuscularly, or intraperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Preferably, the isolated antibodies and/or the tissue-targeting compounds of formula (I) of the present disclosure and/or the combinations of the present disclosure comprising a tissue-targeting compound of formula (I) and a further pharmaceutical agent are administered intravenously and/or orally, either together or separately. Preferably, in the combinations of the present disclosure comprising a tissue-targeting compound of formula (I) and a further pharmaceutical agent, the tissue-targeting compound of formula (I) is administered intravenously, and the further pharmaceutical agent is administered orally.

Illustrative of oils which can be used in the parenteral formulations of this disclosure are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; non-ionic detergents, for example, fatty amine oxides, fatty acid alkanolamides, and poly(oxyethylene-oxypropylene)s or ethylene oxide or propylene oxide copolymers; and amphoteric detergents, for example, alkyl-beta-aminopropionates, and 2-alkylimidazoline quarternary ammonium salts, as well as mixtures.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The isolated antibodies and/or the tissue-targeting compounds of formula (I) of the present disclosure and/or the combinations of the present disclosure comprising a tissue-targeting compound of formula (I) and a further pharmaceutical agent may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

The isolated antibodies and/or the tissue-targeting compounds of formula (I) of the present disclosure and/or the combinations of the present disclosure comprising a tissue-targeting compound of formula (I) and a further pharmaceutical agent may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such material is, for example, cocoa butter and polyethylene glycol.

Controlled release formulations for parenteral administration include liposomal, polymeric microsphere and polymeric gel formulations which are known in the art.

The isolated antibodies and/or the tissue-targeting compounds of formula (I) of the present disclosure and/or the combinations of the present disclosure comprising a tissue-targeting compound of formula (I) and a further pharmaceutical agent may also be in the form of a solid dispersion. The solid dispersion may be a solid solution, glass solution, glass suspension, amorphous precipitation in a crystalline carrier, eutectic or monotectic, compound or complex formation and combinations thereof.

The isolated antibodies and/or the tissue-targeting compounds of formula (I) of the present disclosure and/or the combinations of the present disclosure comprising a tissue-targeting compound of formula (I) and a further pharmaceutical agent can also contain other conventional pharmaceutically acceptable compounding ingredients, generally referred to as carriers or diluents, as necessary or desired. Conventional procedures for preparing such compositions in appropriate dosage forms can be utilized.

Commonly used pharmaceutical ingredients which can be used as appropriate to formulate the composition for its intended route of administration include:
- acidifying agents (examples include but are not limited to acetic acid, citric acid, fumaric acid, hydrochloric acid, nitric acid);
- alkalinizing agents (examples include but are not limited to ammonia solution, ammonium carbonate, diethanolamine, monoethanolamine, potassium hydroxide, sodium borate, sodium carbonate, sodium hydroxide, triethanolamine, trolamine);
- adsorbents (examples include but are not limited to powdered cellulose and activated charcoal);
- aerosol propellants (examples include but are not limited to carbon dioxide, CCl2F2, F2CIC-CCIF2 and CCIF3)
- air displacement agents (examples include but are not limited to nitrogen and argon);
- antifungal preservatives (examples include but are not limited to benzoic acid, butylparaben, ethylparaben, methylparaben, propylparaben, sodium benzoate);
- antimicrobial preservatives (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal);
- antioxidants (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);
- binding materials (examples include but are not limited to block polymers, natural and synthetic rubber, polyacrylates, polyurethanes, silicones, polysiloxanes and styrenebutadiene copolymers);
- buffering agents (examples include but are not limited to potassium metaphosphate, dipotassium phosphate, sodium acetate, sodium citrate anhydrous and sodium citrate dihydrate)
- carrying agents (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection)
- chelating agents (examples include but are not limited to edetate disodium and edetic acid)
- colorants (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);
- clarifying agents (examples include but are not limited to bentonite);
- emulsifying agents (examples include but are not limited to acacia, cetomacrogol, cetyl alcohol, glyceryl monostearate, lecithin, sorbitan monooleate, polyoxyethylene 50 monostearate);
- encapsulating agents (examples include but are not limited to gelatin and cellulose acetate phthalate)
- flavorants (examples include but are not limited to anise oil, cinnamon oil, cocoa, menthol, orange oil, peppermint oil and vanillin);
- humectants (examples include but are not limited to glycerol, propylene glycol and sorbitol);
- levigating agents (examples include but are not limited to mineral oil and glycerin);
- oils (examples include but are not limited to arachis oil, mineral oil, olive oil, peanut oil, sesame oil and vegetable oil);
- ointment bases (examples include but are not limited to lanolin, hydrophilic ointment, polyethylene glycol ointment, petrolatum, hydrophilic petrolatum, white ointment, yellow ointment, and rose water ointment);
- penetration enhancers (transdermal delivery) (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas)
- plasticizers (examples include but are not limited to diethyl phthalate and glycerol);
- scavenging compounds which reduce and/or prevent radiolysis;
- solvents (examples include but are not limited to ethanol, corn oil, cottonseed oil, glycerol, isopropanol, mineral oil, oleic acid, peanut oil, purified water, water for injection, sterile water for injection and sterile water for irrigation);
- stiffening agents (examples include but are not limited to cetyl alcohol, cetyl esters wax, microcrystalline wax, paraffin, stearyl alcohol, white wax and yellow wax);
- suppository bases (examples include but are not limited to cocoa butter and polyethylene glycols (mixtures));
- surfactants (examples include but are not limited to benzalkonium chloride, nonoxynol 10, oxtoxynol 9, polysorbate 80, sodium lauryl sulfate and sorbitan mono-palmitate);
- suspending agents (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);
- sweetening agents (examples include but are not limited to aspartame, dextrose, glycerol, mannitol, propylene glycol, saccharin sodium, sorbitol and sucrose);
- tablet anti-adherents (examples include but are not limited to magnesium stearate and talc);
- tablet binders (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);
- tablet and capsule diluents (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);
- tablet coating agents (examples include but are not limited to liquid glucose, hydroxyethyl cellulose, hydroxypropyl cellulose, hydroxypropyl methylcellulose, methylcellulose, ethylcellulose, cellulose acetate phthalate and shellac);
- tablet direct compression excipients (examples include but are not limited to dibasic calcium phosphate);
- tablet disintegrants (examples include but are not limited to alginic acid, carboxymethylcellulose calcium, microcrystalline cellulose, polacrillin potassium, cross-linked polyvinylpyrrolidone, sodium alginate, sodium starch glycollate and starch);
- tablet glidants (examples include but are not limited to colloidal silica, corn starch and talc);
- tablet lubricants (examples include but are not limited to calcium stearate, magnesium stearate, mineral oil, stearic acid and zinc stearate);
- tablet/capsule opaquants (examples include but are not limited to titanium dioxide);
- tablet polishing agents (examples include but are not limited to carnauba wax and white wax);
- thickening agents (examples include but are not limited to beeswax, cetyl alcohol and paraffin);
- tonicity agents (examples include but are not limited to dextrose and sodium chloride);
- viscosity increasing agents (examples include but are not limited to alginic acid, bentonite, carbomers, carboxymethylcellulose sodium, methylcellulose, polyvinyl pyrrolidone, sodium alginate and tragacanth); and
- wetting agents (examples include but are not limited to heptadecaethylene oxycetanol, lecithin, sorbitol monooleate, polyoxyethylene sorbitol monooleate, and polyoxyethylene stearate).

One skilled in the art, utilizing the preceding information, can utilize the present disclosure to its fullest extent.

### Therapeutically effective dose

Pharmaceutical compositions suitable for use according to the present disclosure include compositions wherein the active ingredients are contained in an effective amount to achieve the intended purpose, e.g., treatment of a particular disease state characterized by an ectopic expression of CEACAM5.

The determination of an effective dose is well within the capability of those skilled in the art.

Determining a therapeutically effective amount of the novel antibody of this disclosure or an antigen-binding fragment thereof or a variant thereof, largely will depend on particular patient characteristics, route of administration, and the nature of the disorder being treated. General guidance can be found, for example, in the publications of the International Conference on Harmonization and in REMINGTON'S PHARMACEUTICAL SCIENCES, chapters 27 and 28, pp. 484-528 (18th ed., Alfonso R. Gennaro, Ed., Easton, Pa.: Mack Pub. Co., 1990). More specifically, determining a therapeutically effective amount will depend on such factors as toxicity and efficacy of the medicament. Toxicity may be determined using methods well known in the art and found in the foregoing references.

For any compound, the therapeutically effective dose can be estimated initially either in cell culture assays, or in animal models, usually mice, rabbits, dogs, pigs or monkeys. The animal model is also used to achieve a desirable concentration range and route of administration. Such information can then be used to determine useful doses and routes for administration in humans.

A therapeutically effective dose refers to that amount of antibody or antigen-binding fragment thereof or that amount of antibody conjugate, that ameliorates the symptoms or condition. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, ED50/LD50. Pharmaceutical compositions that exhibit large therapeutic indices are preferred. The data obtained from cell culture assays and animal studies are used in formulating a range of dosage for human use. The dosage of such compounds lies preferably within a range of circulating concentrations that include the ED50 with little or no toxicity. The dosage varies within this range depending upon the dosage form employed, sensitivity of the patient, and the route of administration.

The exact dosage is chosen by the individual physician in view of the patient to be treated. Dosage and administration are adjusted to provide sufficient levels of the active moiety or to maintain the desired effect. Additional factors that may be taken into account include the severity of the disease state, age, weight and gender of the patient; diet, time and frequency of administration, drug combination(s), reaction sensitivities, and tolerance/response to therapy. Long-acting pharmaceutical compositions might be administered for example every 3 to 4 days, every week, once every two weeks, or once every three weeks, depending on half-life and clearance rate of the particular formulation.

Normal dosage amounts may vary from 0.1 to 100,000 micrograms, up to a total dose of about 10 g, depending upon the route of administration. Guidance as to particular dosages and methods of delivery is provided in the literature. See U.S. Pat. No. 4,657,760; 5,206,344; or 5,225,212.

### Kits

In a further aspect, the present disclosure relates to kits comprising the isolated antibodies or antigen-binding fragments according to the present disclosure or the targeted actinium conjugates according to the present disclosure and instructions for use. In particular embodiments, the kits comprise one or more containers filled with one or more of the ingredients of the aforementioned compositions of the disclosure. Associated with such container(s) can be a notice in the form prescribed by a governmental agency regulating the manufacture, use or sale of pharmaceuticals or biological products, reflecting approval by the agency of the manufacture, use or sale of the product for human administration.

### Diagnostic methods

According to a further aspect the antibodies or antigen-binding fragments according to the present disclosure or the antibody conjugates according to the present disclosure may be utilized, as such or in compositions, in research and diagnostics, or as analytical reference standards, and the like.

Anti-CEACAM5 antibodies or antigen-binding fragments thereof or the antibody conjugates according to the present disclosure can be used for detecting the presence of CEACAM5. Thus, in a further aspect, the present disclosure relates to the isolated antibodies or antigen-binding fragments according to the present disclosure or the antibody conjugates according to the present disclosure for use as a diagnostic agent.

Detection of expression of CEACAM5 generally involves contacting a biological sample (tumor, cells, tissue, or body fluid of an individual) with one or more antibodies or fragments according to the current disclosure (optionally conjugated to a detectable moiety) and detecting whether the sample is positive for CEACAM5, or whether the sample has altered (e.g., reduced or increased) expression as compared to a control sample.

In certain embodiments, provided conjugate comprises one or more detectable moieties, i.e., is "labeled" with one or more such moieties. In some such embodiments, a conjugate of the present disclosure is useful in diagnostic or imaging applications, e.g., diagnosing or imaging cancer. Any of a wide variety of detectable moieties may be used in labeled antibody conjugates described herein. Suitable detectable moieties comprise, without limitation: various ligands, radionuclides; fluorescent dyes (e.g. fluorescent resonance energy dyes (FRET) as i.e.Cy5, Cy7); chemiluminescent agents (such as, for example, acridinum esters, stabilized dioxetanes, and the like); bioluminescent agents; spectrally resolvable inorganic fluorescent semiconductors nanocrystals (i.e., quantum dots); microparticles; metal nanoparticles (e.g, gold, silver, copper, platinum, etc.); nanoclusters; paramagnetic metal ions; enzymes; colorimetric labels (such as, for example, dyes, colloidal gold, and the like); biotin; dioxigenin; haptens; and proteins for which antisera or monoclonal antibodies are available.

According to certain preferred embodiments of the present disclosure, the detectable moiety can be fluorescent dyes as e.g. Alexa Flour dyes (Invitrogen), or fluorescent resonance energy dyes (FRET) as e.g. the pH sensitive dye CypHer5E-NHS-Ester (Cytiva) which can be used for direct labeling of antibodies or antigen-binding fragments via lysine residues.

### PET Imaging

According to a further aspect the antibodies or antigen-binding fragments according to the present disclosure and/or conjugates thereof may be utilized as imaging agents, preferably as PET imaging agents.

Positron emission tomography (PET) is a medical imaging technique that detects gamma photons produced by positron emitters and reconstructs these signals to visualize the location of the positron emitter. When combined with computed tomography (CT), PET/CT allows for the precise localization of the positron emitter within the anatomical framework of the body. Multiple positron emitters are routinely used in the clinic, such as C-11, F-18, Ga-68 and Zr-89. Preferably, Zr-89 is used as positron emitter in combination with antibodies or antigen-binding fragments according to the present disclosure or the antibody conjugates according to the present disclosure, due to its long half-life (78.4 hours) and efficient labeling reaction in gentle conditions.

Suitable chelators for Zr-89 in PET imaging applications include, but are not limited to, for example DFO (Desferrioxamine), N-succinyl-desferrioxamine-tetrafluorophenol ester (N-suc-DFO-TFP ester) p-isothiocyanatobenzyl-desferrioxamine (DFO-Bz-NCS, also known as DFO-Ph-NCS), desferrioxamine-maleimide (DFO-maleimide), desferrioxamine-squaramide (DFO-sq), or DFO*. All these chelators can be conjugated with antibodies or antibody fragments to provide a means of targeting the imaging agent to the tumor to be imaged. According to a preferred embodiment of the present disclosure, the chelators are conjugated the to the rest of the molecule via a terminal amino group of a lysine residue of the monoclonal antibody or antigen-binding fragment thereof.

DFO* is an extended version of DFO and 89Zr-DFO* complexes are shown to exhibit increased in vitro stability and improved in vivo properties compared to DFO complexes (WO2021051168) and therefore is a preferred chelator for Zr-89 when conjugated with antibodies or antibody fragments according to the present disclosure for use in PET imaging.

DFO* refers to 5,11,16,22-Tetraazahexacosanediamide, N¹-[5-(acetylhydroxyamino)pentyl]-N²⁶,5,16-trihydroxy-N²⁶-[5-[[[(4-isothiocyanatophenyl)amino]thioxomethyl]amino]pentyl]-4,12,15,23-tetraoxo (CAS no.1810009-29-0): 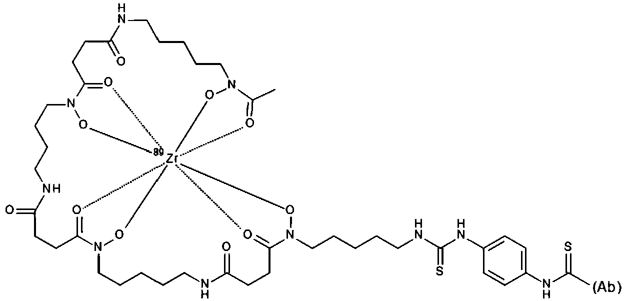 and can according to some embodiments of the present disclosure be conjugated to an antibody or an antigen binding fragment thereof (i.e., (Ab)), preferably to a monoclonal antibody or an antigen-binding fragment thereof binding to CEACAM5. Preferably, the antibody binding to CEACAM5 or an antigen binding fragment thereof is selected from the antibodies according to the first aspect of the present disclosure or an antigen-binding fragment thereof. Preferably, the antibody binding to CEACAM5 or an antigen binding fragment thereof is selected from TPP-29312, TPP-29537, TPP-29538, TPP-29636, TPP-29638, and TPP-29642 or an antigen-binding fragment thereof. More preferably, the antibody binding to CEACAM5 or an antigen binding fragment thereof is selected from TPP- 29537, TPP-29538, TPP-29636 and TPP-29638 or an antigen-binding fragment thereof.

According to certain highly preferred embodiments of the present disclosure, DFO* is coupled to the antibody binding to CEACAM5 or an antigen binding fragment thereof according to the present disclosure via a lysine residue of the monoclonal antibody, thus forming a thiourea moiety that links the chelating moiety with the antibody binding to CEACAM5 or an antigen binding fragment thereof.

One skilled in the art, utilizing the preceding information, can utilize the present disclosure to its fullest extent.

### DESCRIPTION OF THE FIGURES

Figure 1. SPR sensorgram illustrating the binding interactions of antibody DPP62130 with human CEACAM5, conducted with antibodies immobilized on an anti-human Fc chip at a concentration of 50 µg/mL
Figure 2. SPR sensorgram illustrating the binding interactions of antibody DPP62124 with human CEACAM5, conducted with antibodies immobilized on an anti-human Fc chip at a concentration of 50 µg/mL
Figure 3. SPR sensorgram illustrating the binding interactions of antibody DPP62291 with human CEACAM5, conducted with antibodies immobilized on an anti-human Fc chip at a concentration of 50 µg/mL
Figure 4. SPR sensorgram illustrating the binding interactions of antibody DPP62221 with human CEACAM5, conducted with antibodies immobilized on an anti-human Fc chip at a concentration of 50 µg/mL
Figure 5. SPR sensorgram illustrating the binding interactions of antibody DPP60333 with human CEACAM5, conducted with antibodies immobilized on an anti-human Fc chip at a concentration of 50 µg/mL
Figure 6. SPR sensorgram illustrating the binding interactions of antibody DPP64262 with human CEACAM5, conducted with antibodies immobilized on a PAGHC200M chip at a concentration of 5 µg/mL
Figure 7. SPR sensorgram illustrating the binding interactions of antibody DPP64209 with human CEACAM5, conducted with antibodies immobilized on a PAGHC200M chip at a concentration of 5 µg/mL
Figure 8. SPR sensorgram illustrating the binding interactions of antibody DPP64227 with human CEACAM5, conducted with antibodies immobilized on a PAGHC200M chip at a concentration of 5 µg/mL
Figure 9. SPR sensorgram illustrating the binding interactions of antibody DPP64204 with human CEACAM5, conducted with antibodies immobilized on a PAGHC200M chip at a concentration of 5 µg/mL
Figure 10. SPR sensorgram illustrating the binding interactions of antibody DPP64106 with human CEACAM5, conducted with antibodies immobilized on a PAGHC200M chip at a concentration of 5 µg/mL
Figure 11. SPR sensorgram illustrating the binding interactions of antibody DPP64107 with human CEACAM5, conducted with antibodies immobilized on a PAGHC200M chip at a concentration of 5 µg/mL

### EXPERIMENTAL SECTION

Chemical names were generated using the BIOVIA Draw 2019 from Dassault Systèmes. In some cases, generally accepted names of commercially available reagents were used in place of BIOVIA Draw generated names.

The following Table 2 lists the abbreviations used in this paragraph and in the Examples section as far as they are not explained within the text body. Other abbreviations have their meanings customary *per* se to the skilled person.

### Table 2. Abbreviations

The following table lists the abbreviations used herein.
- ²²⁵Ac: actinium-225
- Ac-225: actinium-225
- ACC: antibody-chelator conjugate
- ACN: acetonitrile
- BCA: Bicinchoninic acid
- BRFF-HPC1: Serum free medium
- CAR: chelator-to-antibody ratio
- CEACAM5: carcinoembryonic antigen-related cell adhesion molecule 5
- DCTA: 1,2-diaminocyclohexanetetraacetic acid
- DMA: N,N-dimethylacetamide
- DMSO: dimethyl sulfoxide
- DOTA: 1,4,7,10-tetraazacyclododecane-1,4,7,10-tetraacetic acid
- ESI: electrospray ionization
- EtOH: ethanol
- FBS: fetal bovine serum
- FPLC: fast protein liquid chromatography
- HCl: hydrochloric acid
- HPGe: high purity germanium
- HPLC: high performance liquid chromatography
- iTLC: instant thin layer chromatography
- IRF: immunoreactive fraction
- mAb: monoclonal antibody
- MEM-NEAA: minimum essential medium-non-essential amino acids
- min: minutes
- MS: mass spectrometry
- NaCl: sodium chloride
- nm: nanometer
- nmol: nanomole
- PBS: phosphate buffered saline
- RAC: radioactive concentration
- RCP: radiochemical purity
- RPMI: Roswell Park Memorial Institute 1640 medium
- SEC: size exclusion chromatography
- TAC: targeted actinium conjugate (Ac-225 labelled ACC)
- TFA: trifluoroacetic acid
- TOF: time of flight
- UPLC: ultra performance liquid chromatography
- UV: ultraviolet

All reagents, for which the synthesis is not described in the experimental part, are either commercially available, or known compounds or may be formed from known compounds by known methods by a person skilled in the art.

The various aspects of the disclosure described in this application are illustrated by the following examples which are not meant to limit the disclosure in any way.

The example testing experiments described herein serve to illustrate the present disclosure and the disclosure is not limited to the examples given.

### Antibodies having binding affinity for CEACAM5

### Example 1: CEACAM5 Sequences and Tool Generation

The antigens used in this disclosure, specifically the domains of the CEACAM5 antigen, were procured from reputable commercial suppliers. These materials were not synthesized internally but were selected for their quality and relevance to the study. Table 3 below provides detailed information regarding the sources and specific catalog numbers for each antigen domain:

**Table 3.**

| **DPP-No.** | **Protein** | **Boundaries [aa]** | **Supplier** | **Catalog No.** |
|---|---|---|---|---|
| **DPP21713** | TPP-13110: Labetuzumab-hIgG1 | | | |
| | Human antigen | | | |

### Example 2: Selection and identification of anti-CEACAM5/CEACAM5-binding targeting antibodies

In this example, we describe the identification and development of novel antibodies targeting the CEACAM5 antigen. The foundation for these new antibody sequences is derived from an antibody described in patent document WO2015069430, providing a basis for further enhancement and specificity improvements.

Initially, the known antibody sequence targeting CEACAM5 was obtained from WO2015069430, which served as a template for subsequent modifications. Using advanced molecular engineering techniques, modifications were introduced to enhance the binding affinity and specificity of the antibody to the CEACAM5 antigen.

The modifications involved site-directed mutagenesis and affinity maturation processes, which were guided by computational modeling and empirical data from binding assays. The resulting antibodies were characterized using standard immunological techniques, including ELISA, flow cytometry, and surface plasmon resonance, to confirm their enhanced binding properties compared to the original antibody sequence.

The newly generated antibodies demonstrated improved targeting capabilities, as evidenced by increased binding affinity and specificity for the CEACAM5 antigen, making them suitable candidates for therapeutic applications. These findings underscore the potential of leveraging existing antibody frameworks to develop innovative and highly specific targeting molecules.

In summary, this example illustrates the successful identification and optimization of anti-CEACAM5 antibodies, building upon established scientific knowledge from WO2015069430 to achieve enhanced targeting efficacy.

### Example 3: Sequence Optimization, Germlining & Affinity maturation of DPP21713

IgG antibody DPP21713 underwent lead optimization procedures aimed at (i) enhancing its affinity, (ii) increasing functional efficiency, (iii) reducing the risk of sequence-based immunogenicity, and (iv) improving compatibility with downstream development processes. Affinity maturation was accomplished through an initial round of single mutation gathering, followed by recombination of the most affinity- and potency-enhancing amino acid exchanges in a germlined and sequence-optimized antibody backbone.

Protein variants were custom produced for testing. The closest germline families for the light and heavy chains of DPP21713 were selected and scrutinized for potential CMC-relevant residues. Deviations from the closest human germlines in CDR regions and FW regions, as well as potential CMC-relevant residues in CDR regions, were adjusted by site-directed mutagenesis and tested in functional and biophysical assays, including assessments of unspecific binding and temperature stability via DSF.

The resulting single reversions and subsequent combinatorial IgG and scFv-Fc variants were expressed by transient transfection of mammalian cells. Purified proteins were normalized for antibody concentrations to be screened in binding assays, such as SPR. This process led to the generation of germlined and sequence-optimized molecules, with specific reversions in the light and heavy chains. For the final recombination, single substitution variants demonstrating improved affinity were selected and combined.

More than 700 unique combinatorial amino acid exchange variants were generated, expressed by transient transfection of mammalian cells, purified and resulting proteins were normalized for antibody concentrations to be screened in SPR, FACS, DSF, ELISA, SDS-PAGE and SEC. Based on the results of that assay, mutants were categorized as either 'improved' or 'non-improved'.

Table 4 lists preferred antibodies candidates according to the present disclosure that were selected in the screening step.

**Table 4**

| DPP ID | Name |
|---|---|
| DPP62130 | tpp38518_hD28A_IS32Q-hIgG1 |
| DPP62124 | tpp38518_IS32Q-hIgG1 |
| DPP62291 | dpp45179_wt-hIgG1 |
| DPP62221 | dpp45205_wt-hIgG1 |
| DPP60333 | tpp38527-hIgG1 |
| DPP64262 | D28T_T30S_D54G_T219V-scFv- |
| DPP64209 | S35H_K158R_T165S_W184G-scFv- |
| DPP64227 | S35H_T165S_L212V-scFv- |
| DPP64204 | T30S_T31S_L64V_P88A_G92A_V167L_W184D_T187N-scFv- |
| DPP64106 | A24T_G49S_K65R_P114L_V149L_K158R-scFv- |
| DPP64107 | A40T_F103S_P114L_V149L_K176Q_S210N-scFv- |
| DPP62130 | tpp38518_hD28A_IS32Q-hIgG1 |

### Example 4: Binding to recombinant CEACAM5 by cell surface plasmon resonance

To assess the binding kinetics and affinity of antibodies against human CEACAM5, binding assays were conducted using the Carterra^{®} LSA^{™} instrument under two different experimental conditions.

In one setup, antibodies were immobilized on an anti-human Fc chip (PAHC200M) for 10 minutes at a concentration of 50 µg/mL in HBSTE Buffer, pH 7.4. A 3-fold dilution series of human CEACAM5-His was prepared, ranging from 0.15 to 100 nM. Spike dilutions were injected onto the chip surface from the lowest to the highest concentration, with several buffer injections before the lowest non-zero concentration to ensure signal stabilization. The association phase lasted 420 seconds, and the dissociation phase lasted 600 seconds, with a baseline data collection time of 60 seconds. After each analyte titration, the chip surface was regenerated with three cycles of 3 M MgCl2 at pH 2.1, each involving a 20-second pulse. Kinetic data were analyzed using Carterra KITTM software, fitting the sensorgrams to a 1:1 Langmuir binding model.

In a second setup, antibodies were immobilized on a PAGHC200M chip for 10 minutes at a concentration of 5 µg/mL in the same running buffer. The antigen concentration ranged from 0.047 to 300 nM, with a similar 3-fold dilution series prepared. The baseline data collection lasted 60 seconds, the association phase lasted 5 minutes, and the dissociation phase lasted 25 minutes. Regeneration of the chip surface was performed with three pulses of 10 mM Gly-HCL at pH 1.5, each lasting 20 seconds. Kinetic data were again analyzed using Carterra KITTM software with a 1:1 Langmuir binding model.

Both experimental conditions provided valuable insights into the binding kinetics and affinity of the antibodies, with results summarized in

Table 5. These results show that the isolated antibodies or antigen-binding fragments thereof of the present disclosure exhibit an affinity towards CEACAM5 that is in some cases two orders of magnitude higher than that reported for CEACAM5-binding antibodies known in the prior art (see, for example, Table 21 of EP4079761 - the KD (M) values range from 1.44E-07 to 5.93E-09).

**Table 5. Affinity of anti-CEACAM5/CEACAM5-binding IgG antibodies derived from phage display hits determined by SPR using TPP-30471. n.b. = no binding, n.d. = not determinable**

| Ligand | **ka (1/Ms)** | **kd (1/s)** | **KD (M)** |
|---|---|---|---|
| DPP62130 | 3,73E+04 | 1,12E-04 | 2,99E-09 |
| DPP62124 | 3,75E+04 | 1,10E-04 | 2,92E-09 |
| DPP62291 | 3,57E+04 | 1,40E-03 | 3,92E-08 |
| DPP62221 | 3,63E+04 | 6,59E-04 | 1,82E-08 |
| DPP60333 | 2,93E+04 | 9,74E-04 | 3,32E-08 |
| DPP64262 | 1,10E+05 | 1,00E-05 | 9,07E-11 |
| DPP64209 | 1,16E+05 | 1,00E-05 | 8,62E-11 |
| DPP64227 | 5,06E+05 | 1,00E-05 | 1,98E-11 |
| DPP64204 | 2,87E+05 | 3,19E-05 | 1,11E-10 |
| DPP64106 | 8,98E+04 | 1,00E-05 | 1,11E-10 |
| DPP64107 | 5,23E+05 | 1,00E-05 | 1,91E-11 |

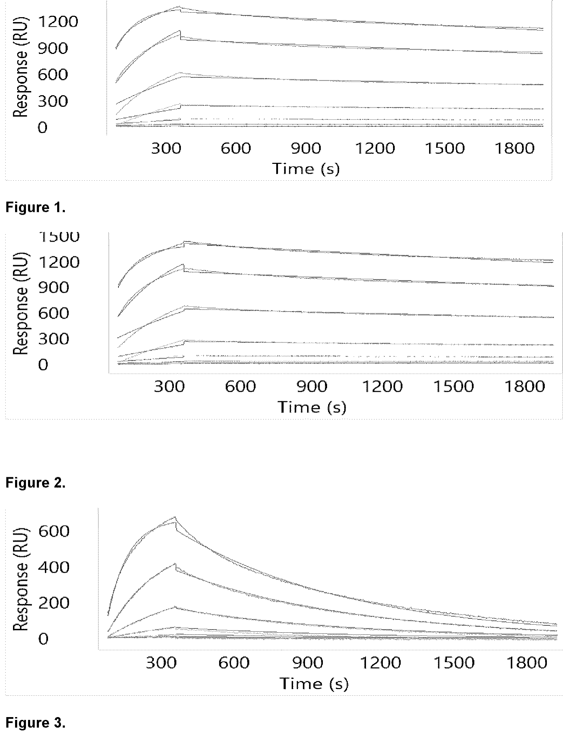

Figure 1 shows an SPR sensorgram illustrating the binding interactions of antibody DPP62130 with human CEACAM5, conducted with antibodies immobilized on an anti-human Fc chip at a concentration of 50 µg/mL. The assay utilized HBSTE Buffer at pH 7.4, with antigen concentrations ranging from 0.15 to 100 nM in a 3-fold dilution series. The association phase lasted 420 seconds, followed by a dissociation phase of 600 seconds. Regeneration was performed using 3 M MgCl2 at pH 2.1, across three cycles of 20-second pulses. These conditions differ from the alternative setup, which employed a lower antibody concentration, extended association and dissociation times, and used 10 mM Gly-HCL for regeneration.

Figure 2 shows an SPR sensorgram illustrating the binding interactions of antibody DPP62124 with human CEACAM5, conducted with antibodies immobilized on an anti-human Fc chip at a concentration of 50 µg/mL. The assay utilized HBSTE Buffer at pH 7.4, with antigen concentrations ranging from 0.15 to 100 nM in a 3-fold dilution series. The association phase lasted 420 seconds, followed by a dissociation phase of 600 seconds. Regeneration was performed using 3 M MgCl2 at pH 2.1, across three cycles of 20-second pulses. These conditions differ from the alternative setup, which employed a lower antibody concentration, extended association and dissociation times, and used 10 mM Gly-HCL for regeneration.

Figure 3 shows an SPR sensorgram illustrating the binding interactions of antibody DPP62291 with human CEACAM5, conducted with antibodies immobilized on an anti-human Fc chip at a concentration of 50 µg/mL. The assay utilized HBSTE Buffer at pH 7.4, with antigen concentrations ranging from 0.15 to 100 nM in a 3-fold dilution series. The association phase lasted 420 seconds, followed by a dissociation phase of 600 seconds. Regeneration was performed using 3 M MgCl2 at pH 2.1, across three cycles of 20-second pulses. These conditions differ from the alternative setup, which employed a lower antibody concentration, extended association and dissociation times, and used 10 mM Gly-HCL for regeneration.

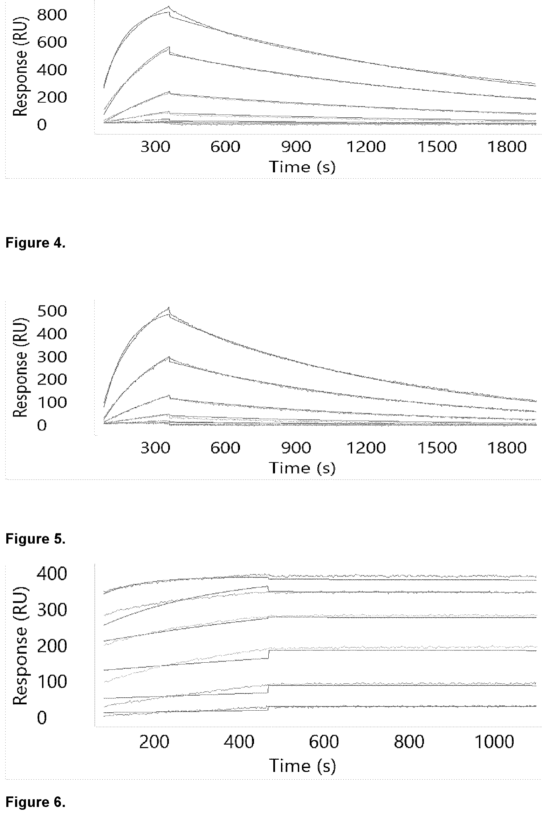

Figure 4 shows an SPR sensorgram illustrating the binding interactions of antibody DPP62221 with human CEACAM5, conducted with antibodies immobilized on an anti-human Fc chip at a concentration of 50 µg/mL. The assay utilized HBSTE Buffer at pH 7.4, with antigen concentrations ranging from 0.15 to 100 nM in a 3-fold dilution series. The association phase lasted 420 seconds, followed by a dissociation phase of 600 seconds. Regeneration was performed using 3 M MgCl2 at pH 2.1, across three cycles of 20-second pulses. These conditions differ from the alternative setup, which employed a lower antibody concentration, extended association and dissociation times, and used 10 mM Gly-HCL for regeneration.

Figure 5 shows an SPR sensorgram illustrating the binding interactions of antibody DPP60333 with human CEACAM5, conducted with antibodies immobilized on an anti-human Fc chip at a concentration of 50 µg/mL. The assay utilized HBSTE Buffer at pH 7.4, with antigen concentrations ranging from 0.15 to 100 nM in a 3-fold dilution series. The association phase lasted 420 seconds, followed by a dissociation phase of 600 seconds. Regeneration was performed using 3 M MgCl2 at pH 2.1, across three cycles of 20-second pulses. These conditions differ from the alternative setup, which employed a lower antibody concentration, extended association and dissociation times, and used 10 mM Gly-HCL for regeneration.

Figure 6 shows an SPR sensorgram illustrating the binding interactions of antibody DPP64262 with human CEACAM5, conducted with antibodies immobilized on a PAGHC200M chip at a concentration of 5 µg/mL. The assay utilized HBSTE Buffer at pH 7.4, with antigen concentrations ranging from 0.047 to 300 nM in a 3-fold dilution series. The association phase lasted 5 minutes, followed by a dissociation phase of 25 minutes. Regeneration was performed using 10 mM Gly-HCL at pH 1.5, across three cycles of 20-second pulses. These conditions differ from the alternative setup, which employed a higher antibody concentration, shorter association and dissociation times, and used 3 M MgCl2 for regeneration.

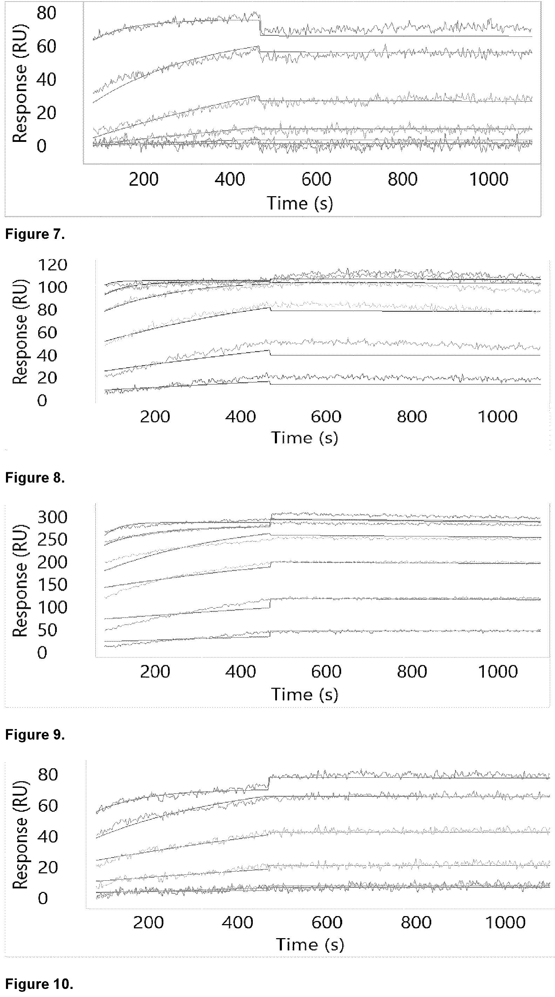

Figure 7 shows an SPR sensorgram illustrating the binding interactions of antibody DPP64209 with human CEACAM5, conducted with antibodies immobilized on a PAGHC200M chip at a concentration of 5 µg/mL. The assay utilized HBSTE Buffer at pH 7.4, with antigen concentrations ranging from 0.047 to 300 nM in a 3-fold dilution series. The association phase lasted 5 minutes, followed by a dissociation phase of 25 minutes. Regeneration was performed using 10 mM Gly-HCL at pH 1.5, across three cycles of 20-second pulses. These conditions differ from the alternative setup, which employed a higher antibody concentration, shorter association and dissociation times, and used 3 M MgCl2 for regeneration.

Figure 8 shows an SPR sensorgram illustrating the binding interactions of antibody DPP64227 with human CEACAM5, conducted with antibodies immobilized on a PAGHC200M chip at a concentration of 5 µg/mL. The assay utilized HBSTE Buffer at pH 7.4, with antigen concentrations ranging from 0.047 to 300 nM in a 3-fold dilution series. The association phase lasted 5 minutes, followed by a dissociation phase of 25 minutes. Regeneration was performed using 10 mM Gly-HCL at pH 1.5, across three cycles of 20-second pulses. These conditions differ from the alternative setup, which employed a higher antibody concentration, shorter association and dissociation times, and used 3 M MgCl2 for regeneration.

Figure 9 shows an SPR sensorgram illustrating the binding interactions of antibody DPP64204 with human CEACAM5, conducted with antibodies immobilized on a PAGHC200M chip at a concentration of 5 µg/mL. The assay utilized HBSTE Buffer at pH 7.4, with antigen concentrations ranging from 0.047 to 300 nM in a 3-fold dilution series. The association phase lasted 5 minutes, followed by a dissociation phase of 25 minutes. Regeneration was performed using 10 mM Gly-HCL at pH 1.5, across three cycles of 20-second pulses. These conditions differ from the alternative setup, which employed a higher antibody concentration, shorter association and dissociation times, and used 3 M MgCl2 for regeneration.

Figure 10 shows an SPR sensorgram illustrating the binding interactions of antibody DPP64106 with human CEACAM5, conducted with antibodies immobilized on a PAGHC200M chip at a concentration of 5 µg/mL. The assay utilized HBSTE Buffer at pH 7.4, with antigen concentrations ranging from 0.047 to 300 nM in a 3-fold dilution series. The association phase lasted 5 minutes, followed by a dissociation phase of 25 minutes. Regeneration was performed using 10 mM Gly-HCL at pH 1.5, across three cycles of 20-second pulses. These conditions differ from the alternative setup, which employed a higher antibody concentration, shorter association and dissociation times, and used 3 M MgCl2 for regeneration.

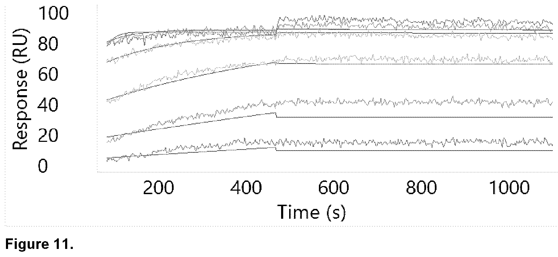

Figure 11 shows an SPR sensorgram illustrating the binding interactions of antibody DPP64107 with human CEACAM5, conducted with antibodies immobilized on a PAGHC200M chip at a concentration of 5 µg/mL. The assay utilized HBSTE Buffer at pH 7.4, with antigen concentrations ranging from 0.047 to 300 nM in a 3-fold dilution series. The association phase lasted 5 minutes, followed by a dissociation phase of 25 minutes. Regeneration was performed using 10 mM Gly-HCL at pH 1.5, across three cycles of 20-second pulses. These conditions differ from the alternative setup, which employed a higher antibody concentration, shorter association and dissociation times, and used 3 M MgCl2 for regeneration.

### Tissue-targeting compounds comprising a monoclonal antibody or an antigen-binding fragment thereof having binding affinity for CEACAM5

### Example 5: Preparation of anti-CEACAM5/CEACAM5-binding conjugates (macropa anti-CEACAM5/CEACAM5-binding conjugates, tissue-targeting-chelators of formula (III))

6-[[16-[(6-carboxy-2-pyridyl)methyl]-1,4,10,13-tetraoxa-7,16-diazacyclooctadec-7-yl]methyl]-4-[2-(4-isothiocyanatophenyl)ethoxy]pyridine-2-carboxylic acid dissolved in DMA (10 mg/mL) was added to anti-CEACAM5 antibodies (molar ratio of antibody:chelator (1:1.3)) in PBS. Solution was adjusted to pH 9 by adding 1M carbonate buffer, pH 9.5, and solution shaken overnight. Product was purified by FPLC (column: HiLoad (16/600 or 10/300) Superdex 200 pg column; running buffer: 6/10/10 mM citrate/histidine/glycine, 50 mM methionine, 0.1 mg/mL PS80 and 100 mM NaCl., pH 6; flow: 1 mL/min; detection: UV 214/254/280 nm) CAR was determined by MS to be in range of 0.5-1. Monomeric purity was determined by SEC-UV.

### ACC characterization by size exclusion chromatography and mass spectrometry

CAR of the ACCs was determined by SEC-MS (Water Acquity HPLC connected to Waters XEVO TOF; running buffer: 50/50/0.1 (v/v/v) ACN/Water/TFA; flow rate: 0.06 mL/min, column for on-line desalting: Waters Acquity BEH SEC, 1.7 µm, 2.1 x 150 mm; ESI+ mode) by using MS peak heights in percentage of major peak height for the components mAb, mAb + 1 chelator, mAb + 2 chelators, mAb + 3 chelators etc. and using the formula CAR = Sum(n*An)/Sum An, where n equals the number of chelators and An equals the intensity of the antibody conjugate with n chelators. Purity and concentration of the ACC were determined by SEC-UV (Agilent 1260 Infinity HPLC system, running buffer: 10% DMSO/PBS; flow rate: 0.3 mL/min, column: Waters Acquity BEH SEC, 1.7 µm, 4.6 x 300 mm, detection: UV at 280 nm). The method separates higher aggregate, dimer and fragments from the ACC monomer.

### Radiolabeling

Macropa-anti-CEACAM5 conjugates (tissue-targeting chelators of formula (III)) were radiolabeled with Ac-225 at a specific activity of 2 MBq/mg and diluted with 6/10/10 mM citrate/histidine/glycine, 50 mM methionine, 0.1 mg/mL PS80 pH 6 to a radioactive concentration of 1.5 kBq/µL. The mixture was incubated for one hour at room temperature. The RCP of the TACs was measured by instant thin layer chromatography using citrate buffer for elution, three replicates for each sample. Unlabeled Ac-225 elutes in the solvent front while the radiolabeled compounds do not move from the application section. The measurement of radioactivity was done with a Digital Gamma-Ray Spectrometer (DSPEC-50, Ortec). The activities were measured at least 6 hours after radiolabeling to allow daughter nuclides to be in equilibrium with Ac-225. The RCP of the samples (defined as [(radioactivity of application section)/(total radioactivity on strip)] x 100) was 100% for Ac-225-macropa-anti-CEACAM5 conjugate (tissue-targeting compounds of formula (I)).

### Size Exclusion Chromatography

Purity and stability of the TACs was determined by SEC-UV and followed over the course of 120 hours. The method separates higher aggregate, dimer and fragments from the ACC monomer. The separation was done using a Acquity UPLC Protein BEH SEC, 1.7 µm, 200Å, 4.6 x 300 mm column operated at 30 °C. The mobile phase was 170 mM Ammonium acetate and 300 mM NaCl containing 10% 2-propanol and 0.1 mM DCTA. The flowrate was 0.3 mL/min, and the ACCs were detected at UV 280 nm. The analysis was performed on an Agilent 1200 series HPLC with 15/20 µL injections.

### Immunoreactive Fraction (IRF)

Both shortly after completion of the radiolabeling and 120 hours after radiolabeling, the binding of each sample to CEACAM5-coated M-270-carboxy Dynabeads was determined. In short, 200 µg beads were placed in 2 mL Eppendorf tubes in 90 µL buffer (PBS/3% BSA). The radiolabeled compounds were diluted to 5 Bq/µL in buffer and added to each bead-containing tube. The binding lasted for 60 min while shaking at 750 rpm at room temperature. After completion of the binding step, a magnet (DynaMag-2) was used to separate the beads from half of the supernatant. The radio activities in the supernatant and in the beads of each sample were measured using a Digital Gamma-Ray Spectrometer. The activities were measured after at least 6 hours, to allow daughter nuclides to be in equilibrium with Ac-225.

### Sequences

**Table 6**

| **DPP ID** | **Sequence Name** | **TPP ID** | **Sequence Region** | **SEQ** | **SEQ ID** |
|---|---|---|---|---|---|
| DPP60333 | tpp38527-hIgG1 | tpp385 27_wt-hIgG1 | HCDR1 | GFTFSSYW | 1 |
| DPP60333 | tpp38527-hIgG1 | tpp385 27_wt-hIgG1 | HCDR2 | IHPDSSTI | 2 |
| DPP60333 | tpp38527-hIgG1 | tpp385 27_wt-hIgG1 | HCDR3 | ASLYFGFPWFAY | 3 |
| DPP60333 | tpp38527-hIgG1 | tpp385 27_wt-hIgG1 | VH | EVQLVESGGGLVQPGGSLRLSCAASGFTFSS YWMSWVRQAPGKGLEWVGEIHPDSSTINYA DSVKGRFTISRDNAKNSLYLQMNSLRAEDTA VYYCASLYFGFPWFAYWGQGTLVTVSS | 4 |
| DPP60333 | tpp38527-hIgG1 | tpp385 27_wt-hIgG1 | HC | EVQLVESGGGLVQPGGSLRLSCAASGFTFSS YWMSWVRQAPGKGLEWVGEIHPDSSTINYA DSVKGRFTISRDNAKNSLYLQMNSLRAEDTA VYYCASLYFGFPWFAYWGQGTLVTVSSASTK GPSVFPLAPSSKSTSGGTAALGCLVKDYFPEP VTVSWNSGALTSGVHTFPAVLQSSGLYSLSS VVTVPSSSLGTQTYICNVNHKPSNTKVDKKVE | 5 |
| | | | | PKSCDKTHTCPPCPAPELLGGPSVFLFPPKP KDTLMISRTPEVTCVVVDVSHEDPEVKFNWY VDGVEVHNAKTKPREEQYNSTYRVVSVLTVL HQDWLNGKEYKCKVSNKALPAPIEKTISKAK GQPREPQVYTLPPSRDELTKNQVSLTCLVKG FYPSDIAVEWESNGQPENNYKTTPPVLDSDG SFFLYSKLTVDKSRWQQGNVFSCSVMHEAL HNHYTQKSLSLSPGK | |
| DPP60333 | tpp38527-hIgG1 | tpp385 27_wt-hIgG1 | LCDR1 | GTSVA | 6 |
| DPP60333 | tpp38527-hIgG1 | tpp385 27_wt-hIgG1 | LCDR2 | YWASTLHTGVPS | 7 |
| DPP60333 | tpp38527-hIgG1 | tpp385 27_wt-hIgG1 | LCDR3 | QQYSLYRS | 8 |
| DPP60333 | tpp38527-hIgG1 | tpp385 27_wt-hIgG1 | VL | DIQMTQSPSSLSASVGDRVTITCKASQDVGT SVAWYQQKPGKAPKLLIYWASTLHTGVPSRF SGSGSGTDFTFTISSLQPEDIATYYCQQYSLY RSFGQGTKVEIK | 9 |
| DPP60333 | tpp38527-hIgG1 | tpp385 27_wt-hIgG1 | LC | DIQMTQSPSSLSASVGDRVTITCKASQDVGT SVAWYQQKPGKAPKLLIYWASTLHTGVPSRF SGSGSGTDFTFTISSLQPEDIATYYCQQYSLY RSFGQGTKVEIKRTVAAPSVFIFPPSDEQLKS GTASVVCLLNNFYPREAKVQWKVDNALQSG NSQESVTEQDSKDSTYSLSSTLTLSKADYEKH KVYACEVTHQGLSSPVTKSFNRGEC | 10 |
| DPP62124 | tpp38518_ lS32Q-hIgG1 | | HCDR1 | GFDFTTYW | 11 |
| DPP62124 | tpp38518_ lS32Q-hIgG1 | | HCDR2 | IHPDSSTI | 12 |
| DPP62124 | tpp38518_ lS32Q-hIgG1 | | HCDR3 | ASLYFGFPWFAY | 13 |
| DPP62124 | tpp38518_ lS32Q-hIgG1 | | VH | EVQLVESGGGLVQPGGSLRLSCAASGFDFTT YWMSWVRQAPGKGLEWIGEIHPDSSTINYA PSLKDRFTISRDNAKNSLYLQMNSLRAEDTG VYYCASLYFGFPWFAYWGQGTPVTVSS | 14 |
| DPP62124 | tpp38518_ lS32Q-hIgG1 | | HC | EVQLVESGGGLVQPGGSLRLSCAASGFDFTT YWMSWVRQAPGKGLEWIGEIHPDSSTINYA PSLKDRFTISRDNAKNSLYLQMNSLRAEDTG VYYCASLYFGFPWFAYWGQGTPVTVSSASTK GPSVFPLAPSSKSTSGGTAALGCLVKDYFPEP VTVSWNSGALTSGVHTFPAVLQSSGLYSLSS VVTVPSSSLGTQTYICNVNHKPSNTKVDKKVE PKSCDKTHTCPPCPAPELLGGPSVFLFPPKP KDTLMISRTPEVTCVVVDVSHEDPEVKFNWY | 15 |
| | | | | VDGVEVHNAKTKPREEQYNSTYRVVSVLTVL HQDWLNGKEYKCKVSNKALPAPIEKTISKAK GQPREPQVYTLPPSRDELTKNQVSLTCLVKG FYPSDIAVEWESNGQPENNYKTTPPVLDSDG SFFLYSKLTVDKSRWQQGNVFSCSVMHEAL HNHYTQKSLSLSPGK | |
| DPP62124 | tpp38518_ lS32Q-hIgG1 | | LCDR1 | GTQLA | 16 |
| DPP62124 | tpp38518_ lS32Q-hIgG1 | | LCDR2 | YWASTLHTGVPS | 17 |
| DPP62124 | tpp38518_ lS32Q-hIgG1 | | LCDR3 | QQYSLYRS | 18 |
| DPP62124 | tpp38518_ lS32Q-hIgG1 | | VL | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIK | 19 |
| DPP62124 | tpp38518_ lS32Q-hIgG1 | | LC | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIKRTVAAPSVFIFPPSDEQLKS GTASVVCLLNNFYPREAKVQWKVDNALQSG NSQESVTEQDSKDSTYSLSSTLTLSKADYEKH KVYACEVTHQGLSSPVTKSFNRGEC | 20 |
| DPP62130 | tpp38518_ hD28A_lS3 2Q-hIgG1 | | HCDR1 | GFAFTTYW | 21 |
| DPP62130 | tpp38518_ hD28A_lS3 2Q-hIgG1 | | HCDR2 | IHPDSSTI | 22 |
| DPP62130 | tpp38518_ hD28A_lS3 2Q-hIgG1 | | HCDR3 | ASLYFGFPWFAY | 23 |
| DPP62130 | tpp38518_ hD28A_lS3 2Q-hIgG1 | | VH | EVQLVESGGGLVQPGGSLRLSCAASGFAFTT YWMSWVRQAPGKGLEWIGEIHPDSSTINYA PSLKDRFTISRDNAKNSLYLQMNSLRAEDTG VYYCASLYFGFPWFAYWGQGTPVTVSS | 24 |
| DPP62130 | tpp38518_ hD28A_lS3 2Q-hIgG1 | | HC | EVQLVESGGGLVQPGGSLRLSCAASGFAFTT YWMSWVRQAPGKGLEWIGEIHPDSSTINYA PSLKDRFTISRDNAKNSLYLQMNSLRAEDTG VYYCASLYFGFPWFAYWGQGTPVTVSSASTK GPSVFPLAPSSKSTSGGTAALGCLVKDYFPEP VTVSWNSGALTSGVHTFPAVLQSSGLYSLSS VVTVPSSSLGTQTYICNVNHKPSNTKVDKKVE PKSCDKTHTCPPCPAPELLGGPSVFLFPPKP KDTLMISRTPEVTCVVVDVSHEDPEVKFNWY VDGVEVHNAKTKPREEQYNSTYRVVSVLTVL HQDWLNGKEYKCKVSNKALPAPIEKTISKAK | 25 |
| | | | | GQPREPQVYTLPPSRDELTKNQVSLTCLVKG FYPSDIAVEWESNGQPENNYKTTPPVLDSDG SFFLYSKLTVDKSRWQQGNVFSCSVMHEAL HNHYTQKSLSLSPGK | |
| DPP62130 | tpp38518_ hD28A_lS3 2Q-hIgG1 | | LCDR1 | GTQLA | 26 |
| DPP62130 | tpp38518_ hD28A_lS3 2Q-hIgG1 | | LCDR2 | YWASTLHTGVPS | 27 |
| DPP62130 | tpp38518_ hD28A_lS3 2Q-hIgG1 | | LCDR3 | QQYSLYRS | 28 |
| DPP62130 | tpp38518_ hD28A_lS3 2Q-hIgG1 | | VL | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIK | 29 |
| DPP62130 | tpp38518_ hD28A_lS3 2Q-hIgG1 | | LC | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIKRTVAAPSVFIFPPSDEQLKS GTASVVCLLNNFYPREAKVQWKVDNALQSG NSQESVTEQDSKDSTYSLSSTLTLSKADYEKH KVYACEVTHQGLSSPVTKSFNRGEC | 30 |
| DPP62136 | tpp38518_ hD28E_lS3 2Q-hIgG1 | | HCDR1 | GFEFTTYW | 31 |
| DPP62136 | tpp38518_ hD28E_lS3 2Q-hIgG1 | | HCDR2 | IHPDSSTI | 32 |
| DPP62136 | tpp38518_ hD28E_lS3 2Q-hIgG1 | | HCDR3 | ASLYFGFPWFAY | 33 |
| DPP62136 | tpp38518_ hD28E_lS3 2Q-hIgG1 | | VH | EVQLVESGGGLVQPGGSLRLSCAASGFEFTT YWMSWVRQAPGKGLEWIGEIHPDSSTINYA PSLKDRFTISRDNAKNSLYLQMNSLRAEDTG VYYCASLYFGFPWFAYWGQGTPVTVSS | 34 |
| DPP62136 | tpp38518_ hD28E_lS3 2Q-hIgG1 | | HC | EVQLVESGGGLVQPGGSLRLSCAASGFEFTT YWMSWVRQAPGKGLEWIGEIHPDSSTINYA PSLKDRFTISRDNAKNSLYLQMNSLRAEDTG VYYCASLYFGFPWFAYWGQGTPVTVSSASTK GPSVFPLAPSSKSTSGGTAALGCLVKDYFPEP VTVSWNSGALTSGVHTFPAVLQSSGLYSLSS VVTVPSSSLGTQTYICNVNHKPSNTKVDKKVE PKSCDKTHTCPPCPAPELLGGPSVFLFPPKP KDTLMISRTPEVTCVVVDVSHEDPEVKFNWY VDGVEVHNAKTKPREEQYNSTYRVVSVLTVL HQDWLNGKEYKCKVSNKALPAPIEKTISKAK GQPREPQVYTLPPSRDELTKNQVSLTCLVKG FYPSDIAVEWESNGQPENNYKTTPPVLDSDG | 35 |
| | | | | SFFLYSKLTVDKSRWQQGNVFSCSVMHEAL HNHYTQKSLSLSPGK | |
| DPP62136 | tpp38518_ hD28E_lS3 2Q-hIgG1 | | LCDR1 | GTQLA | 36 |
| DPP62136 | tpp38518_ hD28E_lS3 2Q-hIgG1 | | LCDR2 | YWASTLHTGVPS | 37 |
| DPP62136 | tpp38518_ hD28E_lS3 2Q-hIgG1 | | LCDR3 | QQYSLYRS | 38 |
| DPP62136 | tpp38518_ hD28E_lS3 2Q-hIgG1 | | VL | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIK | 39 |
| DPP62136 | tpp38518_ hD28E_lS3 2Q-hIgG1 | | LC | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIKRTVAAPSVFIFPPSDEQLKS GTASVVCLLNNFYPREAKVQWKVDNALQSG NSQESVTEQDSKDSTYSLSSTLTLSKADYEKH KVYACEVTHQGLSSPVTKSFNRGEC | 40 |
| DPP62140 | tpp38518_ hF101W_l S32Q-hIgG1 | | HCDR1 | GFDFTTYW | 41 |
| DPP62140 | tpp38518_ hF101W_l S32Q-hIgG1 | | HCDR2 | IHPDSSTI | 42 |
| DPP62140 | tpp38518_ hF101W_l S32Q-hIgG1 | | HCDR3 | ASLYWGFPWFAY | 43 |
| DPP62140 | tpp38518_ hF101W_l S32Q-hIgG1 | | VH | EVQLVESGGGLVQPGGSLRLSCAASGFDFTT YWMSWVRQAPGKGLEWIGEIHPDSSTINYA PSLKDRFTISRDNAKNSLYLQMNSLRAEDTG VYYCASLYWGFPWFAYWGQGTPVTVSS | 44 |
| DPP62140 | tpp38518_ hF101W_l S32Q-hIgG1 | | HC | EVQLVESGGGLVQPGGSLRLSCAASGFDFTT YWMSWVRQAPGKGLEWIGEIHPDSSTINYA PSLKDRFTISRDNAKNSLYLQMNSLRAEDTG VYYCASLYWGFPWFAYWGQGTPVTVSSAST KGPSVFPLAPSSKSTSGGTAALGCLVKDYFPE PVTVSWNSGALTSGVHTFPAVLQSSGLYSLS SVVTVPSSSLGTQTYICNVNHKPSNTKVDKKV EPKSCDKTHTCPPCPAPELLGGPSVFLFPPK PKDTLMISRTPEVTCVVVDVSHEDPEVKFNW YVDGVEVHNAKTKPREEQYNSTYRVVSVLTV LHQDWLNGKEYKCKVSNKALPAPIEKTISKAK GQPREPQVYTLPPSRDELTKNQVSLTCLVKG | 45 |
| | | | | FYPSDIAVEWESNGQPENNYKTTPPVLDSDG SFFLYSKLTVDKSRWQQGNVFSCSVMHEAL HNHYTQKSLSLSPGK | |
| DPP62140 | tpp38518_ hF101W_l S32Q-hIgG1 | | LCDR1 | GTQLA | 46 |
| DPP62140 | tpp38518_ hF101W_I S32Q-hIgG1 | | LCDR2 | YWASTLHTGVPS | 47 |
| DPP62140 | tpp38518_ hF101W_l S32Q-hIgG1 | | LCDR3 | QQYSLYRS | 48 |
| DPP62140 | tpp38518_ hF101W_l S32Q-hIgG1 | | VL | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIK | 49 |
| DPP62140 | tpp38518_ hF101W_l S32Q-hIgG1 | | LC | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIKRTVAAPSVFIFPPSDEQLKS GTASVVCLLNNFYPREAKVQWKVDNALQSG NSQESVTEQDSKDSTYSLSSTLTLSKADYEKH KVYACEVTHQGLSSPVTKSFNRGEC | 50 |
| DPP62144 | tpp38518_ hF101Y_lS 32Q-hIgG1 | | HCDR1 | GFDFTTYW | 51 |
| DPP62144 | tpp38518_ hF101Y_lS 32Q-hIgG1 | | HCDR2 | IHPDSSTI | 52 |
| DPP62144 | tpp38518_ hF101Y_lS 32Q-hIgG1 | | HCDR3 | ASLYYGFPWFAY | 53 |
| DPP62144 | tpp38518_ hF101Y_lS 32Q-hIgG1 | | VH | EVQLVESGGGLVQPGGSLRLSCAASGFDFTT YWMSWVRQAPGKGLEWIGEIHPDSSTINYA PSLKDRFTISRDNAKNSLYLQMNSLRAEDTG VYYCASLYYGFPWFAYWGQGTPVTVSS | 54 |
| DPP62144 | tpp38518_ hF101Y_lS 32Q-hIgG1 | | HC | EVQLVESGGGLVQPGGSLRLSCAASGFDFTT YWMSWVRQAPGKGLEWIGEIHPDSSTINYA PSLKDRFTISRDNAKNSLYLQMNSLRAEDTG VYYCASLYYGFPWFAYWGQGTPVTVSSASTK GPSVFPLAPSSKSTSGGTAALGCLVKDYFPEP VTVSWNSGALTSGVHTFPAVLQSSGLYSLSS VVTVPSSSLGTQTYICNVNHKPSNTKVDKKVE PKSCDKTHTCPPCPAPELLGGPSVFLFPPKP KDTLMISRTPEVTCVVVDVSHEDPEVKFNWY VDGVEVHNAKTKPREEQYNSTYRVVSVLTVL HQDWLNGKEYKCKVSNKALPAPIEKTISKAK | 55 |
| | | | | GQPREPQVYTLPPSRDELTKNQVSLTCLVKG FYPSDIAVEWESNGQPENNYKTTPPVLDSDG SFFLYSKLTVDKSRWQQGNVFSCSVMHEAL HNHYTQKSLSLSPGK | |
| DPP62144 | tpp38518_ hF101Y_lS 32Q-hIgG1 | | LCDR1 | GTQLA | 56 |
| DPP62144 | tpp38518_ hF101Y_lS 32Q-hIgG1 | | LCDR2 | YWASTLHTGVPS | 57 |
| DPP62144 | tpp38518_ hF101Y_lS 32Q-hIgG1 | | LCDR3 | QQYSLYRS | 58 |
| DPP62144 | tpp38518_ hF101Y_lS 32Q-hIgG1 | | VL | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIK | 59 |
| DPP62144 | tpp38518_ hF101Y_lS 32Q-hIgG1 | | LC | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIKRTVAAPSVFIFPPSDEQLKS GTASVVCLLNNFYPREAKVQWKVDNALQSG NSQESVTEQDSKDSTYSLSSTLTLSKADYEKH KVYACEVTHQGLSSPVTKSFNRGEC | 60 |
| DPP62147 | tpp38518_ hW33H_lS 32Q-hIgG1 | | HCDR1 | GFDFTTYH | 61 |
| DPP62147 | tpp38518_ hW33H_lS 32Q-hIgG1 | | HCDR2 | IHPDSSTI | 62 |
| DPP62147 | tpp38518_ hW33H_lS 32Q-hIgG1 | | HCDR3 | ASLYFGFPWFAY | 63 |
| DPP62147 | tpp38518_ hW33H_lS 32Q-hIgG1 | | VH | EVQLVESGGGLVQPGGSLRLSCAASGFDFTT YHMSWVRQAPGKGLEWIGEIHPDSSTINYAP SLKDRFTISRDNAKNSLYLQMNSLRAEDTGV YYCASLYFGFPWFAYWGQGTPVTVSS | 64 |
| DPP62147 | tpp38518_ hW33H_lS 32Q-hIgG1 | | HC | EVQLVESGGGLVQPGGSLRLSCAASGFDFTT YHMSWVRQAPGKGLEWIGEIHPDSSTINYAP SLKDRFTISRDNAKNSLYLQMNSLRAEDTGV YYCASLYFGFPWFAYWGQGTPVTVSSASTKG PSVFPLAPSSKSTSGGTAALGCLVKDYFPEPV TVSWNSGALTSGVHTFPAVLQSSGLYSLSSV VTVPSSSLGTQTYICNVNHKPSNTKVDKKVEP KSCDKTHTCPPCPAPELLGGPSVFLFPPKPK DTLMISRTPEVTCVVVDVSHEDPEVKFNWYV DGVEVHNAKTKPREEQYNSTYRVVSVLTVLH QDWLNGKEYKCKVSNKALPAPIEKTISKAKG QPREPQVYTLPPSRDELTKNQVSLTCLVKGF YPSDIAVEWESNGQPENNYKTTPPVLDSDGS | 65 |
| | | | | FFLYSKLTVDKSRWQQGNVFSCSVMHEALH NHYTQKSLSLSPGK | |
| DPP62147 | tpp38518_ hW33H_lS 32Q-hIgG1 | | LCDR1 | GTQLA | 66 |
| DPP62147 | tpp38518_ hW33H_lS 32Q-hIgG1 | | LCDR2 | YWASTLHTGVPS | 67 |
| DPP62147 | tpp38518_ hW33H_lS 32Q-hIgG1 | | LCDR3 | QQYSLYRS | 68 |
| DPP62147 | tpp38518_ hW33H_lS 32Q-hIgG1 | | VL | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIK | 69 |
| DPP62147 | tpp38518_ hW33H_lS 32Q-hIgG1 | | LC | DIQMTQSPSSLSASVGDRVTITCQASQDIGT QLAWYQQKPGKAPKLLIYWASTLHTGVPSRF SGSGSGTDFTFTISSLQPEDIATYYCQQYSLY RSFGQGTKVEIKRTVAAPSVFIFPPSDEQLKS GTASVVCLLNNFYPREAKVQWKVDNALQSG NSQESVTEQDSKDSTYSLSSTLTLSKADYEKH KVYACEVTHQGLSSPVTKSFNRGEC | 70 |
| DPP62221 | dpp45205 _wt-hIgG1 | | HCDR1 | GFTFSSYW | 71 |
| DPP62221 | dpp45205 _wt-hIgG1 | | HCDR2 | IHPDSSTI | 72 |
| DPP62221 | dpp45205 _wt-hIgG1 | | HCDR3 | ASLYFGFPWFAY | 73 |
| DPP62221 | dpp45205 _wt-hIgG1 | | VH | EVQLVESGGGVVQPGRSLRLSCAASGFTFSS YWMSWVRQAPGKGLEWVGEIHPDSSTINYA DSVKGRFTISRDNAKNTLYLQMNSLRAEDTA VYYCASLYFGFPWFAYWGQGTLVTVSS | 74 |
| DPP62221 | dpp45205 _wt-hIgG1 | | HC | EVQLVESGGGVVQPGRSLRLSCAASGFTFSS YWMSWVRQAPGKGLEWVGEIHPDSSTINYA DSVKGRFTISRDNAKNTLYLQMNSLRAEDTA VYYCASLYFGFPWFAYWGQGTLVTVSSASTK GPSVFPLAPSSKSTSGGTAALGCLVKDYFPEP VTVSWNSGALTSGVHTFPAVLQSSGLYSLSS VVTVPSSSLGTQTYICNVNHKPSNTKVDKKVE PKSCDKTHTCPPCPAPELLGGPSVFLFPPKP KDTLMISRTPEVTCVVVDVSHEDPEVKFNWY VDGVEVHNAKTKPREEQYNSTYRVVSVLTVL HQDWLNGKEYKCKVSNKALPAPIEKTISKAK GQPREPQVYTLPPSRDELTKNQVSLTCLVKG FYPSDIAVEWESNGQPENNYKTTPPVLDSDG SFFLYSKLTVDKSRWQQGNVFSCSVMHEAL HNHYTQKSLSLSPGK | 75 |
| DPP62221 | dpp45205 _wt-hIgG1 | | LCDR1 | GTSLA | 76 |
| DPP62221 | dpp45205 _wt-hIgG1 | | LCDR2 | YDASTLHTGVPS | 77 |
| DPP62221 | dpp45205 _wt-hIgG1 | | LCDR3 | QQYSLYRS | 78 |
| DPP62221 | dpp45205 _wt-hIgG1 | | VL | DIQMTQSPSSLSASVGDRVTITCQASQDIGTS LAWYQQKPGKAPKLLIYDASTLHTGVPSRFS GSGSGTDFTFTISSLQPEDIATYYCQQYSLYR SFGQGTKVEIK | 79 |
| DPP62221 | dpp45205 _wt-hIgG1 | | LC | DIQMTQSPSSLSASVGDRVTITCQASQDIGTS LAWYQQKPGKAPKLLIYDASTLHTGVPSRFS GSGSGTDFTFTISSLQPEDIATYYCQQYSLYR SFGQGTKVEIKRTVAAPSVFIFPPSDEQLKSGT ASVVCLLNNFYPREAKVQWKVDNALQSGNS QESVTEQDSKDSTYSLSSTLTLSKADYEKHKV YACEVTHQGLSSPVTKSFNRGEC | 80 |
| DPP62291 | dpp45179 _wt-hIgG1 | | HCDR1 | GFDFSSYW | 81 |
| DPP62291 | dpp45179 _wt-hIgG1 | | HCDR2 | ISPDSSTI | 82 |
| DPP62291 | dpp45179 _wt-hIgG1 | | HCDR3 | ASLYFGFPWFAY | 83 |
| DPP62291 | dpp45179 _wt-hIgG1 | | VH | EVQLVESGGGLVQPGGSLRLSCAASGFDFS SYWMSWVRQAPGKGLEWIGEISPDSSTINYA PSVKGRFTISRDNAKNTLYLQMNSLRAEDTA VYYCASLYFGFPWFAYWGQGTLVTVSS | 84 |
| DPP62291 | dpp45179 _wt-hIgG1 | | HC | EVQLVESGGGLVQPGGSLRLSCAASGFDFS SYWMSWVRQAPGKGLEWIGEISPDSSTINYA PSVKGRFTISRDNAKNTLYLQMNSLRAEDTA VYYCASLYFGFPWFAYWGQGTLVTVSSASTK GPSVFPLAPSSKSTSGGTAALGCLVKDYFPEP VTVSWNSGALTSGVHTFPAVLQSSGLYSLSS VVTVPSSSLGTQTYICNVNHKPSNTKVDKKVE PKSCDKTHTCPPCPAPELLGGPSVFLFPPKP KDTLMISRTPEVTCVVVDVSHEDPEVKFNWY VDGVEVHNAKTKPREEQYNSTYRVVSVLTVL HQDWLNGKEYKCKVSNKALPAPIEKTISKAK GQPREPQVYTLPPSRDELTKNQVSLTCLVKG FYPSDIAVEWESNGQPENNYKTTPPVLDSDG SFFLYSKLTVDKSRWQQGNVFSCSVMHEAL HNHYTQKSLSLSPGK | 85 |
| DPP62291 | dpp45179 _wt-hIgG1 | | LCDR1 | GTSLA | 86 |
| DPP62291 | dpp45179 _wt-hIgG1 | | LCDR2 | YDASNLHTGVPS | 87 |
| DPP62291 | dpp45179 _wt-hIgG1 | | LCDR3 | QQYSLYRS | 88 |
| DPP62291 | dpp45179 _wt-hIgG1 | | VL | DIQMTQSPSSLSASVGDRVTITCKASQDVGT SLAWYQQKPGKAPKLLIYDASNLHTGVPSRF | 89 |
| | | | | SGSGSGTDFTFTISSLQPEDIATYYCQQYSLY RSFGQGTKVEIK | |
| DPP62291 | dpp45179 _wt-hIgG1 | | LC | DIQMTQSPSSLSASVGDRVTITCKASQDVGT SLAWYQQKPGKAPKLLIYDASNLHTGVPSRF SGSGSGTDFTFTISSLQPEDIATYYCQQYSLY RSFGQGTKVEIKRTVAAPSVFIFPPSDEQLKS GTASVVCLLNNFYPREAKVQWKVDNALQSG NSQESVTEQDSKDSTYSLSSTLTLSKADYEKH KVYACEVTHQGLSSPVTKSFNRGEC | 90 |
| DPP64106 | A24T_G49 S_K65R_P 114L_V14 9L_K158R-scFv- | | HCDR1 | GFDFTTYW | 91 |
| DPP64106 | A24T_G49 S_K65R_P 114L_V14 9L_K158R-scFv- | | HCDR2 | IHPDSSTI | 92 |
| DPP64106 | A24T_G49 S_K65R_P 114L_V14 9L_K158R-scFv- | | HCDR3 | ASLYFGFPWFAY | 93 |
| DPP64106 | A24T_G49 S_K65R_P 114L_V14 9L_K158R-scFv- | | VH | EVQLVESGGGVVQPGRSLRLSCSTSGFDFTT YWMSWVRQAPGKGLEWISEIHPDSSTINYAP SLRDRFTISRDNAKNTLFLQMDSLRPEDTGV YFCASLYFGFPWFAYWGQGTLVTVSS | 94 |
| DPP64106 | A24T_G49 S_K65R_P 114L_V14 9L_K158R-scFv- | | HC | EVQLVESGGGVVQPGRSLRLSCSTSGFDFTT YWMSWVRQAPGKGLEWISEIHPDSSTINYAP SLRDRFTISRDNAKNTLFLQMDSLRPEDTGV YFCASLYFGFPWFAYWGQGTLVTVSSGGGG SGGGGSGGGGSDIQLTQSPSSLSASLGDRV TITCRASQDVGTSVAWYQQKPGKAPKLLIYW TSTRHTGVPSRFSGSGSGTDFTFTISSLQPEDI ATYYCQQYSLYRSFGQGTKVEIK | 95 |
| DPP64106 | A24T_G49 S_K65R_P 114L_V14 9L_K158R-scFv- | | LCDR1 | GTSVA | 96 |
| DPP64106 | A24T_G49 S_K65R_P 114L_V14 9L_K158R-scFv- | | LCDR2 | YWTSTRHTGVPS | 97 |
| DPP64106 | A24T_G49 S_K65R_P 114L_V14 9L_K158R-scFv- | | LCDR3 | QQYSLYRS | 98 |
| DPP64106 | A24T_G49 S_K65R_P 114L_V14 9L_K158R-scFv- | | VL | DIQLTQSPSSLSASLGDRVTITCRASQDVGTS VAWYQQKPGKAPKLLIYWTSTRHTGVPSRFS GSGSGTDFTFTISSLQPEDIATYYCQQYSLYR SFGQGTKVEIK | 99 |
| DPP64107 | A40T_F103 S_P114L_V 149L_K17 6Q_S210N -scFv- | | HCDR1 | GFDFTTYW | 100 |
| DPP64107 | A40T_F103 S_P114L_V 149L_K17 6Q_S210N -scFv- | | HCDR2 | IHPDSSTI | 101 |
| DPP64107 | A40T_F103 S_P114L_V 149L_K17 6Q_S210N -scFv- | | HCDR3 | ASLYFGSPWFAY | 102 |
| DPP64107 | A40T_F103 S_P114L_V 149L_K17 6Q_S210N -scFv- | | VH | EVQLVESGGGVVQPGRSLRLSCSASGFDFTT YWMSWVRQTPGKGLEWIGEIHPDSSTINYAP SLKDRFTISRDNAKNTLFLQMDSLRPEDTGVY FCASLYFGSPWFAYWGQGTLVTVSS | 103 |
| DPP64107 | A40T_F103 S_P114L_V 149L_K17 6Q_S210N -scFv- | | HC | EVQLVESGGGVVQPGRSLRLSCSASGFDFTT YWMSWVRQTPGKGLEWIGEIHPDSSTINYAP SLKDRFTISRDNAKNTLFLQMDSLRPEDTGVY FCASLYFGSPWFAYWGQGTLVTVSSGGGGS GGGGSGGGGSDIQLTQSPSSLSASLGDRVTI TCKASQDVGTSVAWYQQKPGQAPKLLIYWT STRHTGVPSRFSGSGSGTDFTFTINSLQPEDI ATYYCQQYSLYRSFGQGTKVEIK | 104 |
| DPP64107 | A40T_F103 S_P114L_V 149L_K17 6Q_S210N -scFv- | | LCDR1 | GTSVA | 105 |
| DPP64107 | A40T_F103 S_P114L_V 149L_K17 6Q_S210N -scFv- | | LCDR2 | YWTSTRHTGVPS | 106 |
| DPP64107 | A40T_F103 S_P114L_V 149L_K17 6Q_S210N -scFv- | | LCDR3 | QQYSLYRS | 107 |
| DPP64107 | A40T_F103 S_P114L_V 149L_K17 6Q_S210N -scFv- | | VL | DIQLTQSPSSLSASLGDRVTITCKASQDVGTS VAWYQQKPGQAPKLLIYWTSTRHTGVPSRFS GSGSGTDFTFTINSLQPEDIATYYCQQYSLYR SFGQGTKVEIK | 108 |
| DPP64204 | T30S_T31S _L64V_P88 A_G92A_V 167L_W18 4D_T187N -scFv- | | HCDR1 | GFDFSSYW | 109 |
| DPP64204 | T30S_T31S _L64V_P88 A_G92A_V 167L_W18 4D_T187N -scFv- | | HCDR2 | IHPDSSTI | 110 |
| DPP64204 | T30S_T31S _L64V_P88 A_G92A_V 167L_W18 4D_T187N -scFv- | | HCDR3 | ASLYFGFPWFAY | 111 |
| DPP64204 | T30S_T31S _L64V_P88 A_G92A_V 167L_W18 4D_T187N -scFv- | | VH | EVQLVESGGGVVQPGRSLRLSCSASGFDFS SYWMSWVRQAPGKGLEWIGEIHPDSSTINY APSVKDRFTISRDNAKNTLFLQMDSLRAEDTA VYFCASLYFGFPWFAYWGQGTPVTVSS | 112 |
| DPP64204 | T30S_T31S _L64V_P88 A_G92A_V 167L_W18 4D_T187N -scFv- | | HC | EVQLVESGGGVVQPGRSLRLSCSASGFDFS SYWMSWVRQAPGKGLEWIGEIHPDSSTINY APSVKDRFTISRDNAKNTLFLQMDSLRAEDTA VYFCASLYFGFPWFAYWGQGTPVTVSSGGG GSGGGGSGGGGSDIQLTQSPSSLSASVGDR VTITCKASQDVGTSLAWYQQKPGKAPKLLIYD TSNRHTGVPSRFSGSGSGTDFTFTISSLQPED IATYYCQQYSLYRSFGQGTKVEIK | 113 |
| DPP64204 | T30S_T31S _L64V_P88 A_G92A_V 167L_W18 4D_T187N -scFv- | | LCDR1 | GTSLA | 114 |
| DPP64204 | T30S_T31S _L64V_P88 A_G92A_V 167L_W18 4D_T187N -scFv- | | LCDR2 | YDTSNRHTGVPS | 115 |
| DPP64204 | T30S_T31S _L64V_P88 A_G92A_V 167L_W18 4D_T187N -scFv- | | LCDR3 | QQYSLYRS | 116 |
| DPP64204 | T30S_T31S _L64V_P88 A_G92A_V 167L_W18 4D_T187N -scFv- | | VL | DIQLTQSPSSLSASVGDRVTITCKASQDVGTS LAWYQQKPGKAPKLLIYDTSNRHTGVPSRFS GSGSGTDFTFTISSLQPEDIATYYCQQYSLYR SFGQGTKVEIK | 117 |
| DPP64209 | S35H_K15 8R_T165S_ W184G-scFv- | | HCDR1 | GFDFTTYW | 118 |
| DPP64209 | S35H_K15 8R_T165S_ W184G-scFv- | | HCDR2 | IHPDSSTI | 119 |
| DPP64209 | S35H_K15 8R_T165S_ W184G-scFv- | | HCDR3 | ASLYFGFPWFAY | 120 |
| DPP64209 | S35H_K15 8R_T165S_ W184G-scFv- | | VH | EVQLVESGGGVVQPGRSLRLSCSASGFDFTT YWMHWVRQAPGKGLEWIGEIHPDSSTINYA PSLKDRFTISRDNAKNTLFLQMDSLRPEDTG VYFCASLYFGFPWFAYWGQGTPVTVSS | 121 |
| DPP64209 | S35H_K15 8R_T165S_ W184G-scFv- | | HC | EVQLVESGGGVVQPGRSLRLSCSASGFDFTT YWMHWVRQAPGKGLEWIGEIHPDSSTINYA PSLKDRFTISRDNAKNTLFLQMDSLRPEDTG VYFCASLYFGFPWFAYWGQGTPVTVSSGGG GSGGGGSGGGGSDIQLTQSPSSLSASVGDR VTITCRASQDVGSSVAWYQQKPGKAPKLLIY GTSTRHTGVPSRFSGSGSGTDFTFTISSLQPE DIATYYCQQYSLYRSFGQGTKVEIK | 122 |
| DPP64209 | S35H_K15 8R_T165S_ W184G-scFv- | | LCDR1 | GSSVA | 123 |
| DPP64209 | S35H_K15 8R_T165S_ W184G-scFv- | | LCDR2 | YGTSTRHTGVPS | 124 |
| DPP64209 | S35H_K15 8R_T165S_ W184G-scFv- | | LCDR3 | QQYSLYRS | 125 |
| DPP64209 | S35H_K15 8R_T165S_W184G-scFv- | | VL | DIQLTQSPSSLSASVGDRVTITCRASQDVGSS VAWYQQKPGKAPKLLIYGTSTRHTGVPSRFS | 126 |
| | | | | GSGSGTDFTFTISSLQPEDIATYYCQQYSLYR SFGQGTKVEIK | |
| DPP64227 | S35H_T16 5S_L212V-scFv- | | HCDR1 | GFDFTTYW | 127 |
| DPP64227 | S35H_T16 5S_L212V-scFv- | | HCDR2 | IHPDSSTI | 128 |
| DPP64227 | S35H_T16 5S_L212V-scFv- | | HCDR3 | ASLYFGFPWFAY | 129 |
| DPP64227 | S35H_T16 5S_L212V-scFv- | | VH | EVQLVESGGGVVQPGRSLRLSCSASGFDFTT YWMHWVRQAPGKGLEWIGEIHPDSSTINYA PSLKDRFTISRDNAKNTLFLQMDSLRPEDTG VYFCASLYFGFPWFAYWGQGTPVTVSS | 130 |
| DPP64227 | S35H_T16 5S_L212V-scFv- | | HC | EVQLVESGGGVVQPGRSLRLSCSASGFDFTT YWMHWVRQAPGKGLEWIGEIHPDSSTINYA PSLKDRFTISRDNAKNTLFLQMDSLRPEDTG VYFCASLYFGFPWFAYWGQGTPVTVSSGGG GSGGGGSGGGGSDIQLTQSPSSLSASVGDR VTITCKASQDVGSSVAWYQQKPGKAPKLLIY WTSTRHTGVPSRFSGSGSGTDFTFTISSVQPE DIATYYCQQYSLYRSFGQGTKVEIK | 131 |
| DPP64227 | S35H_T16 5S_L212V-scFv- | | LCDR1 | GSSVA | 132 |
| DPP64227 | S35H_T16 5S_L212V-scFv- | | LCDR2 | YWTSTRHTGVPS | 133 |
| DPP64227 | S35H_T16 5S_L212V-scFv- | | LCDR3 | QQYSLYRS | 134 |
| DPP64227 | S35H_T16 5S_L212V-scFv- | | VL | DIQLTQSPSSLSASVGDRVTITCKASQDVGSS VAWYQQKPGKAPKLLIYWTSTRHTGVPSRFS GSGSGTDFTFTISSVQPEDIATYYCQQYSLYR SFGQGTKVEIK | 135 |
| DPP64262 | D28T_T30 S_D54G_T 219V-scFv- | | HCDR1 | GFTFSTYW | 136 |
| DPP64262 | D28T_T30 S_D54G_T 219V-scFv- | | HCDR2 | IHPGSSTI | 137 |
| DPP64262 | D28T_T30 S_D54G_T 219V-scFv- | | HCDR3 | ASLYFGFPWFAY | 138 |
| DPP64262 | D28T_T30 S_D54G_T 219V-scFv- | | VH | EVQLVESGGGVVQPGRSLRLSCSASGFTFST YWMSWVRQAPGKGLEWIGEIHPGSSTINYA | 139 |
| | | | | PSLKDRFTISRDNAKNTLFLQMDSLRPEDTG VYFCASLYFGFPWFAYWGQGTPVTVSS | |
| DPP64262 | D28T_T30 S_D54G_T 219V-scFv- | | HC | EVQLVESGGGVVQPGRSLRLSCSASGFTFST YWMSWVRQAPGKGLEWIGEIHPGSSTINYA PSLKDRFTISRDNAKNTLFLQMDSLRPEDTG VYFCASLYFGFPWFAYWGQGTPVTVSSGGG GSGGGGSGGGGSDIQLTQSPSSLSASVGDR VTITCKASQDVGTSVAWYQQKPGKAPKLLIY WTSTRHTGVPSRFSGSGSGTDFTFTISSLQPE DIAVYYCQQYSLYRSFGQGTKVEIK | 140 |
| DPP64262 | D28T_T30 S_D54G_T 219V-scFv- | | LCDR1 | GTSVA | 141 |
| DPP64262 | D28T_T30 S_D54G_T 219V-scFv- | | LCDR2 | YWTSTRHTGVPS | 142 |
| DPP64262 | D28T_T30 S_D54G_T 219V-scFv- | | LCDR3 | QQYSLYRS | 143 |
| DPP64262 | D28T_T30 S_D54G_T 219V-scFv- | | VL | DIQLTQSPSSLSASVGDRVTITCKASQDVGTS VAWYQQKPGKAPKLLIYWTSTRHTGVPSRFS GSGSGTDFTFTISSLQPEDIAVYYCQQYSLYR SFGQGTKVEIK | 144 |

## Claims

1. An isolated antibody or antigen-binding fragment capable of binding to CEACAM5 comprising:
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291), or
x) a heavy chain antigen-binding region that comprises an H-CDR1 comprising SEQ ID NO: 91, an H-CDR2 comprising SEQ ID NO: 92, and an H-CDR3 comprising SEQ ID NO: 93 and a light chain antigen-binding region that comprises an L-CDR1 comprising SEQ ID NO: 96, an L-CDR2 comprising SEQ ID NO: 97, and an L-CDR3 comprising SEQ ID NO:98 (DPP-64106), or
xi) a heavy chain antigen-binding region that comprises an H-CDR1 comprising SEQ ID NO: 100, an H-CDR2 comprising SEQ ID NO: 101, and an H-CDR3 comprising SEQ ID NO: 102 and a light chain antigen-binding region that comprises an L-CDR1 comprising SEQ ID NO: 105, an L-CDR2 comprising SEQ ID NO: 106, and an L-CDR3 comprising SEQ ID NO:107 (DPP-64107), or
xii) a heavy chain antigen-binding region that comprises an H-CDR1 comprising SEQ ID NO: 109, an H-CDR2 comprising SEQ ID NO: 110 and an H-CDR3 comprising SEQ ID NO: 111 and a light chain antigen-binding region that comprises an L-CDR1 comprising SEQ ID NO: 114, an L-CDR2 comprising SEQ ID NO: 115, and an L-CDR3 comprising SEQ ID NO:116 (DPP-64204), or
xiii) a heavy chain antigen-binding region that comprises an H-CDR1 comprising SEQ ID NO: 118, an H-CDR2 comprising SEQ ID NO: 119, and an H-CDR3 comprising SEQ ID NO: 120 and a light chain antigen-binding region that comprises an L-CDR1 comprising SEQ ID NO: 123, an L-CDR2 comprising SEQ ID NO: 124, and an L-CDR3 comprising SEQ ID NO:125 (DPP-64209), or
xiv) a heavy chain antigen-binding region that comprises an H-CDR1 comprising SEQ ID NO: 127, an H-CDR2 comprising SEQ ID NO: 128, and an H-CDR3 comprising SEQ ID NO: 129 and a light chain antigen-binding region that comprises an L-CDR1 comprising SEQ ID NO: 132, an L-CDR2 comprising SEQ ID NO: 133, and an L-CDR3 comprising SEQ ID NO:134 (DPP-64227), or
xv) a heavy chain antigen-binding region that comprises an H-CDR1 comprising SEQ ID NO: 136, an H-CDR2 comprising SEQ ID NO: 137, and an H-CDR3 comprising SEQ ID NO: 138 and a light chain antigen-binding region that comprises an L-CDR1 comprising SEQ ID NO: 141, an L-CDR2 comprising SEQ ID NO: 142, and an L-CDR3 comprising SEQ ID NO:143 (DPP-64262).

2. The isolated antibody or antigen-binding fragment according to claim 1 comprising
i) a heavy chain antigen-binding region that comprises an H-CDR1 comprising SEQ ID NO: 1, an H-CDR2 comprising SEQ ID NO: 2, and an H-CDR3 comprising SEQ ID NO: 3 and a light chain antigen-binding region that comprises an L-CDR1 comprising SEQ ID NO: 6, an L-CDR2 comprising SEQ ID NO: 7, and an L-CDR3 comprising SEQ ID NO: 8 (DPP-60333), or
ii) a heavy chain antigen-binding region that comprises an H-CDR1 comprising SEQ ID NO: 11, an H-CDR2 comprising SEQ ID NO: 12, and an H-CDR3 comprising SEQ ID NO: 13 and a light chain antigen-binding region that comprises an L-CDR1 comprising SEQ ID NO: 16, an L-CDR2 comprising SEQ ID NO: 17, and an L- CDR3 comprising SEQ ID NO:18 (DPP-62124), or
iii) a heavy chain antigen-binding region that comprises an H-CDR1 comprising SEQ ID NO: 21, an H-CDR2 comprising SEQ ID NO: 22, and an H-CDR3 comprising SEQ ID NO: 23 and a light chain antigen-binding region that comprises an L-CDR1 comprising SEQ ID NO: 26, an L-CDR2 comprising SEQ ID NO: 27, and an L-CDR3 comprising SEQ ID NO:28 (DPP-62130), or
iv) a heavy chain antigen-binding region that comprises an H-CDR1 comprising SEQ ID NO: 31, an H-CDR2 comprising SEQ ID NO: 32, and an H-CDR3 comprising SEQ ID NO: 33 and a light chain antigen-binding region that comprises an L-CDR1 comprising SEQ ID NO: 36, an L-CDR2 comprising SEQ ID NO: 37, and an L-CDR3 comprising SEQ ID NO:38 (DPP-62136), or
v) a heavy chain antigen-binding region that comprises an H-CDR1 comprising SEQ ID NO: 41, an H-CDR2 comprising SEQ ID NO: 42, and an H-CDR3 comprising SEQ ID NO: 43 and a light chain antigen-binding region that comprises an L-CDR1 comprising SEQ ID NO: 46, an L-CDR2 comprising SEQ ID NO: 47, and an L-CDR3 comprising SEQ ID NO:48 (DPP-62140), or
vi) a heavy chain antigen-binding region that comprises an H-CDR1 comprising SEQ ID NO: 51, an H-CDR2 comprising SEQ ID NO: 52, and an H-CDR3 comprising SEQ ID NO: 53 and a light chain antigen-binding region that comprises an L-CDR1 comprising SEQ ID NO: 56, an L-CDR2 comprising SEQ ID NO: 57, and an L-CDR3 comprising SEQ ID NO:58 (DPP-62144), or
vii) a heavy chain antigen-binding region that comprises an H-CDR1 comprising SEQ ID NO: 61, an H-CDR2 comprising SEQ ID NO: 62, and an H-CDR3 comprising SEQ ID NO: 63 and a light chain antigen-binding region that comprises an L-CDR1 comprising SEQ ID NO: 66, an L-CDR2 comprising SEQ ID NO: 67, and an L-CDR3 comprising SEQ ID NO:68 (DPP-62147), or
viii) a heavy chain antigen-binding region that comprises an H-CDR1 comprising SEQ ID NO: 71, an H-CDR2 comprising SEQ ID NO: 72, and an H-CDR3 comprising SEQ ID NO: 73 and a light chain antigen-binding region that comprises an L-CDR1 comprising SEQ ID NO: 76, an L-CDR2 comprising SEQ ID NO: 77, and an L-CDR3 comprising SEQ ID NO:78 (DPP-64221), or
ix) a heavy chain antigen-binding region that comprises an H-CDR1 comprising SEQ ID NO: 81, an H-CDR2 comprising SEQ ID NO: 82, and an H-CDR3 comprising SEQ ID NO: 83 and a light chain antigen-binding region that comprises an L-CDR1 comprising SEQ ID NO: 86, an L-CDR2 comprising SEQ ID NO: 87, and an L- CDR3 comprising SEQ ID NO:88 (DPP-62291).

3. The isolated antibody or antigen-binding fragment according to claim 1 comprising
i) a variable heavy chain comprising SEQ ID NO: 4 and/or a variable light chain domain comprising SEQ ID NO: 9; or
ii) a variable heavy chain domain comprising SEQ ID NO: 14 and/or a variable light chain domain comprising SEQ ID NO: 19; or
iii) a variable heavy chain domain comprising SEQ ID NO: 24 and/or a variable light chain domain comprising SEQ ID NO: 29; or
iv) a variable heavy chain domain comprising SEQ ID NO: 34 and/or a variable light chain domain comprising SEQ ID NO: 39; or
v) a variable heavy chain domain comprising SEQ ID NO: 44 and/or a variable light chain domain comprising SEQ ID NO: 49; or
vi) a variable heavy chain domain comprising SEQ ID NO: 54 and/or a variable light chain domain comprising SEQ ID NO: 59; or
vii) a variable heavy chain domain comprising SEQ ID NO: 64 and/or a variable light chain domain comprising SEQ ID NO: 69; or
viii) a variable heavy chain domain comprising SEQ ID NO: 74 and/or a variable light chain domain comprising SEQ ID NO: 79, or
ix) a variable heavy chain domain comprising SEQ ID NO: 84 and/or a variable light chain domain comprising SEQ ID NO: 89; or
x) a variable heavy chain domain comprising SEQ ID NO: 94 and/or a variable light chain domain comprising SEQ ID NO: 99; or
xi) a variable heavy chain domain comprising SEQ ID NO: 103 and/or a variable light chain domain comprising SEQ ID NO: 108; or
xii) a variable heavy chain domain comprising SEQ ID NO: 112 and/or a variable light chain domain comprising SEQ ID NO: 117; or
xiii) a variable heavy chain domain comprising SEQ ID NO: 121 and/or a variable light chain domain comprising SEQ ID NO: 126; or
xiv) a variable heavy chain domain comprising SEQ ID NO: 130 and/or a variable light chain domain comprising SEQ ID NO: 135; or
xv) a variable heavy chain domain comprising SEQ ID NO: 139 and/or a variable light chain domain comprising SEQ ID NO: 144.

4. The isolated antibody or antigen-binding fragment according to any of claims 1 or 2 comprising
i) a variable heavy chain comprising SEQ ID NO: 4 and/or a variable light chain domain comprising SEQ ID NO: 9; or
ii) a variable heavy chain domain comprising SEQ ID NO: 14 and/or a variable light chain domain comprising SEQ ID NO: 19; or
iii) a variable heavy chain domain comprising SEQ ID NO: 24 and/or a variable light chain domain comprising SEQ ID NO: 29; or
iv) a variable heavy chain domain comprising SEQ ID NO: 34 and/or a variable light chain domain comprising SEQ ID NO: 39; or
v) a variable heavy chain domain comprising SEQ ID NO: 44 and/or a variable light chain domain comprising SEQ ID NO: 49; or
vi) a variable heavy chain domain comprising SEQ ID NO: 54 and/or a variable light chain domain comprising SEQ ID NO: 59; or
vii) a variable heavy chain domain comprising SEQ ID NO: 64 and/or a variable light chain domain comprising SEQ ID NO: 69; or
viii) a variable heavy chain domain comprising SEQ ID NO: 74 and/or a variable light chain domain comprising SEQ ID NO: 79, or
ix) a variable heavy chain domain comprising SEQ ID NO: 84 and/or a variable light chain domain comprising SEQ ID NO: 89.

5. The isolated antibody or antigen-binding fragment according any one of the preceding claims, wherein the isolated antibody or antigen-binding fragment is an IgG antibody.

6. The isolated antibody or antigen-binding fragment according to any one of the preceding claims wherein the isolated antibody or antigen-binding fragment is an IgG1 antibody.

7. The isolated antibody or antigen-binding fragment according to any one of claims 1-6 comprising
i) a heavy chain comprising SEQ ID NO: 5 and/or a light chain comprising SEQ ID NO: 10; or
ii) a heavy chain comprising SEQ ID NO: 15 and/or a light chain comprising SEQ ID NO: 20; or
iii) a heavy chain comprising SEQ ID NO: 25 and/or a light chain comprising SEQ ID NO: 30; or
iv) a heavy chain comprising SEQ ID NO: 35 and/or a light chain comprising SEQ ID NO: 40; or
v) a heavy chain comprising SEQ ID NO: 45 and/or a light chain comprising SEQ ID NO: 50 or
vi) a heavy chain comprising SEQ ID NO: 55 and/or a light chain comprising SEQ ID NO: 60, or
vii) a heavy chain comprising SEQ ID NO: 65 and/or a light chain comprising SEQ ID NO: 70; or
viii) a heavy chain comprising SEQ ID NO: 75 and/or a light chain comprising SEQ ID NO: 80; or
ix) a heavy chain comprising SEQ ID NO: 85 and/or a light chain comprising SEQ ID NO: 90 or
x) a heavy chain comprising SEQ ID NO: 95; or
xi) a heavy chain comprising SEQ ID NO: 104; or
xii) a heavy chain comprising SEQ ID NO: 113; or
xiii) a heavy chain comprising SEQ ID NO: 122; or
xiv) a heavy chain comprising SEQ ID NO: 131; or
xv) a heavy chain comprising SEQ ID NO: 140.

8. The isolated antibody or antigen-binding fragment according to any one of claims 1-6 comprising
i) a heavy chain comprising SEQ ID NO: 5 and/or a light chain comprising SEQ ID NO: 10; or
ii) a heavy chain comprising SEQ ID NO: 15 and/or a light chain comprising SEQ ID NO: 20; or
iii) a heavy chain comprising SEQ ID NO: 25 and/or a light chain comprising SEQ ID NO: 30; or
iv) a heavy chain comprising SEQ ID NO: 35 and/or a light chain comprising SEQ ID NO: 40; or
v) a heavy chain comprising SEQ ID NO: 45 and/or a light chain comprising SEQ ID NO: 50 or
vi) a heavy chain comprising SEQ ID NO: 55 and/or a light chain comprising SEQ ID NO: 60, or
vii) a heavy chain comprising SEQ ID NO: 65 and/or a light chain comprising SEQ ID NO: 70; or
viii) a heavy chain comprising SEQ ID NO: 75 and/or a light chain comprising SEQ ID NO: 80; or
ix) a heavy chain comprising SEQ ID NO: 85 and/or a light chain comprising SEQ ID NO: 90.

9. An antibody conjugate, comprising the monoclonal antibody or antigen-binding fragment according to any one of claims 1-8.

10. An antibody conjugate according to claim 9 comprising a radionuclide, a cytotoxic agent, an organic compound, a protein toxin, an immunomodulator, a further antibody or antigen-binding fragment, a fluorescent moiety or a chimeric antigen receptor.

11. A tissue-targeting compound of formula (I) 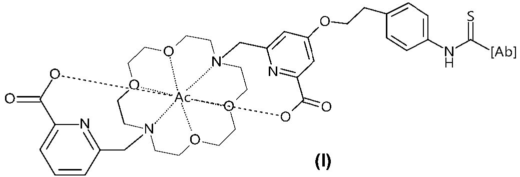 wherein [Ab] is a monoclonal antibody or an antigen-binding fragment thereof capable of binding to CEACAM5 according to any one of claims 1-8.

12. The tissue-targeting compound of formula (I) according to claim 11 wherein [Ab] is bound to the rest of the molecule via a terminal amino group of a lysine residue of the monoclonal antibody or antigen-binding fragment thereof.

13. A process for the preparation of a tissue-targeting compound of formula (I) 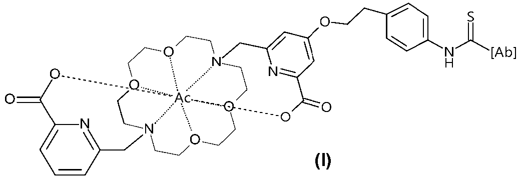
wherein [Ab] is a monoclonal antibody or an antigen-binding fragment thereof having binding affinity for CEACAM5 according to any one of claims 1-8,
said method comprising
(i) coupling a chelating moiety of formula (II) 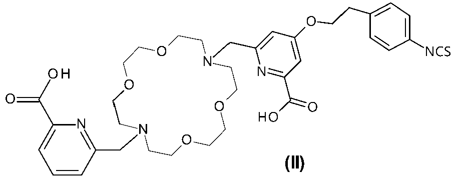 with a monoclonal antibody or an antigen-binding fragment thereof having binding affinity for CEACAM5 according to any one of claims 1-8, and
(ii) contacting the resulting product with an aqueous solution comprising an ion of at least one alpha-emitting actinium isotope.

14. The process according to any one of claims 14 or 15, wherein step (i) comprises coupling a chelating moiety of formula (II) with a monoclonal antibody or an antigen-binding fragment thereof having binding affinity for CEACAM5 via a Lysine residue of the monoclonal antibody or antigen-binding fragment thereof.

15. A tissue-targeting chelator of formula (III) 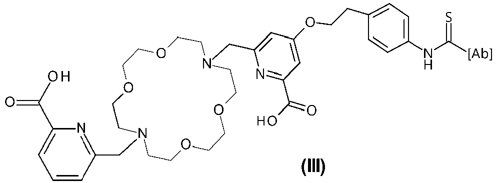 wherein [Ab] is a monoclonal antibody or an antigen-binding fragment thereof having binding affinity for CEACAM5 according to any one of claims 1-8.

16. A pharmaceutical composition comprising an isolated antibody or antigen-binding fragment according to any one of claims 1-8, or an antibody conjugate according to claims 9-10, or a tissue-targeting compound of formula (I) according to any one of claims 11-12 and optionally one or more pharmaceutically acceptable excipients.

17. An antibody or antigen-binding fragment thereof according to any one of claims 1-8 or an antibody conjugate according to any one of claims 9-10 or a tissue-targeting compound of formula (I) according to any one of claims 11-12 or a pharmaceutical composition according to claim 16 for use in the treatment of a disease.

18. An antibody or antigen-binding fragment thereof according to any one of claims 1-8 or an antibody conjugate according to any one of claims 9-10 or a tissue-targeting compound of formula (I) according to any one of claims 11-12 or a pharmaceutical composition according to claim 16 for use according to claim 17, wherein the disease is **characterized by** an ectopic expression of CEACAM5.

19. An antibody or antigen-binding fragment thereof according to any one of claims 1-8 or an antibody conjugate according to any one of claims 9-10 or a tissue-targeting compound of formula (I) according to any one of claims 11-12 or a pharmaceutical composition according to claim 16 for use according to any one of claims 17-18 wherein the disease is cancer, preferably colorectal cancer, non-small-cell lung cancer, gastric cancer, neuroendocrine cancer, pancreatic cancer, breast cancer, liver cancer and thyroid cancer, more preferably colorectal cancer, non-small-cell lung cancer, gastric cancer, neuroendocrine cancer and pancreatic cancer, even more preferably colorectal cancer, non-small-cell lung cancer and gastric cancer.

20. An isolated antibody or antigen-binding fragment according to any one of claims 1-8 or the conjugate according to any one of claims 9-10 or a tissue-targeting compound of formula (I) according to any one of claims 11-12 or the pharmaceutical composition according to claim 16 for use in simultaneous, separate, or sequential combination with one or more further therapeutically active compounds.

21. An isolated antibody or antigen-binding fragment according to any one of claims 1-8, or an antibody conjugate according to any one of claims 9-10, or a tissue-targeting compound of formula (I) according to any one of claims 11-12 for use as a diagnostic or imaging agent.

22. An isolated nucleic acid sequence that encodes the antibody or antigen-binding fragment according to any one of claims 1-8.

23. A vector comprising a nucleic acid sequence according to claim 22.

24. An isolated cell arranged for production of an antibody or antigen-binding fragment according to any one of claims 1-8.

25. A method for producing an antibody or antigen-binding fragment according to any one of claims 1-8 or an antibody conjugate according to any one of claims 9-10, or a tissue-targeting compound of formula (I) according to any one of claims 11-12, comprising culturing a cell according to claim 24 and optionally purification of the antibody or antigen-binding fragment.

26. A kit comprising the isolated antibody or antigen-binding fragment according to any one of claims 1-8 or the conjugate according to any one of claims 9-10 or a tissue-targeting compound of formula (I) according to any one of claims 11-12 or the pharmaceutical composition according to claim 16 and instructions for use.
